# EUROPÄISCHE PATENTSCHRIFT

(11) **EP 2 802 587 B1**
(45) Veröffentlichungstag und Bekanntmachung des Hinweises auf die Patenterteilung: **06.09.2017**
(21) Anmeldenummer: 13700985.8
(22) Anmeldetag: 08.01.2013
(51) Int. Cl.: C07D 487/04, C07D 519/00, A61K 31/519, A61K 31/53, A61P 9/10, A61P 9/12

(54) **SUBSTITUIERTE, ANNELLIERTE IMIDAZOLE UND PYRAZOLE UND IHRE VERWENDUNG**
SUBSTITUTED, ANNELLATED IMIDAZOLES AND PYRAZOLES AND THEIR USE
IMIDAZOLES ET PYRAZOLES SUBSTITUÉES ANELLÉES ET LEUR UTILISATION

(30) Priorität: 11.01.2012 DE 102012200352
(43) Veröffentlichungstag der Anmeldung: 19.11.2014
(73) Patentinhaber: Bayer Intellectual Property GmbH, 40789 Monheim (DE); Bayer Pharma Aktiengesellschaft, 13353 Berlin (DE)
(72) Erfinder: FOLLMANN, Markus, 50859 Köln (DE); STASCH, Johannes-Peter, 42651 Solingen (DE); REDLICH, Gorden, 44799 Bochum (DE); GRIEBENOW, Nils, 41541 Dormagen (DE); LANG, Dieter, 42553 Velbert (DE); WUNDER, Frank, 42117 Wuppertal (DE); PAULSEN, Holger, 40724 Hilden (DE); HÜBSCH, Walter, 42113 Wuppertal (DE); TERSTEEGEN, Adrian, 42111 Wuppertal (DE); VAKALOPOULOS, Alexandros, 40721 Hilden (DE)
(74) Vertreter: BIP Patents
(86) Internationale Anmeldenummer: PCT/EP2013/050180
(87) Internationale Veröffentlichungsnummer: WO 2013/104598

(56) Entgegenhaltungen:
- WO-A1-03/095451
- WO-A1-2004/009590
- WO-A1-2010/065275
- WO-A1-2011/149921
- WO-A1-2012/004258
- WO-A1-2012/143510
- WO-A1-2012/152629

## Beschreibung

Die vorliegende Anmeldung betrifft neue substituierte, annellierte Imidazole und Pyrazole, Verfahren zu ihrer Herstellung, diese zur Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

Vor einigen Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-fmyl)-1-benzylindazol [YC-1; Wu et al., Blood 84 (1994), 4226; Mülsch et al., Brit. J. Pharmacol. 120 (1997), 681]. Zu den neueren Stimulatoren der löslichen Guanylatzyklase gehören u.a. BAY 41-2272, BAY 41-8543 und Riociguat (BAY 63-2521) (siehe z.B. Stasch J.-P. et al., Nat. Rev. Drug Disc. 2006; 5: 755-768; Stasch J.-P. et al., ChemMedChem 2009; 4: 853-865. Stasch J.-P. et al., Circulation 2011; 123: 2263-2273). Interessanterweise zeigen einige dieser sGC Stimulatoren, wie beispielsweise YC-1 oder BAY 41-2272 zusätzlich zur direkten Guanylatzyklasestimulation auch eine PDE5-inhibitorische Wirkung. Um den cGMP-pathway zu maximieren, ist es pharmakologisch wünschenswert, die Synthese von cGMP zu stimulieren und gleichzeitig den Abbau über PDE-5 zu inhibieren. Dieses duale Prinzip ist pharmakologisch besonders vorteilhaft (s. z.B. Oudout et al., Eur. Urol. 2011, 60, 1020-1026.).

Das duale Prinzip wird im Sinne der vorliegenden Erfindung erfüllt, wenn die erfindungsgemäßen Verbindungen eine Wirkung an rekombinanter Guanylatcyclase-Reporterzellinien gemäß der Untersuchung unter B-2 als minimal effective concentration (MEC) von ≤ 3 µM zeigen und eine Inhibition der humanen Phosphodiesterase 5 (PDE5) gemäß der Untersuchung unter B-6 als IC₅₀ < 100 nM zeigen.

Phosphodiesterase-5 (PDE5) ist der Name für eines der Enzyme, die die Phosphorsäureesterbindung in cGMP spalten, wobei 5'-Guanosinmonophosphat (5'-GMP) entsteht. Beim Menschen kommt die Phosphodiesterase-5 vorwiegend in der glatten Muskulatur des Penisschwellkörpers (Corpus cavernosum penis) und der Lungenarterien vor. Blockierung des cGMP-Abbaus durch Hemmung von PDE5 (mit beispielsweise Sildenafil, Vardenafil oder Tadalafil) führt zu vermehrten Signalen der Entspannungs-Signalwege und speziell zu erhöhter Blutzufuhr in den Penisschwellkörper und Druckerniedrigung in den Blutgefäßen der Lunge. Sie werden zur Behandlung der erektilen Dysfunktion und der pulmonalen arteriellen Hypertonie eingesetzt. Neben PDE5 gibt es weitere, ausschließlich cGMP spaltende Phosphodiesterasen (Stasch J.-P. et al. Circulation 2011).

Als Stimulatoren der löslichen Guanylatcyclase werden in WO 00/06568 und WO 00/06569 annellierte Pyrazol-Derivate und in WO 03/095451 Carbamat-substitutierte 3-Pyrimidinyl-Pyrazolopyridine offenbart. 3-Pyrimidinyl-Pyrazolopyridine mit Phenylamid-Substituenten werden in E. M. Becker et al., BMC Pharmacology 1 (13), 2001 beschrieben. WO 2004/009590 beschreibt Pyrazolopyridine mit substituierten 4-Aminopyrimidinen zur Behandlung von ZNS-Erkrankungen. WO 2010/065275 und WO 2011/149921 offenbaren substituierte Pyrrolo- und Dihydropyridopyrimidine als sGC Aktivatoren. Als sGC Stimulatoren werden in WO 2012/004259 annellierte Aminopyrimidine und in WO 2012/004258, WO 2012/143510 und WO 2012/152629 annellierte Pyrimidine und Triazine beschrieben. WO 2012/28647 offenbart Pyrazolopyridine mit verschiedenen Azaheterocyclen zur Behandlung kardiovaskulärer Erkrankungen.

Aufgabe der vorliegenden Erfindung war die Bereitstellung neuer Substanzen, die als Stimulatoren der löslichen Guanylatcyclase und als Stimulatoren der löslichen Guanylatcyclase und Phosphodiesterase-5-Inhibitoren (duales Prinzip) wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung.

Offenbarungsgegenstand sind Verbindungen der allgemeinen Formel (I) 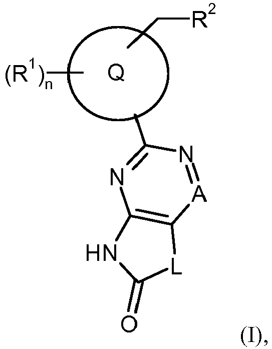 in welcher
- A: für Stickstoff oder CR³ steht,
wobei
R³ für Wasserstoff, Deuterium, Fluor, Chlor, Iod, Difluormethyl, Trifluormethyl, (C₁-C₄)-Alkyl, (C₂-C₄)-Alkenyl, (C₂-C₄)-Alkinyl, Cyclopropyl, Cyclobutyl, Phenyl oder 5- oder 6-gliedriges Heteroaryl steht,
worin (C₁-C₄)-Alkyl, (C₂-C₄)-Alkenyl, (C₂-C₄)-Alkinyl, Phenyl und 5- oder 6-gliedriges Heteroaryl mit 1 bis 3 Substituenten unabhängig voneinander ausgewählt aus der Gruppe mit Fluor, Difluormethyl, Trifluormethyl, (C₁-C₄)-Alkyl, Difluormethoxy, Trifluormethoxy, (C₁-C₄)-Alkoxy, (C₁-C₄)-Alkoxycarbonyl, Cyclopropyl und Cyclobutyl substituiert sein können,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0, 1 oder 2 steht,
R^{4A} für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxy-carbonyl und Amino substituiert sein kann,
R^{4B} für Wasserstoff, Fluor, Difluormethyl, Trifluormethyl, (C₁-C₆)-Alkyl, Cyano, (C₃-C₇)-Cycloalkyl, Difluormethoxy, Trifluormethoxy oder eine Gruppe der Formel -M-R⁶ steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Difluormethoxy und Trifluormethoxy substituiert sein kann,
und worin
M für eine Bindung oder (C₁-C₄)-Alkandiyl steht,
R⁶ für -(C=O)ᵣ-OR⁷, -(C=O)ᵣ-NR⁷R⁸_{,} -C(=S)-NR⁷R⁸, -NR⁷-(C=O)-R¹⁰, -NR⁷-(C=O)-OR¹⁰, -NR⁷-(C=O)-NR⁸R⁹, -NR⁷-SO₂-NR⁸R⁹, -NR⁷-SO₂-R¹⁰, -S(O)ₛ-R¹⁰, -SO₂-NR⁷R⁸, 4- bis 7-gliedriges Heterocyclyl, Phenyl oder 5-oder 6-gliedriges Heteroaryl steht,
worin
r die Zahl 0 oder 1 bedeutet,
s die Zahl 0, 1 oder 2 bedeutet,
R⁷, R⁸ und R⁹ unabhängig voneinander jeweils für Wasserstoff, (C₁-C₆)-Alkyl, (C₃-C₈)-Cycloalkyl, 4- bis 7-gliedriges Heterocyclyl, Phenyl oder 5- oder 6-gliedriges Heteroaryl stehen, oder
R⁷ und R⁸ bilden zusammen mit dem/den Atom/-en, an die sie jeweils gebunden sind, einen 4- bis 7-gliedrigen Heterocyclus, worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, oder
R⁸ und R⁹ bilden zusammen mit dem/den Atom/-en, an die sie jeweils gebunden sind, einen 4- bis 7-gliedrigen Heterocyclus, worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
R¹⁰ für (C₁-C₆)-Alkyl oder (C₃-C₇)-Cycloalkyl steht, oder
R⁷ und R¹⁰ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, und
worin 4- bis 7-gliedriges Heterocyclyl, Phenyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
und
worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen, sofern nicht anders angegeben, jeweils unabhängig voneinander weiterhin mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
oder
- R^{4A} und R^{4B}: zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden,
worin der 3- bis 6-gliedrigen Carbocyclus und der 4- bis 7-gliedrigen Heterocyclus mit 1 oder 2 Substituenten unabhängig voneinander ausgewählt aus der Gruppe Fluor und (C₁-C₄)-Alkyl substituiert sein können,
- R^{5A}: für Wasserstoff, Fluor, (C₁-C₄)-Alkyl oder Hydroxy steht,
- R^{5B}: für Wasserstoff, Fluor, (C₁-C₄)-Alkyl oder Trifluormethyl steht,
der Ring Q für 8- bis 9-gliedriges Heteroaryl steht,
- R¹: für Halogen, Cyano, Difluormethyl, Trifluormethyl, (C₁-C₄)-Alkyl, Hydroxy, Oxo oder (C₁-C₄)-Alkoxy steht,
- n: für eine Zahl 0, 1 oder 2 steht,
- R²: für Trifluormethyl, (C₁-C₆)-Alkyl, (C₃-C₈)-Cycloalkyl, Phenyl oder 5- oder 6-gliedriges Heteroaryl steht,
wobei (C₁-C₆)-Alkyl mit einem Substituenten ausgewählt aus der Gruppe Difluormethyl und Trifluormethyl substituiert ist,
wobei (C₁-C₆)-Alkyl mit 1 bis 3 Substituenten Fluor substituiert sein kann,
wobei (C₃-C₈)-Cycloalkyl mit 1 oder 2 Substituenten unabhängig voneinander ausgewählt aus der Gruppe Fluor, Methyl und Methoxy substituiert sein kann,
wobei Phenyl mit 1 bis 3 Substituenten Fluor substituiert ist,
wobei Phenyl mit 1 oder 2 Substituenten unabhängig voneinander ausgewählt aus der Gruppe Methyl und Methoxy substituiert sein kann,
und
wobei 5- und 6-gliedriges Heteroaryl mit 1 oder 2 Substituenten unabhängig voneinander ausgewählt aus der Gruppe Fluor und Methyl substituiert sein können,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) wie in den Ansprüchen definiert und deren *N*-Oxide, Salze, Solvate und Solvate der *N*-Oxide und Salze, die von Formel (I) umfassten Verbindungen der nachfolgend genannten Formeln und deren *N*-Oxide, Salze, Solvate und Solvate der *N*-Oxide und Salze sowie die von Formel (I) umfassten, nachfolgend als Ausführungsbeispiele genannten Verbindungen und deren *N*-Oxide, Salze, Solvate und Solvate der *N*-Oxide und Salze, soweit es sich bei den von Formel (I) umfassten, nachfolgend genannten Verbindungen nicht bereits um *N*-Oxide, Salze, Solvate und Solvate der *N*-Oxide und Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor Brom und Iod wie ²H (Deuterium), ³H (Tritium), ¹³C ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem offenbart werden Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl. Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.
5- bis 7-gliedrigen gesättigter oder teilweise ungesättigter Carbocyclus steht in Rahmen der Erfindung für einen gesättigten oder teilweise ungesättigten cyclischen Alkylrest mit der jeweils angegebenen Anzahl an Kohlenstoffatomen. Beispielhaft und vorzugsweise seien genannt: Cyclopentyl, Cyclohexyl, Cycloheptyl, Cyclopentenyl, Cyclohexenyl und Cycloheptenyl.

Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, Ethan-1,2-diyl, Ethan-1,1-diyl, Propan-1,3-diyl, Propan-1,1-diyl, Propan-1,2-diyl, Propan-2,2-diyl, Butan-1,4-diyl, Butan-1,2-diyl, Butan-1,3-diyl und Butan-2,3-diyl.

Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy, tert.-Butoxy,

Alkoxycarbonyl steht im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.

Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N-*Dimethylamino, *N,N-*Diethylamino, *N-*Ethyl-*N-*methylamino, *N-*Methyl-N-n-propylamino, *N-*Isopropyl-*N-*n-propylamino, *N-*tert.-Butyl-*N-*methylamino, *N-*Ethyl-N-n-pentylamino und *N-*n-Hexyl-*N-*methylamino.

5- bis 7-gliedrigen gesättigter oder teilweise ungesättigte Heterocyclus steht im Rahmen der Erfindung für einen gesättigten oder teilweise ungesättigten Heterocyclus mit insgesamt 5 bis 7 Ringatomen, der ein Ring-Heteroatome aus der Reihe N, O, S, SO und/oder SO₂ enthält. Beispielhaft seien genannt: Pyrrolidinyl, Tetrahydrofuranyl, Piperidinyl, Tetrahydropyranyl, Dihydropyrrolyl, Dihydropyridyl.

Heterocyclyl bzw. Heterocyclus steht im Rahmen der Erfindung für einen gesättigten Heterocyclus mit insgesamt 4 bis 7 Ringatomen, der ein oder zwei Ring-Heteroatome aus der Reihe N, O, S, SO und/oder SO₂ enthält. Beispielhaft seien genannt: Azetidinyl, Oxetanyl, Pyrrolidinyl, Pyrazolidinyl, Imidazolinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl, Tetrahydropyranyl und Morpholinyl.

5- oder 6-gliedriges Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyrazolyl, Oxazolyl, Thiazolyl, Triazolyl, Oxadiazolyl, Thiadiazolyl, Pyridyl und Pyrimidinyl.

8- oder 9-gliedriges Heteroaryl steht im Rahmen der Erfindung für einen bicyclischen aromatischen oder teilweise ungesättigten Heterocyclus mit insgesamt 8 oder 9 Ringatomen, der mindestens zwei Stickstoffatome und bis zu zwei weitere, gleiche oder verschiedene Ring-Heteroatome aus der Reihe N, O und/oder S enthält. Beispielhaft seien genannt: Dihydrothienopyrazolyl, Thienopyrazolyl, Pyrazolopyrazolyl, Imidazothiazolyl, Tetrahydrocyclopentapyrazolyl, Dihydrocyclopentapyrazolyl, Tetrahydroindazolyl, Dihydroindazolyl, Indazolyl, Pyrazolo[4,3-b]pyridyl, Tetrahydropyrazolopyridinyl, Pyrazolopyrimidinyl, Imidazo[1,5-a]pyridyl und Imidazopyrmidinyl.

Halogen steht im Rahmen der Erfindung für Fluor, Chlor, Brom und Iod. Bevorzugt sind Brom und Iod.

Eine Oxo-Gruppe steht im Rahmen der Erfindung für ein Sauerstoffatom, das über eine Doppelbindung an ein Kohlenstoffatom gebunden ist.

Eine Thiooxo-Gruppe steht im Rahmen der Erfindung für ein Schwefelatom, das über eine Doppelbindung an ein Kohlenstoffatom gebunden ist.

In der Formel der Gruppe, für die L bzw. Q stehen kann, steht der Endpunkt der Linie, an dem das Zeichen #, ##, * und ** steht, nicht für ein Kohlenstoffatom beziehungsweise eine CH₂-Gruppe, sondern ist Bestandteil der Bindung zu dem jeweils bezeichneten Atom, an das L bzw. Q gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Offenbarungsgegenstand sind Verbindungen der Formel (I), in welcher
- A: für Stickstoff oder CR³ steht,
wobei
R³ für Wasserstoff, Deuterium, Fluor, Chlor, Iod, Difluormethyl, Trifluormethyl, (C₁-C₄)-Alkyl, Vinyl, Allyl, Ethinyl, Cyclopropyl, Cyclobutyl, Hydroxy, Pyrazolyl oder Pyridyl steht, worin (C₁-C₄)-Alkyl, Vinyl, Allyl, Ethinyl und Pyridyl mit 1 oder 2 Substituenten unabhängig voneinander ausgewählt aus der Gruppe mit Methyl, Cyclopropyl und Cyclobutyl substituiert sein können,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0, 1 oder 2 steht,
R^{4A} für Wasserstoff, Fluor, Methyl, Ethyl, Hydroxy oder Amino steht,
R^{4B} für Wasserstoff, Fluor, Difluormethyl, Trifluormethyl, (C₁-C₄)-Alkyl, Cyano, Cyclopropyl, Cyclobutyl, Cyclopentyl oder eine Gruppe der Formel -M-R⁶ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R⁶ für -(C=O)ᵣ-OR⁷, -(C=O)ᵣ-NR⁷R⁸, -C(=S)-NR⁷R⁸, -NR⁷-(C=O)-OR¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R⁷ und R⁸ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen, worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁰ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können, oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
der Ring Q für eine Gruppe der Formel 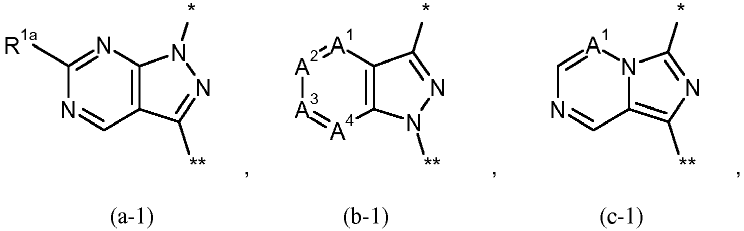 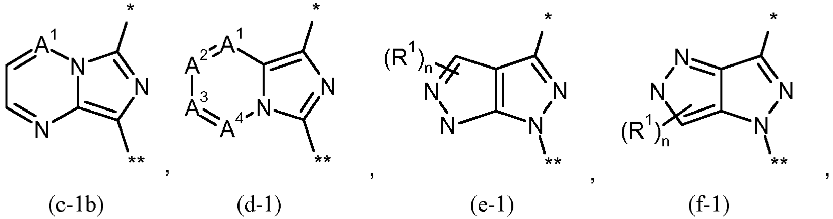 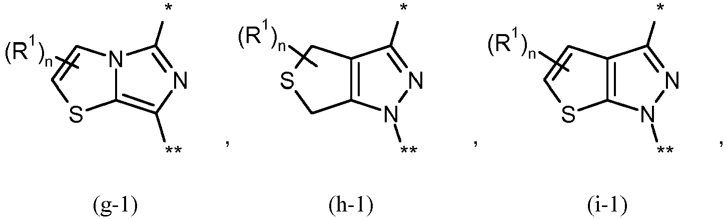 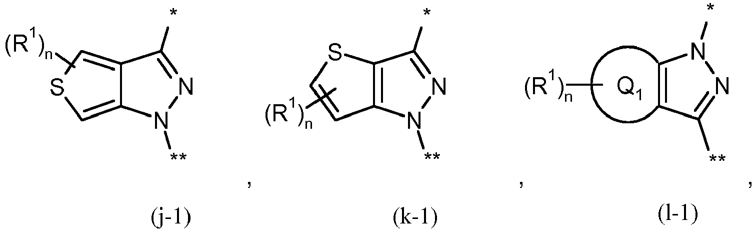 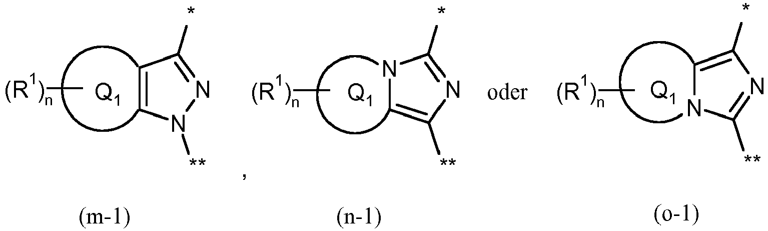 steht, wobei
- *: für die Anknüpfungsstelle an -CH₂-R² steht,
- **: für die Anknüpfungsstelle an den Pyrimidin- bzw. Triazinring steht,
- der Ring Q₁: zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
- R^{1a}: für Wasserstoff oder Methyl steht,
- R¹: für Fluor, Chlor, Methyl, Hydroxy oder Oxo steht,
- n: für eine Zahl 0, 1 oder 2 steht,
- A¹, A², A³ und A⁴: unabhängig voneinander jeweils für N, CH oder CR¹ stehen,
mit der Maßgabe, dass maximal zwei der Gruppen A¹, A², A³ und A⁴ für N stehen,
- R²: für Trifluormethyl, 2,2,2-Trifluorethyl, 3,3,3-Trifluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und
wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
sowie ihre Salze, Solvate und Solvate der Salze. Offenbart werden auch Verbindungen der Formel (I), in welcher
- A: für Stickstoff oder CR³ steht,
wobei
R³ für Wasserstoff, Fluor, Difluormethyl, Trifluormethyl, Methyl, Ethyl, Cyclopropyl, oder Cyclobutyl steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0 steht,
R^{4A} für Wasserstoff, Fluor, Methyl, Ethyl, Hydroxy oder Amino steht,
R^{4B} für Wasserstoff, Fluor, Difluormethyl, Trifluormethyl, Methyl, Ethyl, Cyclopropyl, Cyclobutyl, Cyclopentyl oder eine Gruppe der Formel -M-R⁶ steht,
worin Methyl und Ethyl mit 1 bis 3 Substituenten unabhängig voneinander ausgewählt aus der Gruppe Fluor, Cyano, Trifluormethyl, Cyclopropyl, Cyclobutyl, Difluormethoxy und Trifluormethoxy substituiert sein kann,
und worin
M für eine Bindung steht,
R⁶ für -(C=O)ᵣ-NR⁷R⁸, Phenyl, Thiazolyl, Triazolyl, Oxadiazolyl, Thiadiazolyl oder Pyrimidinyl steht,
worin
r die Zahl 1 bedeutet,
R⁷ und R⁸ unabhängig voneinander jeweils für Wasserstoff, oder Cyclopropyl stehen, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können, oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden, worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
der Ring Q für eine Gruppe der Formel 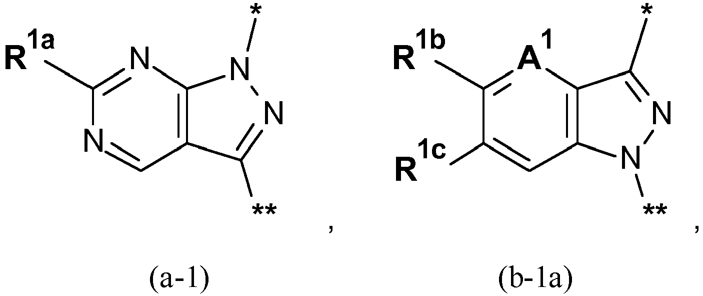 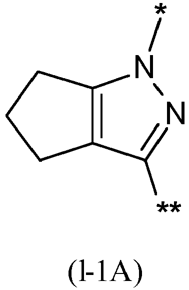 steht, wobei
- *: für die Anknüpfungsstelle an -CH₂-R² steht,
- **: für die Anknüpfungsstelle an den Pyrimidin- bzw. Triazinring steht,
- R¹: für Wasserstoff steht,
- n: für eine Zahl 0 steht,
- R^{1a}: für Wasserstoff oder Methyl steht,
- R^{1b}: für Wasserstoff, Fluor oder Chlor steht,
- R^{1c}: für Wasserstoff oder Fluor steht,
- R^{1d}: für Wasserstoff oder Chlor steht,
- A¹: für N oder CH steht,
- R²: für 3,3,3-Trifluorprop-1-yl, 2,2,3,3-Tetrafluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze. Gegenstand sind im Rahmen der vorliegenden Erfindung Verbindungen der Formel (I), in welcher
- A: für Stickstoff oder CR³ steht,
wobei
R³ für Wasserstoff, Fluor, Difluormethyl, Trifluormethyl, Methyl, Ethyl, Cyclopropyl, oder Cyclobutyl steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0 steht,
R^{4A} für Wasserstoff, Fluor, Methyl, Ethyl, Hydroxy oder Amino steht,
R^{4B} für Wasserstoff, Fluor, Difluormethyl, Trifluormethyl, Methyl, Ethyl, Cyclopropyl, Cyclobutyl, Cyclopentyl oder eine Gruppe der Formel -M-R⁶ steht, worin Methyl und Ethyl mit 1 bis 3 Substituenten unabhängig voneinander ausgewählt aus der Gruppe Fluor, Cyano, Trifluormethyl, Cyclopropyl, Cyclobutyl, Difluormethoxy und Trifluormethoxy substituiert sein kann,
wobei R^{4B} für Wasserstoff, Difluormethyl, Trifluormethyl, Methyl, Ethyl, Cyclopropyl, Cyclobutyl, Cyclopentyl oder eine Gruppe der Formel -M-R⁶ steht, wenn R^{4A} für Hydroxy steht,
und worin
M für eine Bindung steht,
R⁶ für -(C=O)ᵣ-NR⁷R⁸, Phenyl, Thiazolyl, Triazolyl, Oxadiazolyl, Thiadiazolyl oder Pyrimidinyl steht,
worin
r die Zahl 1 bedeutet,
R⁷ und R⁸ unabhängig voneinander jeweils für Wasserstoff, oder Cyclopropyl stehen, und worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
der Ring Q für eine Gruppe der Formel 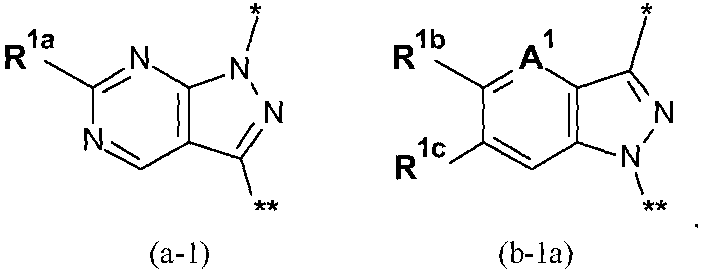 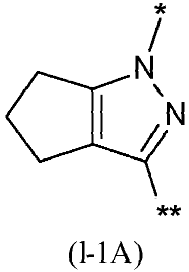 steht, wobei
- *: für die Anknüpfungsstelle an -CH₂-R² steht,
- **: für die Anknüpfungsstelle an den Pyrimidin- bzw. Triazinring steht,
- A¹: für N oder CH steht,
- R¹: für Halogen, Cyano, Difluormethyl, Trifluormethyl, (C₁-C₄)-Alkyl, Hydroxy, Oxo oder (C₁-C₄)Alkoxy steht,
- n: für eine Zahl 0 steht,
- R^{1a}: für Wasserstoff oder Methyl steht,
- R^{1b}: für Wasserstoff, Fluor oder Chlor steht, wenn A¹ für CH steht,
- R^{1b}: für Wasserstoff steht, wenn A¹ für N steht,
- R^{1c}: für Wasserstoff oder Fluor steht,
- R²: für 3,3,3-Trifluorprop-1-yl, 2,2,3,3-Tetrafluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

Besonders bevorzugt im Rahmen der vorliegenden Erfindung sind Verbindungen der Formel (I), in welcher
- A: für Stickstoff oder CR³ steht,
wobei
R³ für Wasserstoff steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
der Ring Q für eine Gruppe der Formel 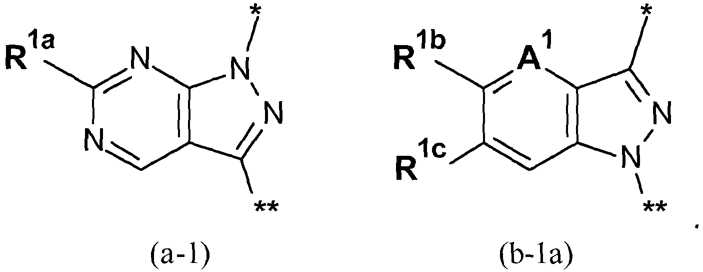 steht, wobei
* für die Anknüpfungsstelle an -CH₂-R² steht,
** für die Anknüpfungsstelle an den Pyrimidin- bzw. Triazinring steht,
R¹ für Halogen, Cyano, Difluormethyl, Trifluormethyl, (C₁-C₄)-Alkyl, Hydroxy, Oxo oder (C₁-C₄)Alkoxy steht,
n für eine Zahl 0 steht,
R^{1a} für Wasserstoff oder Methyl steht,
R^{1b} für Wasserstoff, Fluor oder Chlor steht,
R^{1c} für Wasserstoff oder Fluor steht,
A¹ für N oder CH steht,
R² für 3,3,3-Trifluorprop-1-yl, 2,2,3,3,3-Pentafluorprop-1-yl, Phenyl oder Pyridyl steht, wobei Phenyl mit 1 bis 3 Substituenten Fluor substituiert ist,
   und
   wobei Pyridyl mit 1 Substituenten Fluor substituiert sein kann,
sowie ihre Salze, Solvate und Solvate der Salze.

Besonders bevorzugt im Rahmen der vorliegenden Erfindung sind Verbindungen der Formel (I), in welcher
- A: für Stickstoff oder CR³ steht,
wobei
R³ für Wasserstoff steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht, wobei R^{4B} für Wasserstoff, Trifluormethyl, 2,2,2-Trifluorethyl oder Methyl steht, wenn R^{4A} für Hydroxy steht,
der Ring Q für eine Gruppe der Formel 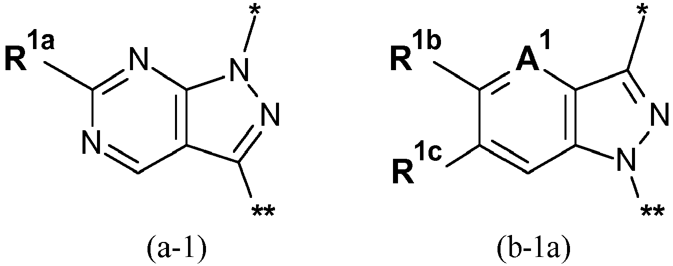 steht, wobei
- *: für die Anknüpfungsstelle an -CH₂-R² steht,
- **: für die Anknüpfungsstelle an den Pyrimidin- bzw. Triazinring steht,
- A¹: für N oder CH steht,
- R¹: für Halogen, Cyano, Difluormethyl, Trifluormethyl, (C₁-C₄)-Alkyl, Hydroxy, Oxo oder (C₁-C₄)Alkoxy steht,
- n: für eine Zahl 0 steht,
- R^{1a}: für Wasserstoff oder Methyl steht,
- R^{1b}: für Wasserstoff, Fluor oder Chlor steht, wenn A¹ für CH steht,
- R^{1b}: für Wasserstoff steht, wenn A¹ für N steht,
- R^{1c}: für Wasserstoff oder Fluor steht,
- R²: für 3,3,3-Trifluorprop-1-yl, 2,2,3,3,3-Pentafluorprop-1-yl, Phenyl oder Pyridyl steht, wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

Besonders bevorzugt im Rahmen der vorliegenden Erfindung sind Verbindungen der Formel (I), in welcher
- A: für Stickstoff oder CR³ steht,
wobei
R³ für Wasserstoff steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Pyrimidin- bzw. Triazinring steht,
m für eine Zahl 0 steht,
- R^{4A}: für Methyl steht,
- R^{4B}: für Methyl steht,
der Ring Q für eine Gruppe der Formel 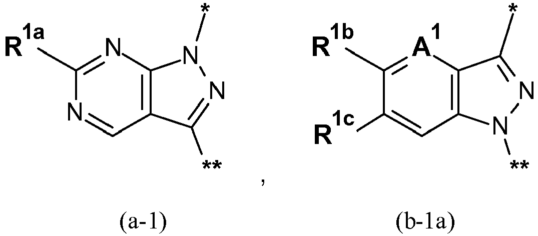 steht, wobei
- *: für die Anknüpfungsstelle an -CH₂-R² steht,
- **: für die Anknüpfungsstelle an den Pyrimidin- bzw. Triazinring steht,
- A¹: für N oder CH steht,
- R¹: für Halogen, Cyano, Difluormethyl, Trifluormethyl, (C₁-C₄)-Alkyl, Hydroxy, Oxo oder (C₁-C₄)Alkoxy steht,
- n: für eine Zahl 0 steht,
- R^{1a}: für Wasserstoff oder Methyl steht,
- R^{1b}: für Wasserstoff, Fluor oder Chlor steht, wenn A¹ für CH steht,
- R^{1b}: für Wasserstoff steht, wenn A¹ für N steht,
- R^{1c}: für Wasserstoff oder Fluor steht,
- R²: für 2,2,3,3,3-Pentafluorprop-1-yl, Phenyl oder Pyridyl steht,
wobei Phenyl mit 1 oder 2 Substituenten Fluor substituiert ist,
und
wobei Pyridyl mit 1 Substituenten Fluor substituiert sein kann,
sowie ihre Salze, Solvate und Solvate der Salze.

Besonders bevorzugt im Rahmen der vorliegenden Erfindung sind Verbindungen der Formel (I), in welcher
- A: für Stickstoff steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Triazinring steht,
m für eine Zahl 0 steht,
- R^{4A}: für Methyl steht,
- R^{4B}: für Methyl steht,
der Ring Q für eine Gruppe der Formel 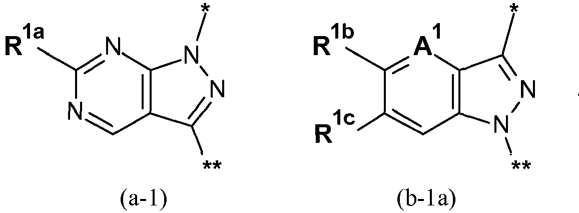 steht, wobei
- *: für die Anknüpfungsstelle an -CH₂-R² steht,
- **: für die Anknüpfungsstelle an den Triazinring steht,
- A¹: für N oder CH steht,
- R¹: für Halogen, Cyano, Difluormethyl, Trifluormethyl, (C₁-C₄)-Alkyl, Hydroxy, Oxo oder (C₁-C₄)Alkoxy steht,
- n: für eine Zahl 0 steht,
- R^{1a}: für Wasserstoff oder Methyl steht,
- R^{1b}: für Wasserstoff, Fluor oder Chlor steht, wenn A¹ für CH steht,
- R^{1b} für: Wasserstoff steht, wenn A¹ für N steht,
- R^{1c}: für Wasserstoff oder Fluor steht,
- R²: für Phenyl steht,
wobei Phenyl mit 1 oder 2 Substituenten Fluor substituiert ist,
sowie ihre Salze, Solvate und Solvate der Salze.

Besonders bevorzugt im Rahmen der vorliegenden Erfindung sind folgende Verbindungen 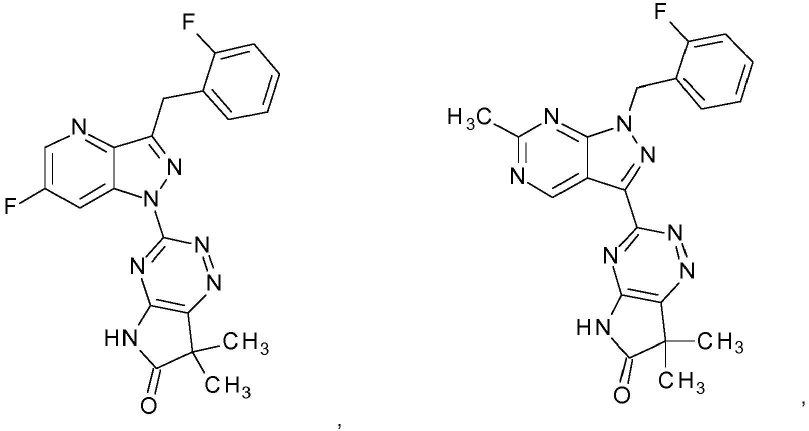 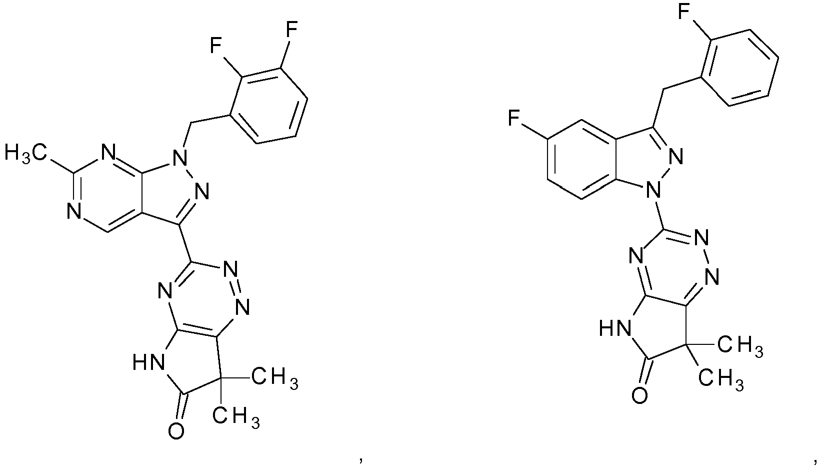 sowie ihre Salze, Solvate und Solvate der Salze.

Bevorzugt sind im Rahmen der vorliegenden Erfindung auch Verbindungen der Formel (I) wie in den Ansprüchen definiert, in welcher A für N oder CH steht, sowie ihre Salze, Solvate und Solvate der Salze.

Bevorzugt sind im Rahmen der vorliegenden Erfindung auch Verbindungen der Formel (I) wie in den Ansprüchen definiert, in welcher A für N steht, sowie ihre Salze, Solvate und Solvate der Salze.

Bevorzugt sind im Rahmen der vorliegenden Erfindung auch Verbindungen der Formel (I) wie in den Ansprüchen definiert, in welcher A für CH steht, sowie ihre Salze, Solvate und Solvate der Salze.

Bevorzugt sind im Rahmen der vorliegenden Erfindung auch Verbindungen der Formel (I), in welcher
- A: für Stickstoff steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Triazinring steht,
m für eine Zahl 0 steht,
R^{4A} für Methyl steht,
R^{4B} für Methyl steht, und
- Q, n, R¹ und R²: jeweils die zuvor als Gegenstand der Erfindung angegebenen Bedeutungen haben,
sowie ihre Salze, Solvate und Solvate der Salze.

Bevorzugt sind im Rahmen der vorliegenden Erfindung auch Verbindungen der Formel (I) wie in den Ansprüchen definiert, in welcher
der Ring Q für eine Gruppe der Formel 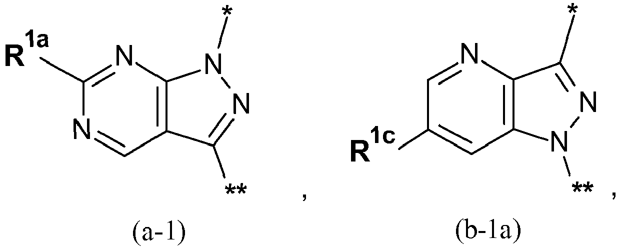 steht, wobei
- *: für die Anknüpfungsstelle an -CH₂-R² steht,
- **: für die Anknüpfungsstelle an den Pyrimidin- bzw. Triazinring steht,
- R¹: für Halogen, Cyano, Difluormethyl, Trifluormethyl, (C₁-C₄)-Alkyl, Hydroxy, Oxo oder (C₁-C₄)Alkoxy steht,
- n: für eine Zahl 0 steht,
- R^{1a}: für Wasserstoff oder Methyl steht,
- R^{1c}: für Wasserstoff oder Fluor steht,
sowie ihre Salze, Solvate und Solvate der Salze.

Bevorzugt sind im Rahmen der vorliegenden Erfindung auch Verbindungen der Formel (I) wie in den Ansprüchen definiert, in welcher
- A: für Stickstoff steht,
- L: für eine Gruppe #-CR^{4A}R^{4B}-(CR^{5A}R^{5B})ₘ-## steht,
wobei
#¹ für die Anknüpfstelle an die Carbonylgruppe steht,
#² für die Anknüpfstelle an den Triazinring steht,
m für eine Zahl 0 steht,
R^{4A} für Wasserstoff, Fluor, Methyl, Ethyl, Hydroxy oder Amino steht,
R^{4B} für eine Gruppe der Formel -M-R⁶ steht,
und worin
M für eine Bindung steht,
R⁶ für -(C=O)ᵣ-NR⁷R⁸, Phenyl, Thiazolyl, Triazolyl, Oxadiazolyl, Thiadiazolyl oder Pyrimidinyl steht,
worin
r die Zahl 1 bedeutet,
R⁷ und R⁸ unabhängig voneinander jeweils für Wasserstoff, oder Cyclopropyl stehen, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können,
sowie ihre Salze, Solvate und Solvate der Salze.

Bevorzugt sind im Rahmen der vorliegenden Erfindung auch Verbindungen der Formel (I) wie in den Ansprüchen definiert, in welcher
- A: für CR³ steht,
wobei
R³ für Wasserstoff steht,
- L: für eine Gruppe #-CR^{4A}R^{4B}-(CR^{5A}R^{5B})ₘ-## steht,
wobei
- #¹: für die Anknüpfstelle an die Carbonylgruppe steht,
- #²: für die Anknüpfstelle an den Pyrimidinring steht,
- m: für eine Zahl 0 steht,
- R^{4A}: für Wasserstoff, Fluor, Methyl, Ethyl, Hydroxy oder Amino steht,
- R^{4B}: für eine Gruppe der Formel -M-R⁶ steht,
und worin
M für eine Bindung steht,
R⁶ für -(C=O)ᵣ-NR⁷R⁸, Phenyl, Thiazolyl, Triazolyl, Oxadiazolyl, Thiadiazolyl oder Pyrimidinyl steht,
worin
r die Zahl 1 bedeutet,
R⁷ und R⁸ unabhängig voneinander jeweils für Wasserstoff, oder Cyclopropyl stehen, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können,
sowie ihre Salze, Solvate und Solvate der Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 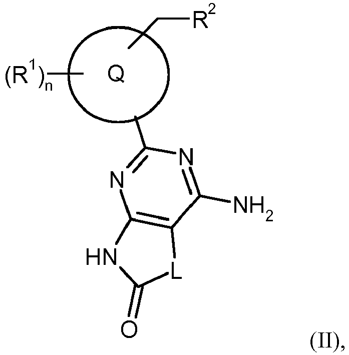 in welcher n, L, Q, R¹ und R² jeweils die oben angegebenen Bedeutungen haben,
   umsetzt, diese dann mit iso-Pentylnitrit und einem Halogen-Äquivalent in eine Verbindung der Formel (I-A) 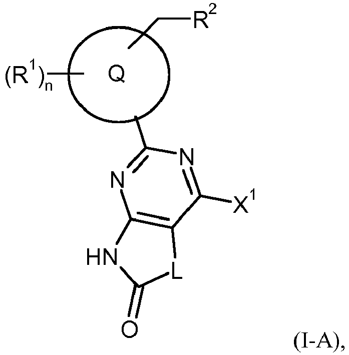 in welcher n, L, Q, R¹ und R² jeweils die oben angegebenen Bedeutungen haben und
   - X¹: für Brom oder Iod steht,
   überführt,
   oder
[B] eine Verbindung der Formel (I-A) in einen inerten Lösungsmittel in Gegenwart eines geeigneten Übergangsmetallkatalysators zu einer Verbindung der Formel (I-B) 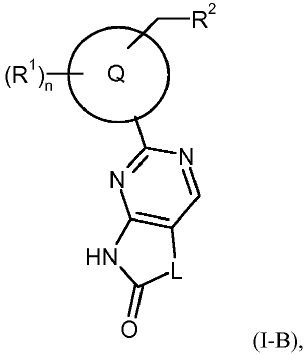 in welcher n, L, Q, R¹ und R² jeweils die oben angegebenen Bedeutungen haben, umsetzt,
   oder
[C] eine Verbindung der Formel (I-A) in einem inerten Lösungsmittel in Gegenwart eines geeigneten Übergangsmetallkatalysators mit einer Verbindung der Formel (III-A), (III-B) bzw. (III-C) 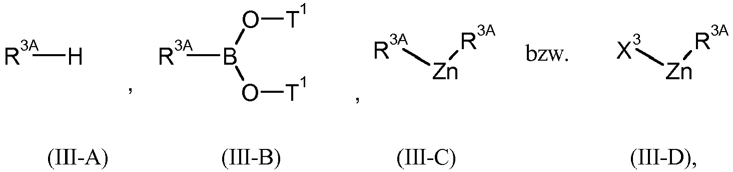 in welchen
   - R^{3A}: für Halogen, Difluormethyl, Trifluormethyl, (C₁-C₄)-Alkyl, (C₂-C₄)-Alkenyl, (C₂-C₄)-Alkinyl, Cyclopropyl, Cyclobutyl, Phenyl oder 5- oder 6-gliedriges Heteroaryl steht,
   worin (C₁-C₄)-Alkyl, (C₂-C₄)-Alkenyl, (C₂-C₄)-Alkinyl, Phenyl und 5- oder 6-gliedriges Heteroaryl mit 1 bis 3 Substituenten unabhängig voneinander ausgewählt aus der Gruppe mit Fluor, Difluormethyl, Trifluormethyl, (C₁-C₄)-Alkyl, Difluormethoxy, Trifluormethoxy, (C₁-C₄)-Alkoxy, (C₁-C₄)-Alkoxycarbonyl, Cyclopropyl und Cyclobutyl substituiert sein kann,
   - T¹: für Wasserstoff oder (C₁-C₄)-Alkyl steht, oder beide Reste R¹¹ zusammen eine -C(CH₃)₂-C(CH₃)₂-Brücke bilden,
   und
   - X³: für Brom oder Iod steht,
   zu einer Verbindung der Formel (I-C) 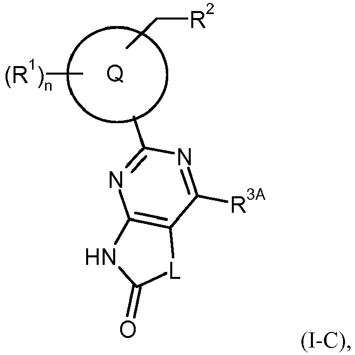 in welcher n, L, Q, R¹, R² und R^{3A} jeweils die oben angegebenen Bedeutungen haben, umsetzt,
   oder
[D] in einem inerten Lösungsmittel in Gegenwart einer geeigneten Base mit Hydrazinhydrat zu einer Verbindung der Formel (IV) 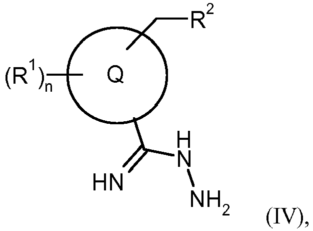 in welcher n, L, Q, R¹ und R² jeweils die oben genannten Bedeutungen haben, umsetzt, diese dann in einem inerten Lösungsmittel mit einer Verbindung der Formel (V) 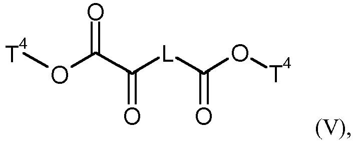 in welcher L die oben angegebene Bedeutung hat und
   - T⁴: für (C₁-C₄)-Alkyl steht,
   zu einer Verbindung der Formel (VI) 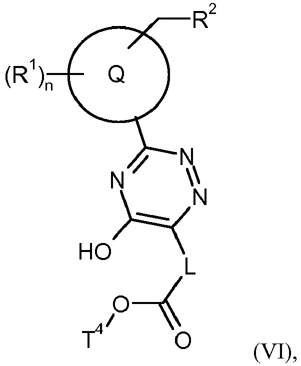 in welcher n, L, Q, R¹, R² und T⁴ jeweils die oben angegebenen Bedeutungen haben, reagiert, diese anschliessend mit Phosphorylchlorid in eine Verbindung der Formel (VII) 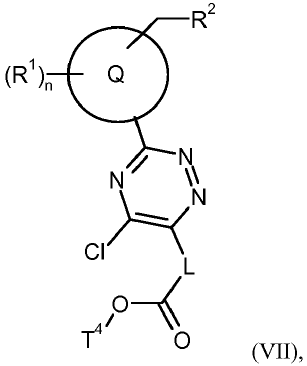 in welcher n, L, Q, R¹, R² und T⁴ jeweils die oben angegebenen Bedeutungen haben, überführt, und diese direkt mit Ammoniak zu einer Verbindung der Formel (VIII) 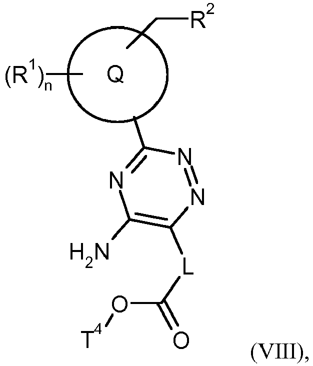 in welcher n, L, Q, R¹, R² und T⁴ jeweils die oben angegebenen Bedeutungen haben, umsetzt, und schliesslich in einem inerten Lösungsmittel gegebenenfalls in Gegenwart einer geeigneten Base zu einer Verbindung der Formel (I-D) 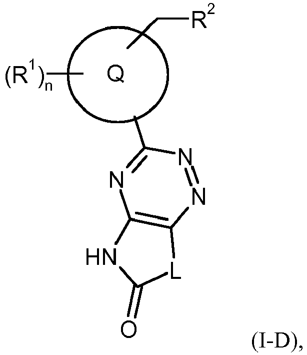 in welcher n, L, Q, R¹ und R² jeweils die oben angegebenen Bedeutungen haben, cyclisiert,
   oder
[E] eine Verbindung der Formel (X) 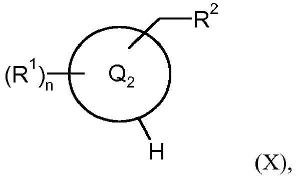 in welcher n, R¹ und R² jeweils die oben angegebenen Bedeutungen haben und der Ring Q² für eine Gruppe der Formel 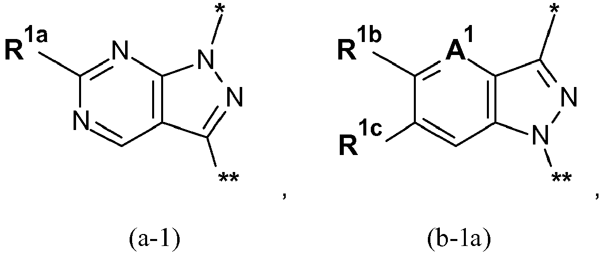 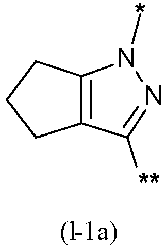 steht, wobei
   * für die Anknüpfungsstelle an -CH₂-R² steht,
   ** für die Anknüpfungsstelle das Wasserstoffatom steht,
   R^{1a}, R^{1b}, R^{1c} und A1 jeweils die oben angegebenen Bedeutungen haben, in einem inerten Lösungsmittel gegebenenfalls in Gegenwart einer geeigneten Base mit einer Verbindung der Formel (XI)

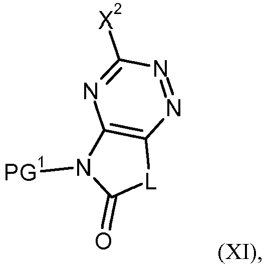

   in welcher L die oben angegebene Bedeutung hat,
   X² für Chlor oder Brom steht und
   PG¹ für eine geeignete Aminoschutzgruppe, insbesondere p-Methoxybenzyl steht, zu einer Verbindung der Formel (XII) 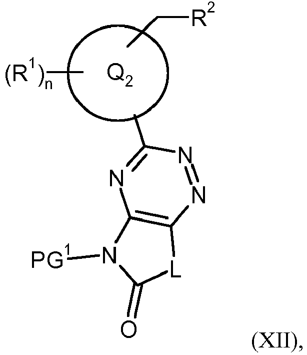
   in welcher n, L, Q₂, R¹, R² und PG¹ jeweils die oben angegebenen Bedeutungen haben, umsetzt, von dieser anschliessend die Schutzgruppe PG¹ zu einer Verbindung der Formel (I-E) 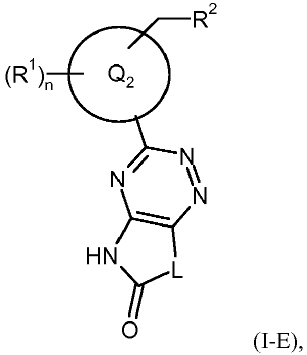 in welcher n, L, Q₂, R¹ und R² jeweils die oben angegebenen Bedeutungen haben, abspaltet
   und gegebenenfalls die resultierenden Verbindungen der Formeln (I-A), (I-B), (I-C), (I-D) und (I-E) gegebenenfalls mit den entsprechenden (i) Lösungsmitteln und/oder (ii) Säuren oder Basen in ihre Solvate, Salze und/oder Solvate der Salze überführt.

Die Verbindungen der Formeln ((I-A), (I-B), (I-C), (I-D) und (I-E) bilden zusammen die Gruppe der erfindungsgemäßen Verbindungen der Formel (I) so wie in den Ansprüchen definiert. Der Verfahrensschritt (II) → (I-A) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (II) → (I-A) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (II) → (I-A) eignen sich beispielsweise Diiodmethan, eine Mischung aus Cäsiumiodid, Iod und Kupfer-(I)-iodid oder Kupfer -(II)-bromid.

Der Verfahrensschritt (II) → (I-A) erfolgt im Fall von Diiodmethan als Halogenquelle mit einem Molverhältnis von 10 bis 30 Mol Isopentylnitrit und 10 bis 30 Mol des Iod-Äquivalents bezogen auf 1 Mol der Verbindung der Formel (II).

Inerte Lösungsmittel für den Verfahrensschritt (I-A) → (I-B) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion (I-A) → (I-B) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (I-A) → (I-B) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Der Verfahrensschritt (I-A) + (III-A) bzw. (III-B) bzw. (III-C) bzw. (III-D) → (I-C) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Gegebenenfalls kann die Umsetzung (I-A) + (III-A) bzw. (III-B) bzw. (III-C) bzw. (III-D) → (I-C) in Gegenwart eines geeigneten Palladium- und/oder Kupferkatalysators erfolgen. Als PalladiumKatalysator ist beispielsweise Palladium auf Aktivkohle, Palladium(II)-acetat, Tetrakis-(triphenylphosphin)-palladium(O), Bis-(triphenylphosphin)-palladium(II)-chlorid, Bis-(acetonitril)-palladium(II)-chlorid, [1,1'-Bis(diphenylphosphino)ferrocen]dichlorpalladium(II) und entsprechender Dichlormethan-Komplex, gegebenenfalls in Verbindung mit zusätzlichen Phosphanliganden wie beispielsweise (2-Biphenyl)di-*tert*.-butylphosphin, Dicyclohexyl[2',4',6'-tris(1-methylethyl)biphenyl-2-yl]phosphan (XPHOS), Bis(2-phenylphosphinophenyl)ether (DPEphos) or 4,5-Bis(diphenyl-phosphino)-9,9-dimethylxanthen (Xantphos) [vgl. z.B. Hassan J. et al., Chem. Rev. 102, 1359-1469 (2002)] geeignet. Als Kupfer-Katalysatoren eignen sich beispielsweise Kupferbronze, Kupfer-(I)-oxid, Kupfer-(I)-iodid oder Kupfer-(I)-bromid.

Die Umsetzung (I-A) + (III-A) bzw. (III-B) bzw. (III-C) bzw. (III-D) → (I-C) erfolgt in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N-*Methylmorpholin, *N-*Methylpiperidin, *N*,*N-*Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natriumhydrid oder Cäsiumcarbonat verwendet.

Die Reaktion (I-A) + (III-A) bzw. (III-B) bzw. (III-C) bzw. (III-D) → (I-C) wird im Allgemeinen in einem Temperaturbereich von 0°C bis +200°C, bevorzugt bei +10°C bis +150°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Ist der Rest R^{3A} ungesättigt, kann dieser anschließend vollständig oder teilweise gesättigt werden. Die Reduktion erfolgt mit Wasserstoff in Verbindung mit Übergangsmetallkatalysatoren wie beispielsweise Palladium (10% auf Aktivkohle), Raney-Nickel oder Palladiumhydroxid. Die Reduktion erfolgt im Allgemeinen in einem Temperaturbereich von +20°C bis +50°C. Die Umsetzung kann bei normalem oder erhöhtem Druck durchgeführt werden (z.B. im Bereich von 1 bis 150 bar). Im Allgemeinen arbeitet man bei 80 bis 100 bar.

Die Umsetzung (VI) → (VII) kann in einem unter den Reaktionsbedingungen inerten Lösungsmittel oder ohne Lösungsmittel erfolgen. Bevorzugtes Lösungsmittel ist Sulfolan.

Die Reaktion (VI) → (VII) erfolgt im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (VI) → (VII) ohne Lösungsmittel in einem Temperaturbereich von 0°C bis +50°C bei Normaldruck.

Der Verfahrensschritt (VII) → (VIII) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Die Reaktion (VII) → (VIII) erfolgt im Allgemeinen in einem Temperaturbereich von +20°C bis +100°C, bevorzugt von +40°C bis +70°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Cyclisierung (VIII) → (I-D) erfolgt in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran (THF), Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N-*Methylpyrrolidon (NMP), Pyridin, Acetonitril oder Sulfolan. Ebenso ist es möglich, Gemische der genannten Lösungsmittel einzusetzen. Bevorzugt ist THF.

Geeignete Basen für den Verfahrensschritt (VIII) → (I-D) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkali-alkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (VIII) → (I-D) erfolgt im Allgemeinen in einem Temperaturbereich von 0°C bis +50°C, bevorzugt von +10°C bis +30°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Bevorzugt erfolgt die Cyclisierung zu (I-D) direkt bei der Umsetzung (VII) → (VIII) ohne Zugabe weiterer Reagenzien.

In einer alternativen Durchführung des Verfahrens [D] kann die Umwandlung (IV) + (V) → (VI) → (VII) → (VIII) → (I-D) ohne Isolierung der Zwischenstufen durchgeführt werden.

Bevorzugt erfolgen die Umsetzungen (VI) → (VII) → (VIII) → (I-D) ohne Isolierung der Zwischenstufen.

Inerte Lösungsmittel für den Verfahrensschritt (IV) + (V) → (VI) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Die Reaktion (IV) + (V) → (VI) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Der Verfahrensschritt (X) + (XI) → (XII) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist NMP.

Die Umsetzung (X) + (XI) → (XII) erfolgt in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid. Bevorzugt wird Natriumhydrid verwendet.

Die Reaktion (X) + (XI) → (XII) wird im Allgemeinen in einem Temperaturbereich von +20°C bis +150°C, bevorzugt bei +40°C bis +100°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Abspaltung der Schutzgruppe PG¹ erfolgt nach den dem Fachmann bekannten Methoden, siehe z.B. T.W. Greene und P.G.M. Wuts, Protective Groups in Organic Synthesis, Wiley, New York, 1999.

In einer alternativen Verfahrensvariante kann der Verfahrensschritt (X) + (XI) auch ohne Schutzgruppe PG1 erfolgen, hierbei erfolgt die Umsetzung bevorzugt ohne Base in NMP bei 80°C bis 100°C.

Die beschriebenen Herstellverfahren können durch die folgenden Syntheseschemata (Schema 1 bis 4) beispielhaft verdeutlicht werden: 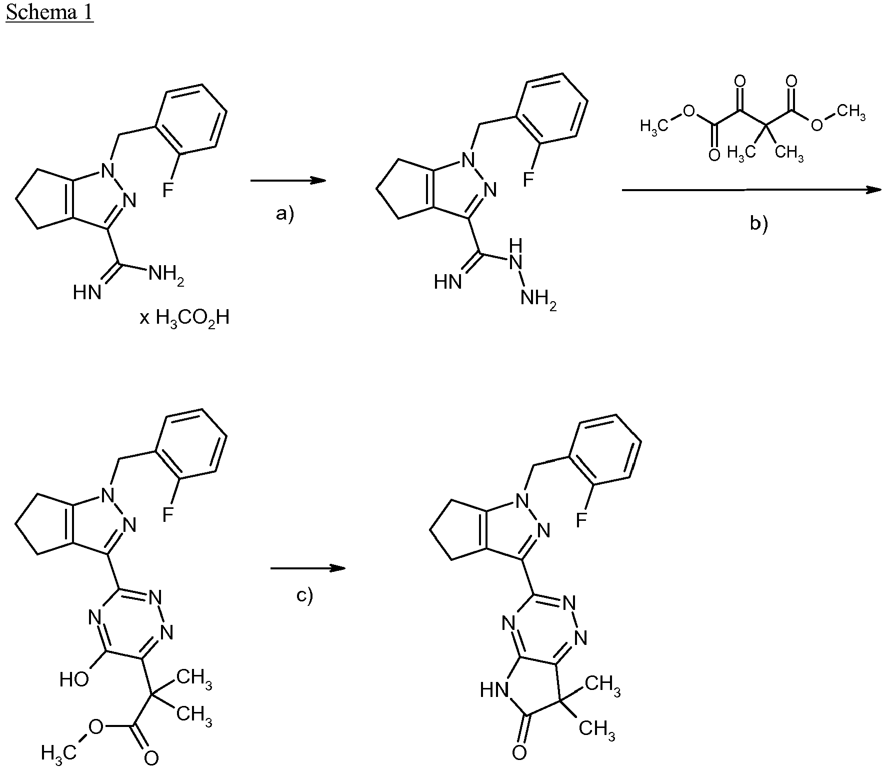 [a): Hydrazinhydrat, NEt₃, EtOH b): EtOH c): 1. POCl₃; 2. konz. NH₃, Acetonitril]. 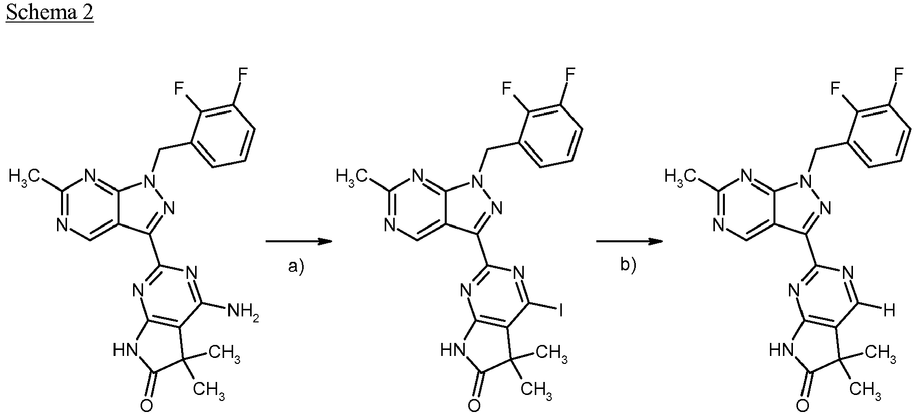 [a): Diiodmethan, iso-Pentylnitrit; b): Pd/C, Wasserstoff, DMF]. 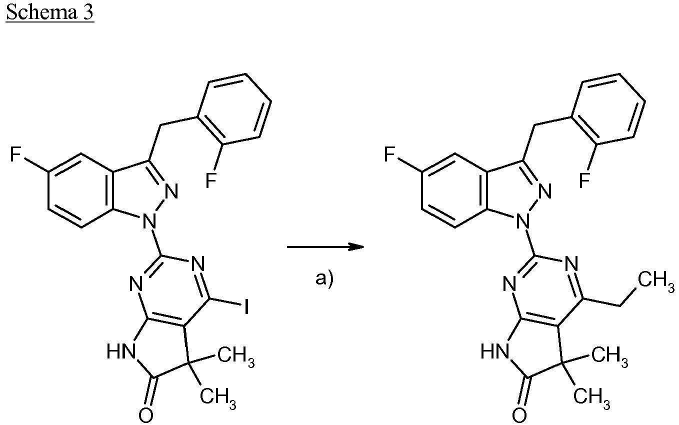 [a): Diethylzink, PdCl₂(dppf), Dioxan, 90°C]. 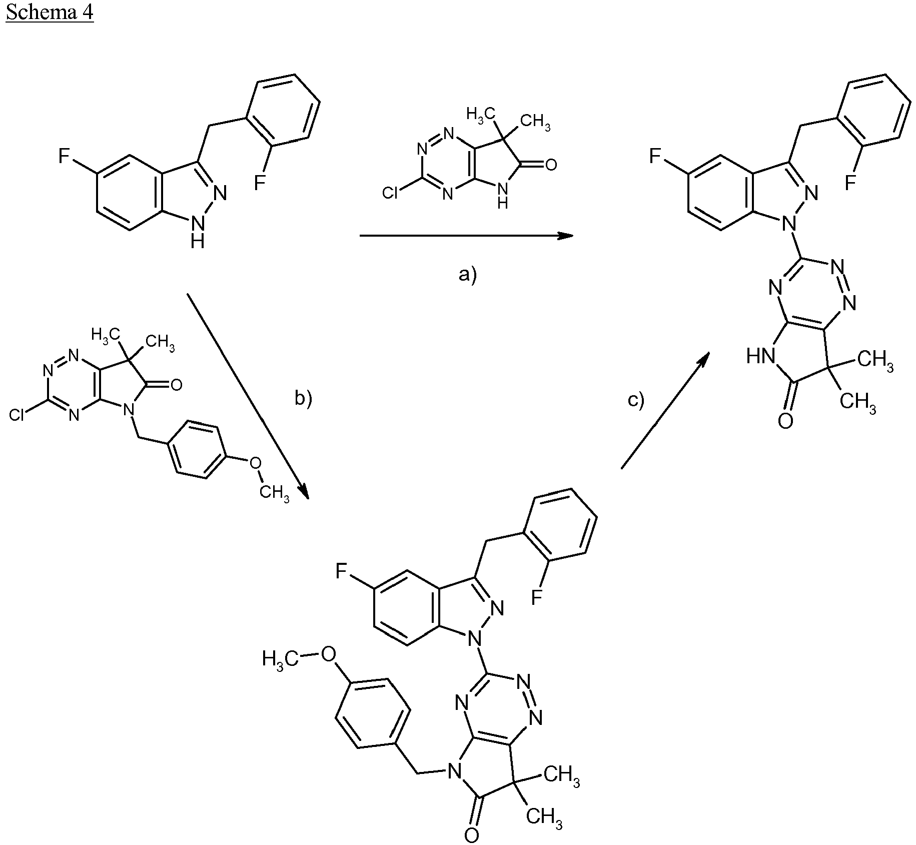 [a): NMP, 80°C - 100°C, b): NaH, NMP, RT - 80°C, c): Ammoniumcer(IV)nitrat, Acetonitril, Wasser, 0°C - RT]

Weitere erfindungsgemäße Verbindungen können gegebenenfalls auch hergestellt werden durch Umwandlungen von funktionellen Gruppen einzelner Substituenten, insbesondere den unter L und R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die Verbindungen der Formel (II) sind literaturbekannt (siehe z.B. WO 2010/065275, WO 2011/ 115804 und WO 2011/149921) oder können in Analogie zu literaturbekannten Verfahen hergestellt werden.

Die Verbindungen der Formel (IV) können hergestellt werden, indem man eine Verbindung der Formel (IX) 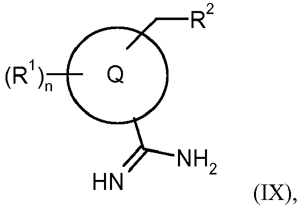 in welcher n, Q, R¹ und R² jeweils die oben genannten Bedeutungen haben,
in einem inerten Lösungsmittel in Gegenwart einer geeigneten Base mit Hydrazinhydrat umsetzt.

Inerte Lösungsmittel für den Verfahrensschritt (IX) → (IV) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Ethanol.

Geeignete Basen für den Verfahrensschritt (IX) → (IV) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkali-alkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Triethylamin.

Die Reaktion (IX) → (IV) erfolgt im Allgemeinen in einem Temperaturbereich von 0°C bis +60°C, bevorzugt von +10°C bis +30°C. Die Umsetzung kann bei normalem oder erhöhtem Druck durchgeführt werden (z.B. im Bereich von 0.5 bis 5 bar). Im Allgemeinen arbeitet man bei Normaldruck.

Die Verbindungen der Formel (XI) können wie im vorliegenden experimentellen Teil für die Beispiele 44A bis 48A beschrieben und in Analogie dazu hergestellt werden. Das nachfolgende Schema 5 verdeutlicht die Herstellung beispielhaft: 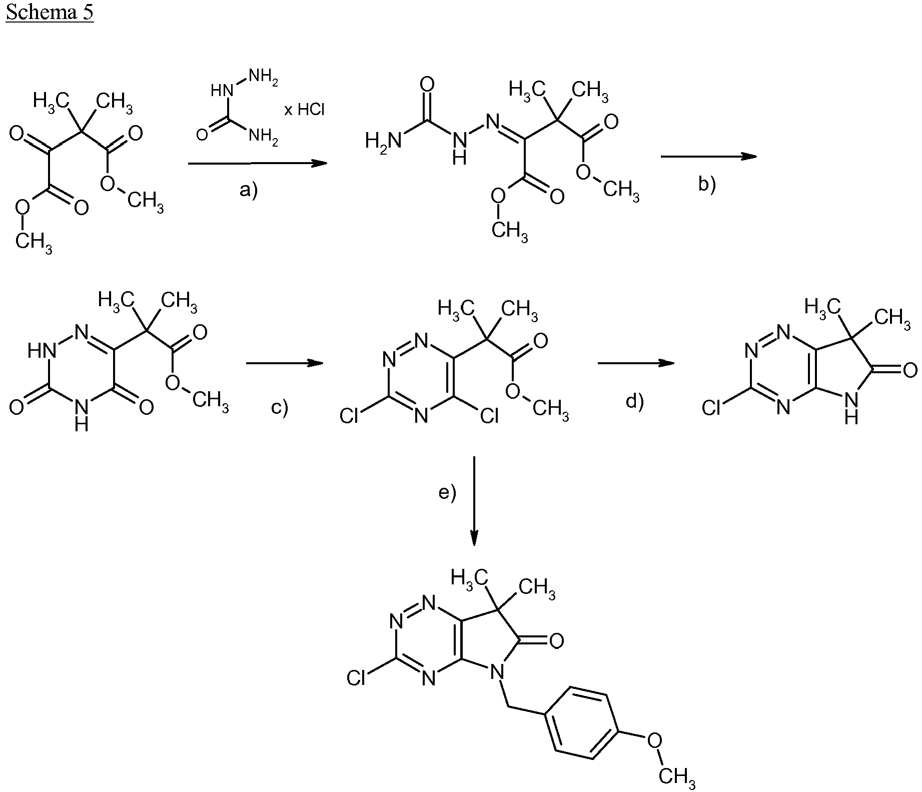 [a): NaOAc, Wasser, RT: b): NaOMe, MeOH, Rückfluß: c): POCl₃, DMF, Rückfluß; d): aq. Ammoniak, Dioxan, RT; e): 4-Methoxy-benzylamin, Diisopropylethylamin, THF, 0°C - RT].

Die Verbindungen der Formeln (III-A), (III-B), (III-C), (V) und (IX) sind kommerziell erhältlich, literaturbekannt (vgl. z.B. WO 2010/065275, WO 2011/ 115804 und WO 2011/149921) oder können in Analogie zu literaturbekannten Verfahren hergestellt werden.

Die erfindungsgemäßen Verbindungen wirken als potente Stimulatoren der löslichen Guanylatcyclase und Inhibitoren von Phosphodiesterase-5, besitzen wertvolle pharmakologische Eigenschaften, und weisen ein verbessertes therapeutisches Profil auf, wie beispielsweise hinsichtlich ihrer *in-vivo* Eigenschaften und/oder ihres pharmakokinetischen Verhaltens und/oder metabolischen Profils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.Die erfindungsgemäßen Verbindungen eignen sich auch zur Behandlung der Muskeldystrophie, wie der Muskeldystrophie Becker-Kiener (BMD) und Muskeldystrophie Duchenne (DMD).

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF). Außerdem können die genannten Verbindungen als Bronchodilatatoren eingesetzt werden.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankengen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Offenbarungsgegenstand ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen. Weiterhin offenbart ist ein Verfahren zur Behandlung und/oder Prophylaxe von Herzinsuffizienz, Angina pectoris, Hypertonie, pulmonaler Hypertonie, Ischämien,

Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

Beispiele der vorliegenden Erfindung sind nur solche, die unter die Formel (I) wie in den Ansprüchen definiert fallen.

### Abkürzungen und Akronyme:

- aq.: wässrige Lösung
- ber.: berechnet
- br s: breites Singulett (bei NMR)
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: gefunden
- h: Stunde(n)
- HPLC: Hochdruck-, Hochleistungsflüssigchromatographie
- HRMS: hochaufgelöste Massenspektrometrie
- konz.: konzentriert
- LC-MS: Flüssigchromatographie-gekoppelte Massenspektrometrie
- Me: Methyl
- min: Minute(n)
- MS: Massenspektrometrie
- NMR: Kernresonanzspektrometrie
- PdCl₂(dppf)xCH₂Cl₂: [1,1'-Bis(diphenylphosphin)ferrocen]dichlorpalladium(II)- Dichlormethan-Komplex
- Ph: Phenyl
- RT: Raumtemperatur
- Rt: Retentionszeit (bei HPLC)
- THF: Tetrahydrofuran
- UV: Ultraviolett-Spektrometrie
- v/v: Volumen zu Volumen-Verhältnis (einer Lösung)

### LC/MS-Methoden:

### Methode 1:

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 208 - 400 nm.

### Methode 2:

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 3:

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 4:

Gerätetyp MS: Waters (Micromass) Quattro Micro; Gerätetyp HPLC: Agilent 1100 Serie; Säule : Thermo Hypersil GOLD 3 µ 20 x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100% A → 3.0 min 10% A → 4.0 min 10% A; Ofen: 50°C; Fluss: 2 ml/min; UV-Detektion: 210 nm

### Ausgangsverbindungen und Intermediate:

### Beispiel 1A

### 5-Chlor-3-(2,3,6-trifluorbenzyl)-1H-indazol-1-carboximidamid

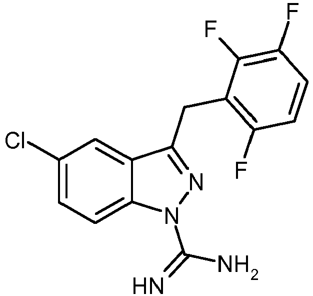

4.60 g (10.96 mmol) 2-[1-(2-Brom-5-chlorphenyl)-2-(2,3,6-trifluorphenyl)ethyliden]-hydrazin-carboximidamid (die Synthese dieser Verbindung ist beschrieben in WO 2010/065275, Example 3, Step B, page 36-37) und 2.09 g (10.96 mmol) Kupfer-(I)-iodid wurden in NMP (150 ml) in einem 1L-Rundkolben vorgelegt, und dann 14 min in einem auf 170°C vorgeheizten Ölbad gerührt. Das Reaktionsgemisch wurde anschließend im Eisbad abgekühlt, mit einem Eis/Wasser-Gemisch (400 ml) versetzt und konzentrierter wäßriger Ammoniak-Lösung (200 mL) hinzugegeben. Nach 15 minütigem Rühren wurde der Feststoff abgesaugt. Der Rückstand wurde in Essigsäureethylester gelöst, zweimal mit Wasser gewaschen, getrocknet und das Lösungsmittel am Rotationsverdampfer entfernt. Es wurden 3.00 g (39 % d. Th., 49%ige Reinheit) der Titelverbindung erhalten. Das Rohprodukt wurde ohne Reinigung weiter umgesetzt.
LC-MS (Methode 1): Rₜ= 0.80 min; MS (ESIpos): m/z = 339 (M+H)⁺

### Beispiel 2A

### Methyl-3,3-dicyan-2,2-dimethylpropanoat

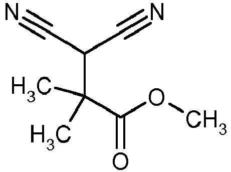

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben und das Reaktionsgemisch wurde über Nacht bei Raumtemperatur gerührt. Danach wurden nochmals 5.876 ml (45.411 mmol) Methyl-2-brom-2-methylpropanoat zugegeben und das Reaktionsgemisch wurde über Nacht auf 50°C erhitzt. Dann wurden abermals 1.762 ml (13.623) mmol) Methyl-2-brom-2-methylpropanoat zugegeben und das Reaktionsgemisch wurde weitere 4h auf 50°C erhitzt. Der Ansatz wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, über Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.
Ausbeute: 6.47 g (85% d. Th.)
¹H-NMR (400 MHz, DMSO-d₆): δ [ppm] = 1.40 (s, 6H), 3.74 (s, 3H), 5.27 (s, 1H).

### Beispiel 3A

### 4-Amino-2-[5-chlor-3-(2,3,6-trifluorbenzyl)-1H-indazol-1-yl]-5,5-dimethyl-5,7-dihydro-6H-pyrrolo[2,3-d]pyrimidin-6-on

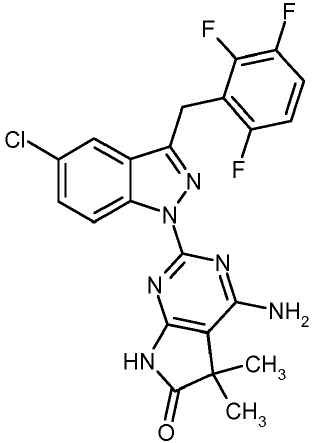

350 mg (0.52 mmol, 49%-ige Reinheit) des Rohprodukts von Beispiel 1A und 301 mg (1.81 mmol) Beispiel 2A wurden in tert.-Butanol (2.3 ml) vorgelegt und 98.6 mg (0.88 mmol) Kalium-tert.-Butylat hinzugegeben. Das Reaktionsgemisch wurde 18 h zum Rückfluss erhitzt. Nach dem Abkühlen wurde das Reaktionsgemisch mit Essigsäureethylester verdünnt und mit ca. 7%-iger wässriger Ammoniumchlorid-Lösung gewaschen. Die organische Phase wurde getrocknet und das Lösungsmittel am Rotationsverdampfer entfernt. Der Rückstand wurde an 150 ml Kieselgel mit Cyclohexan / Essigsäureethylester 1:1 chromatographisch gereinigt. Es wurden 120 mg (38 % d. Th.) der Titelverbindung erhalten.
LC-MS (Methode 2): Rₜ= 1.14 min; MS (ESIpos): m/z = 473 (M+H)⁺
¹H-NMR (400MHz, DMSO-d₆): δ [ppm]= 1.34 (s, 6H), 4.46 (s, 2H), 7.00 (br. s., 2H), 7.16 - 7.25 (m, 1H), 7.42 - 7.51 (m, 1H), 7.51 - 7.58 (m, 1H), 7.90 - 7.94 (m, 1H), 8.83 (d, 1H), 11.10 (s, 1H).

### Beispiel 4A

### 1-(2-Bromphenyl)-2-(2-fluorphenyl)ethanon

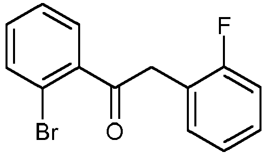

15.0 g (69.8 mmol) 2-Brombenzoesäuremethylester und 11.8 g (76.7 mmol) 2-Fluorphenylessigsäure wurden unter Argonatmosphäre in THF (278 ml) bei -70°C vorgelegt und 174 ml einer 1M Lösung von Natriumhexamethyldisilazan in THF über 20 min. zugetropft. Das Reaktionsgemisch wurde auf 0°C erwärmt, 30 min bei dieser Temperatur gerührt und 1N Salzsäure (278 ml) hinzugefügt. Nach 1 h kräftigem Rühren unter Gasentwicklung (CO₂-Abspaltung) wurde das Reaktionsgemisch mit Essigsäureethylester (500 ml) extrahiert. Die organische Phase wurde zweimal mit gesättigter Natriumhydrogencarbonat-Lösung, einmal mit Wasser und einmal mit gesättigter, wässriger Natriumchloridlösung gewaschen. Nach dem Trocknen und dem Entfernen des Lösungsmittels am Rotationsverdampfer wurden 16.8 g Rückstand (55%ige Reinheit) erhalten. Der Rückstand wurde in THF (140 ml) gelöst, mit 1N Natronlauge (70 ml) versetzt und 4 h bei RT gerührt, um überschüssigen Ester zu verseifen. Das THF wurde am Rotationsverdampfer entfernt, die wässrige Phase mit Diethylether extrahiert und die organische Phase mit gesättigter Natriumhydrogencarbonat-Lösung sowie gesättigter, wässriger Natriumchloridlösung gewaschen. Nach der Trocknung und dem Entfernen des Lösungsmittels wurden 12.2 g Rückstand erhalten (ca. 80%ige Reinheit). Der Rückstand wurde in THF (100 ml) gelöst, mit 1N Natronlauge (40 ml) versetzt und über Nacht bei RT gerührt. Das THF wurde am Rotationsverdampfer entfernt, die wässrige Phase mit Diethylether extrahiert und die organische Phase mit gesättigter Natriumhydrogencarbonat-Lösung sowie gesättigter, wässriger Natriumchloridlösung gewaschen. Nach der Trocknung und dem Entfernen des Lösungsmittels wurden 7.90 g (37 % d. Th.) der Titelverbindung isoliert. ¹H-NMR (400MHz, DMSO-d₆): δ [ppm]= 4.35 (s, 2H), 7.14 - 7.22 (m, 2H), 7.30 - 7.39 (m, 2H), 7.41 - 7.47 (m, 1H), 7.49 - 7.55 (m, 1H), 7.70 - 7.78 (m, 2H).

### Beispiel 5A

### 2-[1-(2-Bromphenyl)-2-(2-fluorphenyl)ethyliden]hydrazincarboximidamid

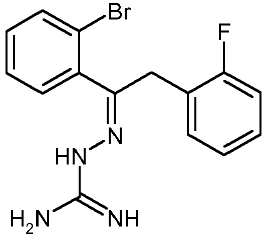

7.80 g (26.6 mmol) Beispiel 4A und 5.88 g (53.2 mmol) Aminoguanidin-Hydrochlorid wurden in Ethylenglykol (193 ml) vorgelegt und 8.50 g (59.9 mmol) Bortrifluorid-Diethylether-Komplex hinzugegeben. Das Reaktionsgemisch wurde 2 h bei 120°C an einer Destillationsbrücke erhitzt. Nach Abkühlung wurden nochmals 5.88 g (53.2 mmol) Aminoguanidin-Hydrochlorid sowie 8.50 g (59.9 mmol) Bortrifluorid-Diethylether-Komplex hinzugegeben und 3h bei 120°C gerührt. Nach dem Abkühlen wurde mit Wasser (750 ml) versetzt und mit 1N Natronlauge ein pH Wert von 11-12 eingestellt. Nach beginnender Kristallbildung wurden 300 g Eis hinzugegeben, 5 min gerührt und der Feststoff dann abfiltriert. Der Rückstand wurde zuerst mit Wasser, dann mit Pentan gewaschen und im Vakuum getrocknet. Es wurden 8.30 g (87 % d. Th.) der Titelverbindung erhalten.
LC-MS (Methode 1): Rₜ= 0.78 min; MS (ESIpos): m/z (Br-Isotop 1 + 2) = 349 + 351 (M+H)⁺

### Beispiel 6A

### 3-(2-Fluorbenzyl)-1H-indazol-1-carboximidamid

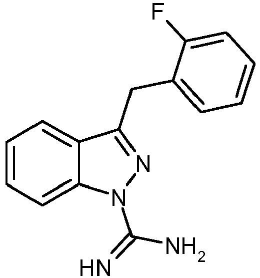

320 ml N-Methylpyrrolidon wurden auf 140°C erhitzt, 8.20 g (23.5 mmol) Beispiel 5A und 4.47 g (23.5 mmol) Kupfer-(I)-iodid hinzugegeben und 14 min bei 170° Badtemperatur gerührt. Das Reaktionsgemisch wurde anschließend langsam auf 1 L Eiswasser gegeben und konzentrierte wäßrige Ammoniak-Lösung (350 mL) hinzugegeben. Nach 5 minütigem Rühren wurde 1 L Essigsäureethylester hinzugegeben und das Gemisch 10 min gerührt. Die wässrige Phase wurde einmal mit Essigsäureethylester extrahiert und die vereinigten organischen Phasen dreimal mit Wasser gewaschen. Nach dem Trocknen und dem Entfernen des Lösungsmittels am Rotationsverdampfer wurden 7.10 g (74 % d.Th., 66%ige Reinheit) der Titelverbindung erhalten. Das Rohprodukt wurde ohne Reinigung weiter umgesetzt.
LC-MS (Methode 1): Rₜ= 0.68 min; MS (ESIpos): m/z = 269 (M+H)⁺

### Beispiel 7A

### 4-Amino-2-[3-(2-fluorbenzyl)-1H-indazol-1-yl]-5,5-dimethyl-5,7-dihydro-6H-pyrrolo[2,3-d]pyrimidin-6-on

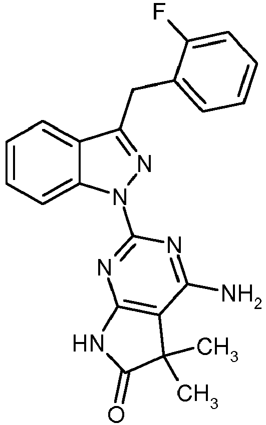

7.00 g (17.2 mmol, 66 %-ige Reinheit) des Rohprodukts von Beispiel 6A und 5.72 g (34.4 mmol) Beispiel 2A wurden in tert.-Butanol (77.0 ml) vorgelegt und 3.29 g (29.3 mmol) Kalium-tert.-Butylat hinzugegeben. Das Reaktionsgemisch wurde 18 h zum Rückfluss erhitzt. Nach dem Abkühlen wurde das Reaktionsgemisch mit Essigsäureethylester verdünnt und mit ca. 7%-iger wässriger Ammoniumchlorid-Lösung gewaschen. Die organische Phase wurde mit gesättigter, wässriger Natriumchloridlösung gewaschen, getrocknet und das Lösungsmittel am Rotationsverdampfer entfernt. Der Rückstand wurde an 600 ml Kieselgel mit Cyclohexan / Essigsäureethylester 2:3 chromatographisch gereinigt. Es wurden 2.20 g (29 % d. Th.) der Titelverbindung als Feststoff erhalten.
LC-MS (Methode 3): Rₜ= 2.19 min; MS (ESIpos): m/z = 403 (M+H)⁺
¹H-NMR (400MHz, DMSO-d₆): δ [ppm]= 1.35 (s, 6H), 4.39 (s, 2H), 6.97 (br. s., 2H), 7.11 - 7.18 (m, 1H), 7.21 (d, 1H), 7.24 - 7.33 (m, 2H), 7.36 (t, 1H), 7.50 (t, 1H), 7.70 (d, 1H), 8.82 (d, 1H), 11.10 (s, 1H).

### Beispiel 8A

### 1-(2-Brom-5-fluorphenyl)-2-(2-fluorphenyl)ethanon

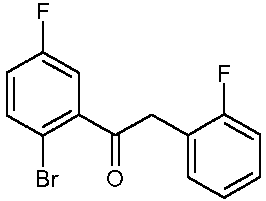

15.0 g (63.1 mmol) 2-Brom-5-fluorbenzoesäuremethylester und 11.7 g (75.7 mmol) 2-Fluorphenylessigsäure wurden unter Argonatmosphäre in THF (278 ml) bei -70°C vorgelegt und eine 1M Lösung von Nahexamethyldisilazan in THF (158 ml) über 20 min. zugetropft. Das Reaktionsgemisch wurde 30 min. bei dieser Temperatur gerührt, auf 0°C erwärmt, weitere 30 min bei 0°C gerührt und dann 1N Salzsäure (251 ml) hinzugefügt. Nach 1 h kräftigem Rühren unter Gasentwicklung (CO₂-Abspaltung) wurde das Reaktionsgemisch mit Essigsäureethylester (700 ml) extrahiert. Die organische Phase wurde zweimal mit gesättigter wässriger Natriumhydrogencarbonat-Lösung, einmal mit Wasser und einmal mit gesättigter, wässriger Natriumchloridlösung gewaschen. Nach dem Trocknen und dem Entfernen des Lösungsmittels am Rotationsverdampfer wurden 16.9 g Rückstand erhalten (50%ige Reinheit). Der Rückstand wurde in THF (200 ml) gelöst, mit 1N Natronlauge (100 ml) versetzt und über Nacht bei RT gerührt. Das THF wurde am Rotationsverdampfer entfernt, die wässrige Phase mit Diethylether extrahiert und die organische Phase mit gesättigter, wässriger Natriumhydrogencarbonat-Lösung sowie gesättigter, wässriger Natriumchloridlösung gewaschen. Nach dem Trocknen und dem Entfernen des Lösungsmittels am Rotationsverdampfer wurden 9.10 g (42 % d.Th.) der Titelverbindung als Feststoff isoliert.
¹H-NMR (400MHz, DMSO-d₆): δ [ppm]= 4.36 (s, 2H), 7.14 - 7.24 (m, 2H), 7.30 - 7.39 (m, 3H), 7.71 - 7.80 (m, 2H).

### Beispiel 9A

### 2-[1-(2-Brom-5-fluorphenyl)-2-(2-fluorphenyl)ethyliden]hydrazincarboximidamid

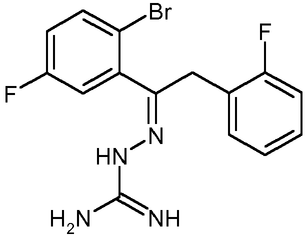

9.00 g (28.9 mmol) Beispiel 8A und 6.40 g (58.9 mmol) Aminoguanidin-Hydrochlorid wurden in Ethylenglykol (207 ml) vorgelegt und 9.24 g (65.1 mmol) Bortrifluorid-Diethylether-Komplex hinzugegeben. Das Reaktionsgemisch wurde 2 h bei 120°C an einer Destillationsbrücke erhitzt. Nach Abkühlung wurden nochmals 6.40 g (58.9 mmol) Aminoguanidin-Hydrochlorid sowie 9.24 g (65.1 mmol) Bortrifluorid-Diethylether-Komplex hinzugegeben und 3h bei 120°C gerührt. Nach dem Abkühlen wurde das Reaktionsgemisch langsam auf Wasser (800 ml) gegeben und mit 1N Natronlauge ein pH Wert von 11-12 eingestellt. Nach beginnender Niederschlagsbildung wurden 300 g Eis hinzugegeben und 15 min gerührt. Wegen der klebrigen Beschaffenheit des Niederschlags wurde das Wasser abdekantiert und der Rückstand noch zweimal mit je 200 ml Wasser ausgerührt. Der klebrige Niederschlag wurde in Diethylether gelöst, mit Wasser gewaschen, die organische Phase getrocknet, das Lösungsmittel am Rotationsverdampfer entfernt und 6.00 g (54 % d. Th.) der Titelverbindung als Schaum isoliert.
LC-MS (Methode 1): Rₜ=0.80 min; MS (ESIpos): m/z = 367 + 369 (M+H)⁺

### Beispiel 10A

### 5-Fluor-3-(2-fluorbenzyl)-1H-indazol-1-carboximidamid

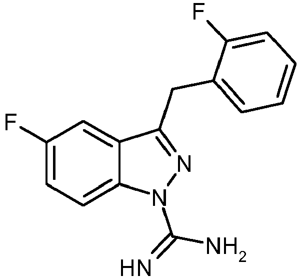

222 ml N-Methylpyrrolidon wurden auf 140°C erhitzt, 6.00 g (16.3 mmol) von Beispiel 9A und 3.11 g (16.3 mmol) Kupfer-(I)-iodid hinzugegeben und 14 min bei 170°C Badtemperatur gerührt. Das Reaktionsgemisch wurde anschließend langsam auf 700 ml Eiswasser gegeben und konzentrierte, wäßrige Ammoniak-Lösung (230 mL) hinzugegeben. Nach 5 minütigem Rühren wurde 700 ml Essigsäureethylester hinzugegeben und 10 min gerührt. Die wässrige Phase wurde noch einmal mit Essigsäureethylester extrahiert und die vereinten organischen Phasen dreimal mit Wasser gewaschen. Nach dem Trocknen und dem Entfernen des Lösungsmittels am Rotationsverdampfer wurden 6.00 g (64 % d.Th., 50%ige Reinheit) Produkt erhalten. Das Rohprodukt wurde ohne Reinigung weiter umgesetzt.
LC-MS (Methode 3): Rₜ= 1.60 min; MS (ESIpos): m/z = 287 (M+H)⁺

### Beispiel 11A

### 4-Amino-2-[5-fluor-3-(2-fluorbenzyl)-1H-indazol-1-yl]-5,5-dimethyl-5,7-dihydro-6H-pyrrolo[2,3-d]pyrimidin-6-on

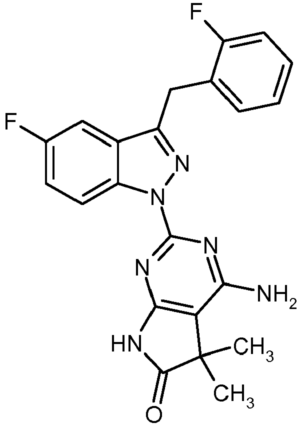

6.00 g (ca. 10.5 mmol, 50%-ige Reinheit) des Rohprodukts von Beispiel 10A und 5.22 g (31.4 mmol) Beispiel 2A wurden in tert.-Butanol (46.0 ml) vorgelegt und 2.00 g (17.8 mmol) Kalium-tert.-Butylat hinzugegeben. Das Reaktionsgemisch wurde 18 h zum Rückfluss erhitzt. Nach dem Abkühlen wurde mit Essigsäureethylester verdünnt und mit ca. 7%-iger wässriger Ammoniumchlorid-Lösung extrahiert. Die organische Phase wurde mit gesättigter, wässriger Natriumchloridlösung gewaschen, getrocknet und das Lösungsmittel am Rotationsverdampfer entfernt. Der Rückstand wurde an 600 ml Kieselgel mit Cyclohexan / Essigsäureethylester 2:3 chromatographisch gereinigt. Die produkthaltigen Fraktionen wurden eingeengt und mit ca. 20 ml Diethylether verrührt, abgesaugt und mit Diethylether gewaschen. Es wurden 1.80 g (37 % d. Th.) der Titelverbindung als Feststoff erhalten.
LC-MS (Methode 1): Rₜ=1.00 min; MS (ESIpos): m/z = 421 (M+H)⁺

### Beispiel 12A

### 3,5-Difluorpyridin-2-carbonylchlorid

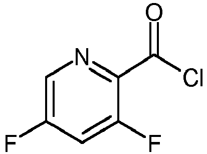

Eine Suspension von 5.00 g (31.4 mmol) 3,5-Difluorpyridin-2-carbonsäure in Thionylchlorid (21 mL) wurde 5 h zum Rückfluss erhitzt. Die Lösung wurde eingeengt, der Rückstand zweimal in wenig Toluol aufgenommen und wieder eingeengt. Man erhielt 3.80 g eines Feststoffs, der ohne weitere Reinigung direkt weiter umgesetzt wurde.

### Beispiel 13A

### Methyl-3-(3,5-difluorpyridin-2-yl)-2-(2-fluorphenyl)-3-oxopropanoat

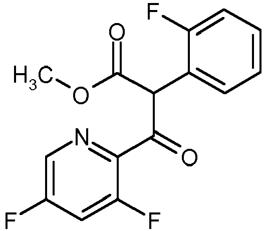

In THF (30 mL) wurden unter Argon 21.4 mL (21.4 mmol) Lithiumhexamethyldisilazid (1.0 M in THF) vorgelegt und bei -78°C eine Lösung von 3.00 g (17.8 mmol) 2-Fluorphenylessigsäuremethylester in THF (15 mL) zugetropft. Die Reaktionsmischung wurde 1 h bei -78°C gerührt und anschließend eine Lösung von 3.80 g (21.4 mmol) der Verbindung aus Beispiel 12A in THF (15 mL) zugetropft. Die Lösung wurde 1h bei -78°C gerührt, danach auf RT gebracht und portionsweise mit gesättigter wässriger Ammoniumchlorid-Lösung versetzt. Die Mischung wurde mit Wasser verdünnt und zweimal mit Essigsäureethylester extrahiert. Die vereinigten organischen Phasen wurden über Natriumsulfat getrocknet, filtriert und eingeengt. Der Rückstand wurde mit MTBE verrührt, der Feststoff abfiltriert und das Filtrat eingeengt. Kieselgelchromatographie (Laufmittel: Cyclohexan-Essigsäureethylester 30:1, 20:1) des Rückstands lieferte 3.66 g (87%-ige Reinheit, 57 % d. Th.) der Titelverbindung. Das Rohprodukt wurde ohne weitere Reinigung umgesetzt.
LC-MS (Methode 1): Rₜ= 1.05 min; MS (ESIpos): m/z = 310 (M+H)⁺
¹H-NMR (400 MHz, DMSO-d₆): δ = 3.66 (s, 3H), 6.25 (s, 1H), 7.20 - 7.28 (m, 4H), 7.31 - 7.38 (m, 1H), 8.15 - 8.23 (m, 1H), 8.68 - 8.71 (m, 1H).

### Beispiel 14A

### 1-(3,5-Difluorpyridin-2-yl)-2-(2-fluorphenyl)ethanon

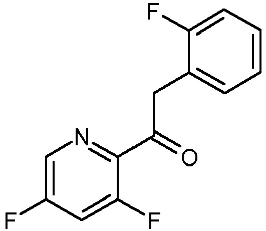

In DMSO (37 mL) wurden 11.65 g (37.67 mmol) der Verbindung aus Beispiel 13A vorgelegt. Anschließend wurden 2.42 g (41.44 mmol) Natriumchlorid sowie Wasser (7 mL) zugefügt und die Mischung 30 min bei 150°C in der Mikrowelle gerührt. Die Reaktionsmischung wurde mit Essigsäureethylester verdünnt, die organische Phase dreimal mit Wasser sowie einmal mit gesättigter wässriger Natriumchlorid-Lösung gewaschen, über Natriumsulfat getrocknet, filtriert und eingeengt. Man erhielt 9.07 g (89%ig, 85 % d. Th.) der gewünschten Verbindung als Feststoff, der ohne weitere Reinigung umgesetzt wurde.
LC-MS (Methode 1): Rₜ= 1.05 min; MS (ESIpos): m/z = 252 (M+H)⁺
¹H-NMR (400 MHz, DMSO-d₆): δ = 4.53 (s, 2H), 7.15 - 7.22 (m, 2H), 7.30 - 7.37 (m, 2H), 8.11 - 8.18 (m, 1H), 8.70 - 8.72 (m, 1H).

### Beispiel 15A

### 6-Fluor-3-(2-fluorbenzyl)-1H-pyrazolo[4,3-b]pyridin

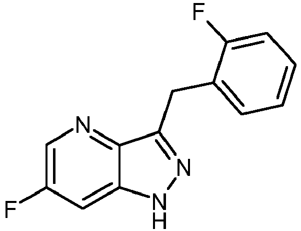

In Pyridin (84 mL) wurden 9.07 g (32.4 mmol) der Verbindung aus Beispiel 14A vorgelegt. Anschließend wurden 8.10 g (162 mmol) Hydrazinhydrat sowie 19.8 mg (0.162 mmol) 4-Dimethylaminopyridin zugefügt und die Mischung 30 min zum Rückfluss erhitzt. Die Reaktionsmischung wurde bei RT mit Essigsäureethylester verdünnt und viermal mit 10%-iger wässriger Zitronensäure-Lösung gewaschen. Die organische Phase wurde anschließend mit gesättigter wässriger Natriumchlorid-Lösung gewaschen, über Natriumsulfat getrocknet, filtriert und eingeengt. Der Rückstand wurde mit MTBE versetzt und der Feststoff abfiltriert. Dieser wurde im Hochvakuum getrocknet und lieferte 1.79 g (79%ig, 18% d. Th.) der Titelverbindung. Das Filtrat wurde eingeengt und lieferte weitere 4.86 g (61%ig, 37% d. Th.) der Titelverbindung. Die beiden Fraktionen wurden vereinigt und ohne weitere Reinigung umgesetzt.
LC-MS (Methode 4): Rₜ=1.87 min; MS (ESIpos): m/z = 246 (M+H)⁺
¹H-NMR (400 MHz, DMSO-d₆): δ = 4.33 (s, 2H), 7.06 - 7.12 (m, 1H), 7.12 - 7.19 (m, 1H), 7.22 - 7.29 (m, 1H), 7.29 - 7.35 (m, 1H), 7.87 (dd, 1H), 7.84 - 7.89 (m, 1H), 8.48 - 8.51 (br. s, 1H).

### Beispiel 16A

### 1,4,5,6-Tetrahydrocyclopenta[c]pyrazol-3-carbonitril

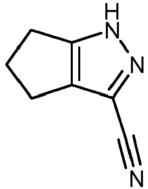

Die Darstellung der Verbindung ist beschrieben in: Org. Process Res. Dev. 2009, 13, 543.

### Beispiel 17A

### 1-(2-Fluorbenzyl)-1,4,5,6-tetrahydrocyclopenta[c]pyrazol-3-carbonitril

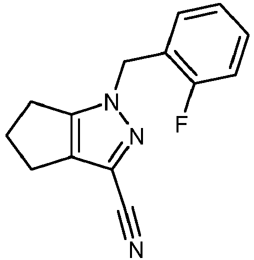

10.320 g (77.50 mmol) 1,4,5,6-Tetrahydrocyclopenta[c]pyrazol-3-carbonitril wurden in 100 ml DMF gelöst, mit 30.304 g (93.01 mmol) Cäsiumcarbonat und 16.116 g (85.26 mmol) 2-Fluorbenzylbromid versetzt und bei RT über Nacht gerührt. Der Ansatz wurde eingeengt und in Dichlormethan aufgenommen und mit Wasser versetzt. Die organische Phase wurde abgetrennt und die wässrige Phase zweimal mit Dichlormethan extrahiert. Die vereinigten organischen Phasen wurden mit gesättigter wässriger Natriumchlorid-Lösung gewaschen, über einen Siliconfilter filtriert und eingeengt. Der Rückstand wurde durch Flashchromatographie an Kieselgel (Eluent: Hexan/Essigsäureethylester, Gradient) gereinigt. Es wurden 11.37 g (60% d. Th.) der Zielverbindung erhalten.
¹H-NMR (400MHz, DMSO-d₆): δ [ppm]= 2.59-2.64 (m, 4H), 5.33 (s, 2H), 7.15-7.23 (m, 2H), 7.27-7.33 (m, 1H), 7.36-7.43 (m, 1H).

### Beispiel 18A

### 1-(2-Fluorbenzyl)-1,4,5,6-tetrahydrocyclopenta[c]pyrazol-3-carboximidamid

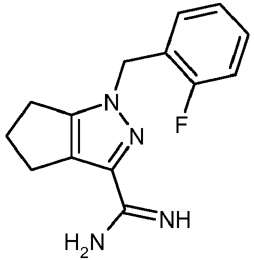

Unter einer Stickstoffatmosphäre wurden 3.600 g (14.92 mmol) 1-(2-Fluorbenzyl)-1,4,5,6-tetrahydrocyclopenta[c]pyrazol-3-carbonitril in 37 ml absolutem Methanol gelöst. Es wurden 1.306 (24.17 mmol) Natriummethylat zugegeben und 4 h bei RT gerührt. Es wurden 1.452 g (24.17 mmol) Essigsäure sowie 1.197 g (22.38 mmol) Ammoniumchlorid addiert und die Suspension über Nacht bei 50°C gerührt. Das Reaktionsgemisch wurde eingeengt und der Rückstand in 100 ml Wasser und 25 ml 1N Salzsäure suspendiert. Das Gemisch wurde mit Dichlormethan extrahiert. Die wässrige Phase wurde mit 2N Natronlauge basisch gestellt (pH = 12) und dreimal mit einem Gemisch aus Dichlormethan/Methanol (v/v = 8:2) extrahiert. Die vereinigten organischen Phasen wurden mit Natriumsulfat getrocknet, eingeengt, mit Toluol versetzt und erneut zur Trockne einrotiert. Es wurden 1.94 g (50% d. Th.) der Zielverbindung erhalten.

### Beispiel 19A

### 4-Amino-2-[1-(2-fluorbenzyl)-1,4,5,6-tetrahydrocyclopenta[c]pyrazol-3-yl]-5,5-dimethyl-5,7-dihydro-6H-pyrrolo[2,3-d]pyrimidin-6-on

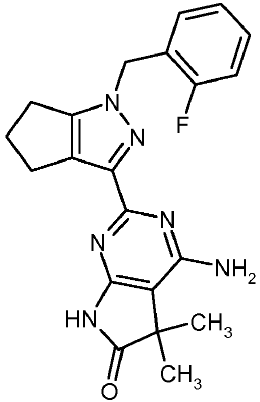

300 mg (1.15 mmol) 1-(2-Fluorbenzyl)-1,4,5,6-tetrahydrocyclopenta[c]pyrazol-3-carboximidamid wurden mit 2 ml *tert*.-Butanol, 287 mg (1.38 mmol) Methyl-3,3-dicyan-2,2-dimethylpropanoat gelöst in 2 ml *tert*.-Butanol sowie 181 mg (1.61 mmol) Kalium-*tert.*-butylat versetzt und 72 h unter Rückfluss erhitzt. Es wurde zur Trockne eingeengt und der Rückstand mit Wasser/Isopropanol (v/v = 3:1) verrührt. Der Feststoff wurde abfiltriert und im Hochvakuum getrocknet. Es wurden 385 mg (80% d. Th.) der Zielverbindung erhalten.
LC-MS (Methode 1): Rₜ=0.83 min; MS (ESIpos): m/z = 393 (M+H)⁺

### Beispiel 20A

### 1-(2-Fluorbenzyl)-1,4,5,6-tetrahydrocyclopenta[c]pyrazol-3-carboximidohydrazid

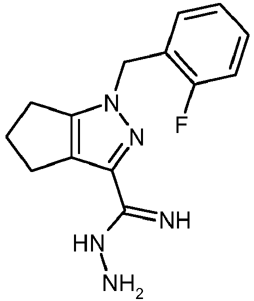

200 mg (0.77 mmol) 1-(2-Fluorbenzyl)-1,4,5,6-tetrahydrocyclopenta[c]pyrazol-3-carboximidamid wurden in 4 ml Ethanol vorgelegt und auf 0°C gekühlt. Es wurden 310 mg (3.07 mmol) Triethylamin sowie 48 mg (0.77 mmol) 80%-iges Hydrazinhydrat zugegeben und 72 h bei Raumtemperatur gerührt. Das Gemisch wurde am Rotationsverdampfer eingeengt, in Essigsäureethylester aufgenommen und dreimal mit gesättigter wässriger Natriumchlorid-Lösung gewaschen. Die organische Phase wurde über Natriumsulfat getrocknet, am Rotationsverdampfer eingeengt und im Hochvakuum getrocknet. Es wurden 209 mg (100 % d. Th.) der Zielverbindung erhalten.
LC-MS (Methode 1): Rₜ=0.58 min; MS (ESIpos): m/z = 274 (M+H)⁺

### Beispiel 21A

### Methyl-2-{3-[1-(2-fluorbenzyl)-1,4,5,6-tetrahydrocyclopenta[c]pyrazol-3-yl]-5-hydroxy-1,2,4-triazin-6-yl} -2-methylpropanoat

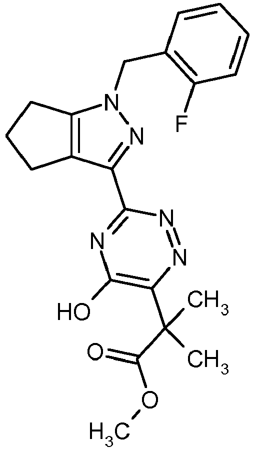

Es wurden 218 mg (1.13 mmol) Dimethyl-2,2-dimethyl-3-oxobutandioat in 5 ml Ethanol vorgelegt und zum Rückfluss erhitzt. Anschließend wurden 205 mg (0.75 mmol) 1-(2-Fluorbenzyl)-1,4,5,6-tetrahydrocyclopenta[c]pyrazol-3-carboximidohydrazid suspendiert in 5 ml Ethanol addiert und über Nacht unter Rückfluss gekocht. Nach dem Abkühlen wurde das Gemisch filtriert, der Filterkuchen mit wenig Ethanol gewaschen und das Filtrat eingeengt. Der Rückstand wurde mittels präparativer HPLC (Eluent: Acetonitril/Wasser, Gradient 20:80 → 100:0) gereinigt. Es wurden 48 mg der Zielverbindung erhalten (Reinheit 54%, 8% d. Th.).
LC-MS (Methode 1): Rₜ=1.03 min; MS (ESIpos): m/z = 412 (M+H)⁺

### Beispiel 22A

### 4-Chlor-6-methyl-1H-pyrazolo [3,4-d]pyrimidin

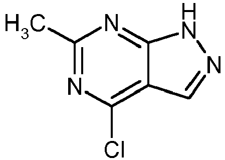

Die Darstellung der Verbindung ist beschrieben in: J. Org. Chem. 1958, 23, 191.

### Beispiel 23A

### 6-Methyl-1H-pyrazolo [3,4-d]pyrimidin

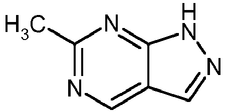

4.464 g (ca. 24.28 mmol, Reinheit 92%) 4-Chlor-6-methyl-1H-pyrazolo[3,4-d]pyrimidin wurden in 180 ml Dioxan gelöst und mit 2.948 g (29.14 mmol) Triethylamin sowie 5.629 g 20% Palladiumhydroxid auf Kohle versetzt und mit Wasserstoff bei 3 bar und RT für 2 d hydriert. Es wurden 100 ml Essigsäureethylester, 2.948 g (29.14 mmol Triethylamin sowie 2.000 g 20% Palladiumhydroxid auf Kohle addiert. Das Gemisch wurde mit Wasserstoff bei 3 bar und RT für 3 h hydriert. Es wurde über Celite filtriert mit wenig Dioxan/Essigsäureethylester gewaschen und das Filtrat am Rotationsverdampfer eingeengt. Es wurden 2.180 g (Reinheit 73%, 49% d. Th.) der Zielverbindung erhalten.
LC-MS (Methode 3): Rₜ=0.40 min; MS (ESIpos): m/z = 135 (M+H)⁺

### Beispiel 24A

### 3-Iod-6-methyl-1H-pyrazolo[3,4-d]pyrimidin

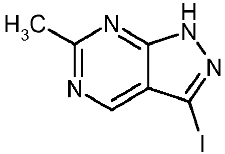

Es wurden 2.180 g (Reinheit 73%, ca. 11.82 mmol) 6-Methyl-1H-pyrazolo[3,4-d]pyrimidin und 3.987 g (17.72 mmol) N-Iodsuccinimid in 30 ml DMF gelöst und 2 h bei 80°C erhitzt. Nach dem Abkühlen wurde das Gemisch am Rotationsverdampfer eingeengt und der Rückstand mit Dichlormethan verrührt, abgesaugt und im Hochvakuum getrocknet. Es wurden 7.950 g (38% Reinheit, 100% d. Th.) der Zielverbindung erhalten.
LC-MS (Methode 1): Rₜ=0.52 min; MS (ESIpos): m/z = 261 (M+H)⁺

### Beispiel 25A

### 1-(2-Fluorbenzyl)-3-iod-6-methyl-1H-pyrazolo [3,4-d]pyrimidin

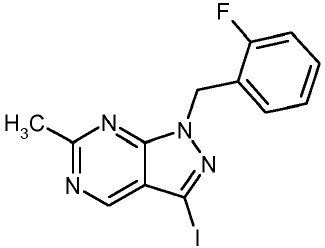

Es wurden 7.950 g (13.76 mmol) 3-Iod-6-methyl-1H-pyrazolo[3,4-d]pyrimidin und 4.930 g (15.13 mmol) Cäsiumcarbonat in 20 ml DMF vorgelegt und mit 2.860 g (15.13 mmol) 2-Fluorbenzylbromid gelöst in 5 ml DMF versetzt. Das Reaktionsgemisch wurde über Nacht bei RT gerührt, mit 100 ml Wasser verdünnt und mit Essigsäureethylester extrahiert. Die organische Phase wurde über Natriumsulfat getrocknet und am Rotationsverdampfer eingeengt. Der Rückstand wurde mittels präparativer HPLC (Eluent: Acetonitril/Wasser, Gradient 30:70 → 95:5) gereinigt. Es wurden 1.030 g der Zielverbindung erhalten (20% d. Th.).
LC-MS (Methode 3): Rₜ= 2.27 min; MS (ESIpos): m/z = 369 (M+H)⁺

### Beispiel 26A

### 1-(2-Fluorbenzyl)-6-methyl-1H-pyrazolo [3,4-d]pyrimidin-3-carbonitril

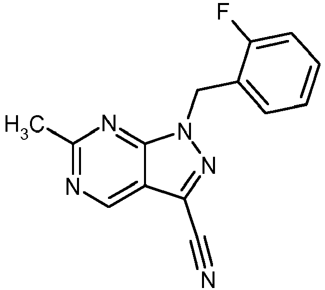

Es wurden 1.485 g (4.03 mmol) 1-(2-Fluorbenzyl)-3-iod-6-methyl-1H-pyrazolo[3,4-d]pyrimidin und 397 mg (4.44 mmol) Kupfer(I)cyanid in 11 ml absolutem DMSO vorgelegt und 2 h bei 150°C erhitzt. Nach dem Abkühlen wurde über Celite filtriert und mit Essigsäureethylester sowie THF nachgewaschen. Die organische Phase wurde mit 25%-iger wässriger Ammoniak-Lösung, gesättigter wässriger Ammoniumchlorid-Lösung sowie gesättigter wässriger Natriumchlorid-Lösung gewaschen, über Natriumsulfat getrocknet und am Rotationsverdampfer eingeengt. Es wurden 994 mg (Reinheit 81%, 75 % d. Th.) der Zielverbindung erhalten.
LC-MS (Methode 1): Rₜ= 0.96 min; MS (ESIpos): m/z = 268 (M+H)⁺

### Beispiel 27A

### 1-(2-Fluorbenzyl)-6-methyl-1H-pyrazolo [3,4-d]pyrimidin-3-carboximidamid

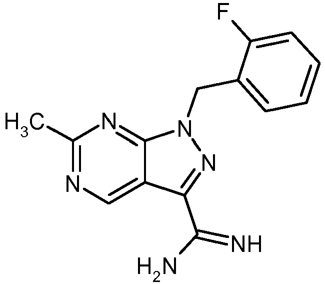

Unter einer Argonatmosphäre wurden 994 mg (Reinheit 81%, ca. 3.01 mmol) 1-(2-Fluorbenzyl)-6-methyl-1H-pyrazolo[3,4-d]pyrimidin-3-carbonitril in 15 ml absolutem Methanol gelöst. Es wurden 209 mg (3.72 mmol) Natriummethylat zugegeben und 1 h bei RT gerührt. Anschließend wurden 31 mg (0.56 mmol) Natriummethylat nachgesetzt und 15 min bei RT gerührt. Es wurden 871 mg (14.50 mmol) Essigsäure sowie 489 mg (4.46 mmol) Ammoniumchlorid addiert und die Mischung für 45 min bei 45°C gerührt. Das Reaktionsgemisch wurde eingeengt, der Rückstand mit 1N Natronlauge verrührt, der Niederschlag abgesaugt und im Hochvakuum getrocknet. Es wurden 918 mg (Reinheit 91%, 97% d. Th.) der Zielverbindung erhalten.
LC-MS (Methode 2) Rₜ= 0.53 min; MS (ESIpos): m/z = 285 (M+H)⁺

### Beispiel 28A

### 4-Amino-2-[1-(2-fluorbenzyl)-6-methyl-1H-pyrazolo[3,4-d]pyrimidin-3-yl]-5,5-dimethyl-5,7-dihydro-6H-pyrrolo[2,3-d]pyrimidin-6-on

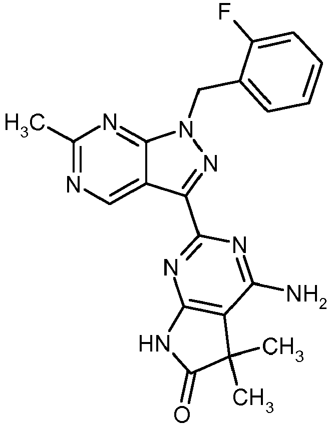

200 mg (0.70 mmol) 1-(2-Fluorbenzyl)-6-methyl-1H-pyrazolo[3,4-d]pyrimidin-3-carboximidamid wurden mit 3 ml *tert.*-Butanol, 146 mg (0.70 mmol) Methyl-3,3-dicyan-2,2-dimethylpropanoat gelöst in 1.5 ml *tert*.-Butanol sowie 94 mg (0.84 mmol) Kalium-tert.-butylat versetzt und 48 h unter Rückfluss erhitzt. Es wurde Wasser addiert und der Niederschlag abfiltriert. Das Filtrat wurde mit Dichlormethan extrahiert, die organische Phase über Natriumsulfat getrocknet und am Rotationsverdampfer eingeengt. Der Rückstand wurde mit Wasser/Ethanol verrührt. Der Feststoff wurde abfiltriert und im Hochvakuum getrocknet. Es wurden 102 mg (34% d. Th.) der Zielverbindung erhalten.
LC-MS (Methode 1): Rₜ=0.81 min; MS (ESIpos): m/z = 419 (M+H)⁺

### Beispiel 29A

### 1-(2-Fluorbenzyl)-6-methyl-1H-pyrazolo [3,4-d]pyrimidin-3-carboximidohydrazid

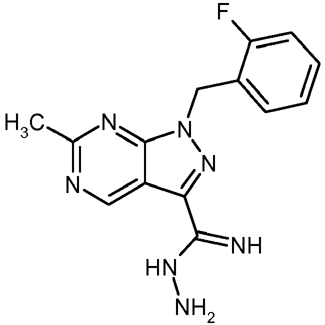

688 mg (ca. 2.20 mmol, Reinheit 92%) 1-(2-Fluorbenzyl)-6-methyl-1H-pyrazolo[3,4-d]pyrimidin-3-carboximidamid wurden in 10 ml Ethanol vorgelegt und auf 0°C gekühlt. Es wurden 891 mg (8.80 mmol) Triethylamin sowie 138 mg (2.20 mmol) 80%-iges Hydrazinhydrat zugegeben und 18 h bei Raumtemperatur gerührt. Das Gemisch wurde am Rotationsverdampfer eingeengt, in Essigsäureethylester aufgenommen und dreimal mit gesättigter wässriger Natriumchlorid-Lösung gewaschen. Die organische Phase wurde über Natriumsulfat getrocknet, am Rotationsverdampfer eingeengt und im Hochvakuum getrocknet. Es wurden 654 mg (Reinheit 93%, 92 % d. Th.) der Zielverbindung erhalten.
LC-MS (Methode 1): Rₜ=0.54 min; MS (ESIpos): m/z = 300 (M+H)⁺

### Beispiel 30A

### Methyl-2-{3-[1-(2-fluorbenzyl)-6-methyl-1H-pyrazolo[3,4-d]pyrimidin-3-yl]-5-hydroxy-1,2,4-triazin-6-yl}-2-methylpropanoat

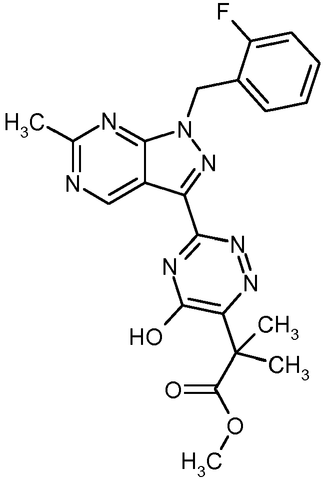

Es wurden 615 mg (3.27 mmol) Dimethyl-2,2-dimethyl-3-oxobutandioat in 13 ml Ethanol vorgelegt und zum Rückfluss erhitzt. Anschließend wurden 652 mg (2.18 mmol) 1-(2-Fluorbenzyl)-6-methyl-1H-pyrazolo[3,4-d]pyrimidin-3-carboximidohydrazid suspendiert in 13 ml Ethanol addiert und über Nacht unter Rückfluss gekocht. Nach dem Abkühlen wurde das Gemisch filtriert, der Filterkuchen mit wenig Ethanol gewaschen und das Filtrat eingeengt. Der Rückstand wurde mittels präparativer HPLC (Eluent: Acetonitril/Wasser, Gradient 30:70 → 100:0) gereinigt. Es wurden 182 mg der Zielverbindung erhalten (19% d. Th.).
LC-MS (Methode 1): Rt=0.91 min; MS (ESIpos): m/z = 438 (M+H)⁺

### Beispiel 31A

### 1-(2,3-Difluorbenzyl)-3-iod-6-methyl-1H-pyrazolo [3,4-d]pyrimidin

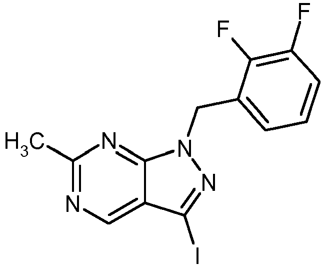

Es wurden 7.100 g (ca. 10.92 mmol, Reinheit 40%) 3-Iod-6-methyl-1H-pyrazolo[3,4-d]pyrimidin und 4.626 g (14.20 mmol) Cäsiumcarbonat in 100 ml DMF vorgelegt und mit 2.939 g (14.20 mmol) 2,3-Difluorbenzylbromid gelöst in 50 ml DMF versetzt. Das Reaktionsgemisch wurde 3 h bei RT gerührt, auf 1.5 1 Eiswasser gegeben und mit Diethylether extrahiert. Die organische Phase wurde über Natriumsulfat getrocknet und am Rotationsverdampfer eingeengt. Der Rückstand wurde mittels präparativer HPLC (Eluent: Acetonitril/Wasser, Gradient 30:70 → 95:5) gereinigt. Es wurden 1.360 g der Zielverbindung erhalten (31% d. Th.).
LC-MS (Methode 1): Rₜ=1.04 min; MS (ESIpos): m/z = 387 (M+H)⁺

### Beispiel 32A

### 1-(2,3-Difluorbenzyl)-6-methyl-1H-pyrazolo [3,4-d]pyrimidin-3-carbonitril

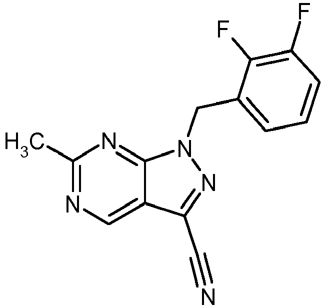

Es wurden 1.360 g (3.35 mmol) 1-(2,3-Difluorbenzyl)-3-iod-6-methyl-1H-pyrazolo[3,4-d]pyrimidin und 330 mg (3.68 mmol) Kupfer(I)cyanid in 10 ml absolutem DMSO vorgelegt und 2 h bei 150°C erhitzt. Nach dem Abkühlen wurde Ansatz auf 200 ml Essigsäureethylester gegeben und mit einer Mischung aus konzentrierter wässriger Ammoniak-Lösung sowie halbgesättigter wässriger Ammoniumchlorid-Lösung (v/v = 1:3) gewaschen. Die organische Phase wurde mit gesättigter wässriger Natriumchlorid-Lösung gewaschen, über Natriumsulfat getrocknet und am Rotationsverdampfer eingeengt. Es wurden 1.040 g (Reinheit 92%, 100 % d. Th.) der Zielverbindung erhalten.
LC-MS (Methode 1): Rₜ=0.98 min; MS (ESIpos): m/z = 286 (M+H)⁺

### Beispiel 33A

### 1-(2,3-Difluorbenzyl)-6-methyl-1H-pyrazolo [3,4-d]pyrimidin-3-carboximidamid

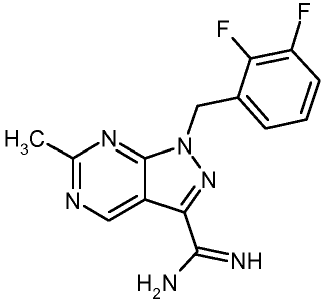

Unter Argonatmosphäre wurden 0.73 ml (3.35 mmol) einer 25%-igen Natriummethylat-Lösung in Methanol vorgelegt und mit 1.040 g (Reinheit 92%, 3.35 mmol) 1-(2,3-Difluorbenzyl)-6-methyl-1H-pyrazolo[3,4-d]pyrimidin-3-carbonitril gelöst in 4 ml absolutem Methanol versetzt. Es wurde 1 h bei RT gerührt. Anschließend wurden 215 mg (4.03 mmol) Ammoniumchlorid sowie 786 mg (13.08 mmol) Essigsäure addiert und die Mischung für 2 h zum Rückfluss erhitzt. Das Reaktionsgemisch wurde eingeengt, der Rückstand mit 10 ml Essigsäureethylester sowie 15 ml Wasser versetzt und mit 2N Natronlauge basisch gestellt (pH=10). Es wurde 1 h bei RT gerührt, Wasser zugegeben und mit Essigsäureethylester extrahiert. Die organische Phase wurde über Natriumsulfat getrocknet, eingeengt und im Hochvakuum getrocknet. Es wurden 840 mg (Reinheit 85%, 70% d. Th.) der Zielverbindung erhalten.
LC-MS (Methode 1): Rₜ= 0.64 min; MS (ESIpos): m/z = 303 (M+H)⁺

### Beispiel 34A

### 4-Amino-2-[1-(2,3-difluorbenzyl)-6-methyl-1H-pyrazolo[3,4-d]pyrimidin-3-yl]-5,5-dimethyl-5,7-dihydro-6H-pyrrolo[2,3-d]pyrimidin-6-on

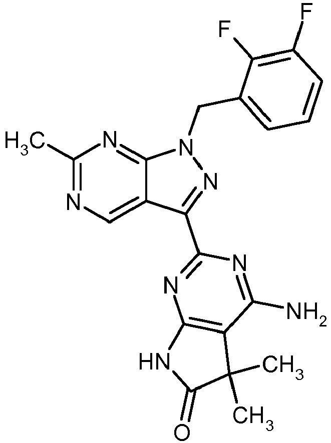

400 mg (1.11 mmol) 1-(2,3-Difluorbenzyl)-6-methyl-1H-pyrazolo[3,4-d]pyrimidin-3-carboximid-amid wurden mit 3 ml *tert.*-Butanol, 277 mg (1.33 mmol) Methyl-3,3-dicyan-2,2-dimethylpropanoat gelöst in 2 ml *tert*.-Butanol sowie 175 mg (1.56 mmol) Kalium-*tert*.-butylat versetzt und 24 h zum Rückfluss erhitzt. Es wurde wenig Wasser addiert und die Reaktionslösung über präparative HPLC (Eluent: Acetonitril/Wasser, Gradient 30:70 → 95:5) gereinigt. Die produkthaltigen Fraktionen wurden eingeengt und der Rückstand wurde mit Wasser/Isopropanol verrührt. Der Feststoff wurde abfiltriert und erneut mittels präparativer HPLC (Eluent: Acetonitril/Wasser mit 0.1% Salzsäure, Gradient 20:80 → 100:0) gereinigt. Es wurden 170 mg (35% d. Th.) der Zielverbindung erhalten.
LC-MS (Methode 1): Rₜ=0.87 min; MS (ESIpos): m/z = 437 (M+H)⁺

### Beispiel 35A

### 1-(2,3-Difluorbenzyl)-6-methyl-1H-pyrazolo [3,4-d]pyrimidin-3-carboximidohydrazid

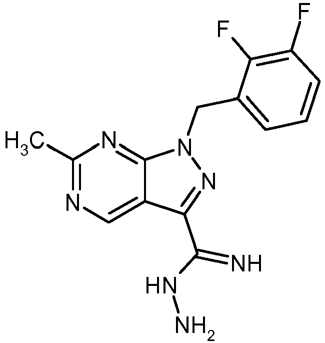

Unter einer Argonatmosphäre wurden 440 mg (1.237 mmol, Reinheit 85%) 1-(2,3-Difluorbenzyl)-6-methyl-1H-pyrazolo[3,4-d]pyrimidin-3-carboximidamid in 6 ml Ethanol vorgelegt und auf 0°C gekühlt. Es wurden 500 mg (4.49 mmol) Triethylamin sowie 85 mg (1.361 mmol) 80%-iges Hydrazinhydrat zugegeben und 72 h bei Raumtemperatur gerührt. Das Gemisch wurde am Rotationsverdampfer eingeengt, in Essigsäureethylester aufgenommen und dreimal mit gesättigter wässriger Natriumchlorid-Lösung gewaschen. Die organische Phase wurde über Natriumsulfat getrocknet, am Rotationsverdampfer eingeengt und im Hochvakuum getrocknet. Es wurden 421 mg (Reinheit 84%, 90 % d. Th.) der Zielverbindung erhalten.
LC-MS (Methode 1): Rₜ=0.55 min; MS (ESIpos): m/z = 318 (M+H)⁺

### Beispiel 36A

### Methyl-2-{3-[1-(2,3-difluorbenzyl)-6-methyl-1H-pyrazolo[3,4-d]pyrimidin-3-yl]-5-hydroxy-1,2,4-triazin-6-yl} -2-methylpropanoat

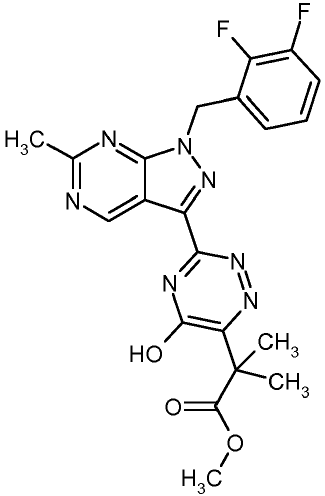

Es wurden 374 mg (1.985 mmol) Dimethyl-2,2-dimethyl-3-oxobutandioat in 8 ml Ethanol vorgelegt und zum Rückfluss erhitzt. Anschließend wurden 420 mg (1.324 mmol) 1-(2,3-Difluorbenzyl)-6-methyl-1H-pyrazolo[3,4-d]pyrimidin-3-carboximidohydrazid suspendiert in 8 ml Ethanol addiert und über Nacht zum Rückfluss erhitzt. Es wurden erneut 299 mg (1.588 mmol) Dimethyl-2,2-dimethyl-3-oxobutandioat gelöst in 3 ml Ethanol addiert und über Nacht unter Rückfluss gekocht. Nach dem Abkühlen wurde das Gemisch filtriert und direkt mittels präparativer HPLC (Eluent: Acetonitril/Wasser, Gradient 30:70 → 100:0) gereinigt. Es wurden 163 mg der Zielverbindung erhalten (Reinheit 89%, 24% d. Th.).
LC-MS (Methode 1): Rₜ= 0.96 min; MS (ESIpos): m/z = 456 (M+H)⁺

### Beispiel 37A

### 1H-pyrazolo [3,4-d]pyrimidin

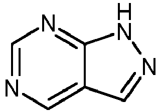

Die Darstellung der Verbindung ist beschrieben in: J. Am. Chem.Soc. 1956, 78, 784.

### Beispiel 38A

### 3-Iod-1H-pyrazolo[3,4-d]pyrimidin

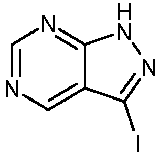

Es wurden 520 mg (4.33 mmol) 1H-Pyrazolo[3,4-d]pyrimidin und 1.461 g (6.496 mmol) N-Iodsuccinimid in 10 ml DMF gelöst und 3 h bei 80°C erhitzt. Nach dem Abkühlen wurde das Gemisch am Rotationsverdampfer eingeengt und der Rückstand mit Dichlormethan verrührt, abgesaugt und im Hochvakuum getrocknet. Es wurden 569 mg (53% d. Th.) der Zielverbindung erhalten.
LC-MS (Methode 3): Rₜ=1.23 min; MS (ESIpos): m/z = 247 (M+H)⁺

### Beispiel 39A

### 1-(2-Fluorbenzyl)-3-iod-1H-pyrazolo[3,4-d]pyrimidin

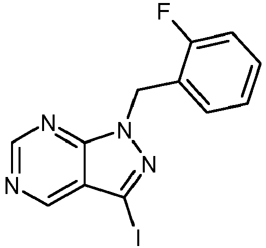

Es wurden 569 mg (2.313 mmol) 3-Iod-1H-pyrazolo[3,4-d]pyrimidin und 828 mg (2.544 mmol) Cäsiumcarbonat in 10 ml DMF vorgelegt und mit 481 mg (2.544 mmol) 2-Fluorbenzylbromid gelöst in 2 ml DMF versetzt. Das Reaktionsgemisch wurde über Nacht bei RT gerührt, mit 50 ml Wasser verdünnt, abgesaugt und der Rückstand im Hochvakuum getrocknet. Es wurden 733 mg der Zielverbindung erhalten (Reinheit 83%, 74% d. Th.).
LC-MS (Methode 1): Rₜ=0.96 min; MS (ESIpos): m/z = 355 (M+H)⁺

### Beispiel 40A

### 1-(2-Fluorbenzyl)-1H-pyrazolo[3,4-d]pyrimidin-3-carbonitril

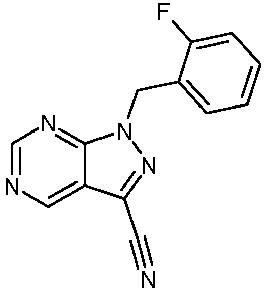

Es wurden 950 mg (Reinheit 85%, ca. 2.281 mmol) 1-(2-Fluorbenzyl)-3-iod-1H-pyrazolo[3,4-d]pyrimidin und 225 mg (2.509 mmol) Kupfer(I)cyanid in 6 ml absolutem DMSO vorgelegt und 2 h bei 150°C erhitzt. Nach dem Abkühlen wurde über Celite filtriert und mit Essigsäureethylester sowie THF nachgewaschen. Die organische Phase wurde mit 25%-iger wässriger Ammoniak-Lösung, gesättigter wässriger Ammoniumchlorid-Lösung sowie gesättigter wässriger Natriumchlorid-Lösung gewaschen, über Natriumsulfat getrocknet und am Rotationsverdampfer eingeengt. Es wurden 685 mg (Reinheit 84%, 99% d. Th.) der Zielverbindung erhalten.
LC-MS (Methode 2) Rₜ=0.95 min; MS (ESIpos): m/z = 254 (M+H)⁺

### Beispiel 41A

### 1-(2-Fluorbenzyl)-1H-pyrazolo[3,4-d]pyrimidin-3-carboximidamid-Acetat

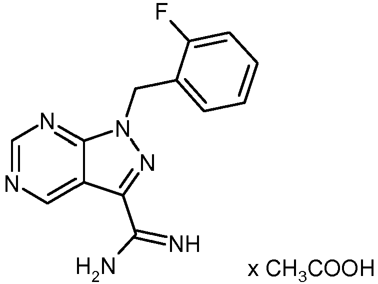

Unter einer Argonatmosphäre wurden 685 mg (Reinheit 84%, ca. 2.273 mmol) 1-(2-Fluorbenzyl)-1H-pyrazolo[3,4-d]pyrimidin-3-carbonitril in 8 ml absolutem Methanol gelöst. Es wurden 127 mg (2.273 mmol) Natriummethylat zugegeben und 1 h bei RT gerührt. Es wurden 532 mg (8.864 mmol) Essigsäure sowie 299 mg (2.273 mmol) Ammoniumchlorid addiert und die Mischung für 45 min unter Rückfluss gekocht. Das Reaktionsgemisch wurde eingeengt, der Rückstand mit 20 ml 1N Natronlauge verrührt, der Niederschlag abgesaugt und im Hochvakuum getrocknet. Es wurden 610 mg (Reinheit 86%, 86% d. Th.) der Zielverbindung erhalten.
LC-MS (Methode 1): Rₜ=0.53 min; MS (ESIpos): m/z = 271 (M+H)⁺

### Beispiel 42A

### 1-(2-Fluorbenzyl)-1H-pyrazolo[3,4-d]pyrimidin-3-carboximidohydrazid

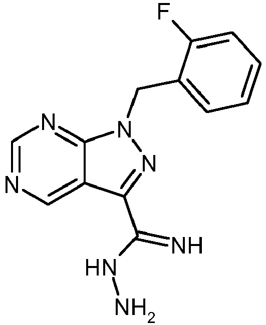

610 mg (ca. 1.92 mmol, Reinheit 86%) 1-(2-Fluorbenzyl)-1H-pyrazolo[3,4-d]pyrimidin-3-carboximidamid wurden in 10 ml Ethanol vorgelegt und auf 0°C gekühlt. Es wurden 777 mg (7.68 mmol) Triethylamin sowie 120 mg (1.920 mmol) 80%-iges Hydrazinhydrat zugegeben und 18 h bei Raumtemperatur gerührt. Das Gemisch wurde am Rotationsverdampfer eingeengt, in Essigsäureethylester aufgenommen und dreimal mit gesättigter wässriger Natriumchlorid-Lösung gewaschen. Die organische Phase wurde über Natriumsulfat getrocknet, am Rotationsverdampfer eingeengt und im Hochvakuum getrocknet. Es wurden 590 mg (Reinheit 89%, 95 % d. Th.) der Zielverbindung erhalten.
LC-MS (Methode 3): Rₜ=1.37 min; MS (ESIpos): m/z = 286 (M+H)⁺

### Beispiel 43A

### Methyl-2-{3-[1-(2-fluorbenzyl)-1H-pyrazolo[3,4-d]pyrimidin-3-yl]-5-hydroxy-1,2,4-triazin-6-yl}-2-methylpropanoat

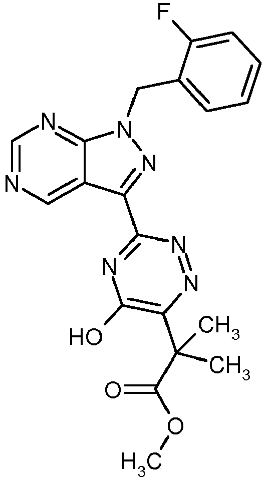

Es wurden 584 mg (3.103 mmol) Dimethyl-2,2-dimethyl-3-oxobutandioat in 12 ml Ethanol vorgelegt und zum Rückfluss erhitzt. Anschließend wurden 590 mg (2.069 mmol) 1-(2-Fluorbenzyl)-1H-pyrazolo[3,4-d]pyrimidin-3-carboximidohydrazid suspendiert in 12 ml Ethanol addiert und über Nacht unter Rückfluss gekocht. Nach dem Abkühlen wurde das Gemisch filtriert, der Filterkuchen mit wenig Ethanol gewaschen und das Filtrat eingeengt. Der Rückstand wurde mit 10 ml Acetonitril verrührt. Es wurden 188 mg der Zielverbindung erhalten (Reinheit 93%, 20% d. Th.).
LC-MS (Methode 2) Rₜ=0.90 min; MS (ESIpos): m/z = 424 (M+H)⁺

### Beispiel 44A

### Dimethyl-3-(2-carbamoylhydrazinyliden)-2,2-dimethylbutandioat

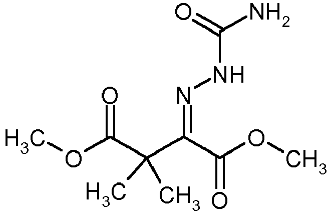

10 g (90 mmol) Semicarbazid-Hydrochlorid, 15.5g (82 mmol) Dimethyl-2,2-dimethyl-3-oxobutandioat und 8.1g (82 mmol) Natriumacetat wurden in 135 ml Wasser bei RT über Nacht gerührt und zwei Tage stehen gelassen. Es wurde mit Eiswasser gekühlt, der farblose Niederschlag abfiltriert, mit etwas Wasser gewaschen und getrocknet.
Ausb.: 15.4 g (77% d.Th.)
LC-MS (Methode 3): Rₜ=1.51 min; MS (ESIpos): m/z = 246 (M+H)⁺
¹H NMR (400 MHz, DMSO-*d₆*) δ [ppm] = 1.37 (s, 6 H), 3.59 (s, 3 H), 3.76 (s, 3 H), 6.77 (br. s., 2 H), 10.82 (s, 1 H)

### Beispiel 45A

### Methyl-2-(3,5-dioxo-2,3,4,5-tetrahydro-1,2,4-triazin-6-yl)-2-methylpropanoat

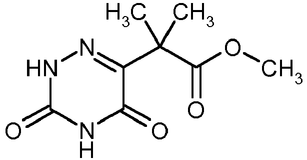

15.35 g (62.5 mmol) Beispiel 44A wurden in 235 ml Methanol gelöst und mit 6.8 g (125 mmol) Natriummethylat versetzt, wobei schnell ein farbloser Niederschlag ausfiel. Es wurde mit 235 ml Methanol verdünnt und das Reaktionsgemisch anschließend 1.5 h zum Rückfluß erhitzt. Nach dem Abkühlen wurde eingeengt und der Rückstand langsam zu einer Lösung von 14.4 ml (251 mmol) Eisessig in 150 ml Wasser gegeben. Das Gemisch wurde teilweise eingeengt, mit Eiswasser gekühlt, der ausgefallene Niederschlag abgesaugt, mit wenig Wasser nachgewaschen und über Nacht bei 45°C im Vakuum getrocknet.
Ausb.: 11.0 g (82% d. Th.).
LC-MS (Methode 3): Rₜ=1.28 min; MS (ESIpos): m/z = 212 (M-H)⁻
¹H NMR (400 MHz, DMSO-*d₆*) δ [ppm] = 1.35 (s, 6 H), 3.55 (s, 3 H), 12.16 (br. s., 2 H)

### Beispiel 46A

### Methyl-2-(3,5-dichlor-1,2,4-triazin-6-yl)-2-methylpropanoat

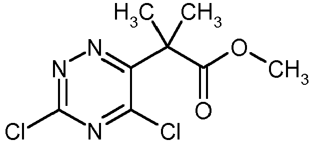

5.15 g (24 mmol) Beispiel 45A wurden in 100 ml Phosphoroxychlorid und 1 ml DMF über Nacht zum Rückfluss erhitzt. Das Reaktionsgemisch wurde eingeengt und anschliessend unter externer Kühlung im Eisbad vorsichtig mit Eis verrieben wurde. Nach Zugabe von Dichlormethan wurde unter Rühren mit festem Natriumhydrogencarbonat pH 6 eingestellt, die Phasen getrennt und die Wasserphase mit Dichlormethan extrahiert. Die vereinigten organischen Phasen wurden getrocknet und eingeengt. Der Rückstand (4.88g) wurde mit Dichlormethan/Essigsäureethylester (10:1) über eine Kieselgelschicht filtriert.
Ausb.: 3.1 g (51% d. Th.)
LC-MS (Methode 3): Rₜ=2.08 min; MS (ESIpos): m/z = 250 (M+H)⁺

### Beispiel 47A

### 3-Chlor-7,7-dimethyl-5,7-dihydro-6H-pyrrolo[2,3-e][1,2,4]triazin-6-on

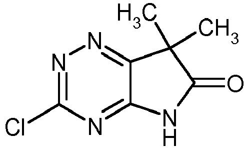

2 g (8 mmol) Beispiel 46A wurden in 30 ml Dioxan gelöst, mit 10 ml aq. konz. Ammoniak versetzt und über Nacht bei RT gerührt. Die Reaktionslösung wurde eingeengt. Der Rückstand mit 50 ml Wasser verrührt und dann abgesaugt.
Ausb.: 1.12 g (71 % d. Th)
LC-MS (Methode 3): Rₜ=1.15 min; MS (ESIpos): m/z = 199 (M+H)⁺
¹H NMR (400 MHz, DMSO-*d₆*) δ [ppm] = 1.40 (s, 6 H), 12.40 (br. s, 1H)

### Beispiel 48A

### 3-Chlor-5-(4-methoxybenzyl)-7,7-dimethyl-5,7-dihydro-6H-pyrrolo[2,3-e][1,2,4]triazin-6-on

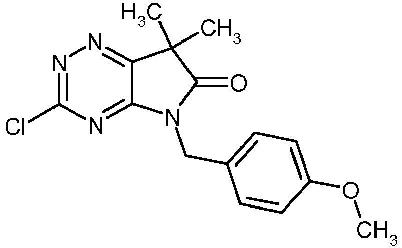

Zu einer Lösung von 390 mg (2.84 mmol) 4-Methoxy-benzylamin und 2.3ml (13.2 mmol) Diisopropylethylamin in 10 ml THF tropfte man bei 0°C eine Lösung von 645 mg (2.58 mmol) Beispiel 46A in 5ml THF und rührte die Suspension über Nacht bei RT nach. Die Reaktionsmischung wurde unter vermindertem Druck eingeengt. Ausb.: 1.4 g Rohprodukt. 1.1g des Rohproduktes wurden zweimal mit Dichlormethan/Methanol (100:1) per Säulenchromatographie an Kieselgel gereinigt.
Ausb.: 395 mg (60% d. Th.)
LC-MS (Methode 1): Rₜ=1.01 min; MS (ESIpos): m/z = 319 (M+H)⁺

### Beispiel 49A

### 3-[6-Fluor-3-(2-fluorbenzyl)-1H-pyrazolo [4,3-b]pyridin-1-yl]-5-(4-methoxybenzyl)-7,7-dimethyl-5,7-dihydro-6H-pyrrolo[2,3-e][1,2,4]triazin-6-on

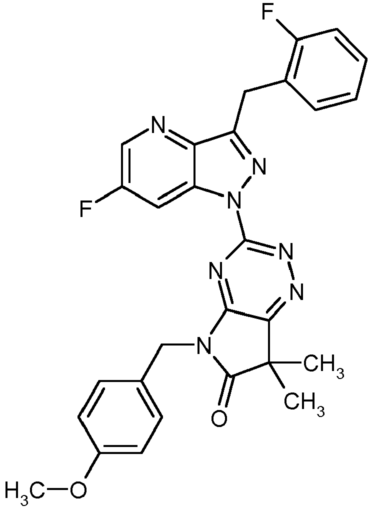

200 mg (0.82 mmol) Beispiel 15A wurden in 3ml NMP gelöst, mit 40 mg (1 mmol) Natriumhydrid (60%ig) versetzt und 30 min bei RT gerührt. Anschließend wurden 235 mg Rohprodukt aus Beispiel 48A zugegeben und 2h bei 80°C gerührt. Die Reaktionsmischung wurde mit Wasser versetzt und eingeengt. Eine Reinigung über präp. HPLC (Reprosil C18, Gradient aus Acetonitril/0.01% aq. Ameisensäure) ergab 131 mg (30% d. Th.) der Titelverbindung.
LC-MS (Methode 1) Rₜ=1.29 min; MS (ESIpos): m/z = 528 (M+H)⁺
¹H NMR (400 MHz, DMSO-*d₆*) δ [ppm] = 1.50 (s, 6 H), 3.72 (s, 3 H), 3.72 (s, 3 H), 4.49 (s, 2 H), 4.92 (s, 2 H), 6.88 (d, 2 H), 7.10 - 7.24 (m, 2 H), 7.26 - 7.38 (m, 3 H), 7.42 (t, 1 H), 8.51 (dd, 1 H), 8.75 (m, 1 H)

### Beispiel 50A

### 5-Fluor-3-(2-fluorbenzyl)-1H-indazol

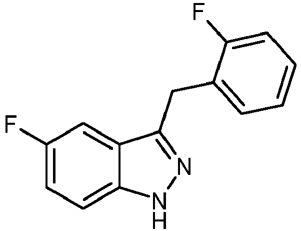

Die Titelverbindung wurde ausgehend von 2,5-Difluorbenzoylchlorid und 2-Fluorphenylessigsäuremethylester analog der Verfahren für Beispiel 12A, 13A, 14A und 15A hergestellt LC-MS (Methode 1): Rₜ=0.97 min; MS (ESIpos): m/z = 245 (M+H)⁺
¹H NMR (400 MHz, DMSO-*d₆)* δ [ppm] = 4.27 (s, 2 H), 7.01 - 7.42 (m, 6 H), 7.51 (dd, 1 H), 12.90 (br. s., 1 H)

### Beispiel 51A

### 5-Fluor-3-(3,3,4,4,4-pentafluorbutyl)-1H-indazol

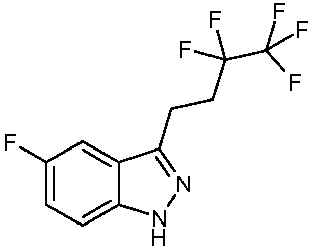

### Schritt a) Herstellung von Iod(3,3,4,4,4-pentafluorbutyl)zink (analog J. Org. Chem. 2002, 76, 6863 -6870):

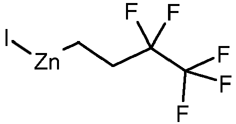

Unter Argon wurden 7.16 g (110 mmol) Zinkpulver in 22 ml THF vorgelegt. Unter Rühren wurde 1.45 g (7.7 mmol) 1,2-Dibromethan zugegeben und die Mischung mit dem Heißluftfön viermal jeweils kurz zum Sieden erhitzt und wieder auf RT abgekühlt. Dann wurden 230 mg (2.12 mmol) Trimethylsilychlorid zugegeben, die Mischung wurde 10 min bei RT gerührt und schließlich unter externer Kühlung mit Eiswasser eine Lösung von 10 g (36.5 mmol) 1,1,1,2,2-Pentafluor-4-iodbutan in 22 ml THF bei RT zugetropft und 15 min nachgerührt. Aus der dunkelgrauen Suspension wurde das Reagenz über einen Mikrofilter mit einer Spritze entnommen. Für die Lösung wurde ein Gehalt von 0.83 M angenommen.

### Schritt b) 5-Fluor-3-(3,3,4,4,4-pentafluorbutyl)-1H-indazol

Eine Lösung von 2.0 g (5.52 mmol) tert.-Butyl-5-fluor-3-iod-1H-indazol-1-carboxylat (Herdemann M. et al. Bioorg. Med. Chem. Lett., 2010 , 20, 6998 - 7003) in 30 ml wasserfreiem THF wurde jeweils dreimal evakuiert und mit Argon belüftet. Dann wurden 506 mg (0.55 mol) Tris(dibenzylidenaceton)dipalladium und 256.5 mg (1.11 mmol) Tri(2-furyl)phosphin zugegeben, nochmals evakuiert und mit Argon belüftet. Nun wurden 9.32 ml (ca. 7.73 mmol) der Lösung aus Schritt a) innerhalb 20 min bei 4-6°C zugegeben, 10 min bei 4°C und über Nacht ohne externe Kühlung weitergerührt. Es wurde Wasser zugesetzt und dreimal mit Essigsäureethylester extrahiert. Die vereinigten organischen Phasen wurden mit gesättigter wässriger Natriumchlorid-Lösung gewaschen, getrocknet und eingengt. Ein Teil des Rückstandes (470 mg) wurde über die präparativer HPLC (Reprosil C18, Gradient aus Acetonitril/0.01% aq. Ameisensäure) gereinigt. Ausb.: 190 mg (13% d. Th.).

Der zweite Teil des Rückstandes (1.9 g) wurde an 120 g Kieselgel mit i-Hexan / Essigsäureethylester (Gradient 10:1 bis 2:1) chromatographisch gereinigt. Es wurden 915 mg (Reinheit: 86%, entspr. 51% d. Th.) der Titelverbindung erhalten. Gesamtausb.: 64% d. Th.
LC-MS (Methode 1): Rₜ=1.05 min; MS (ESIpos): m/z = 283 (M+H)⁺
¹H-NMR (400MHz, DMSO-d₆): δ [ppm]= 2.60 - 2.84 (m, 2H), 3.10 - 3.25 (m, 2H), 7.23 (td, 1H), 7.44 - 7.57 (m, 1H), 7.63 (dd, 1H), 12.93 (s, 1H).

### Beispiel 52A

### 3-[6-Fluor-3-(3,3,4,4,4-pentafluorbutyl)-1H-pyrazolo[4,3-b]pyridin-1-yl]-5-(4-methoxybenzyl)-7,7-dimethyl-5,7-dihydro-6H-pyrrolo[2,3-e][1,2,4]triazin-6-on

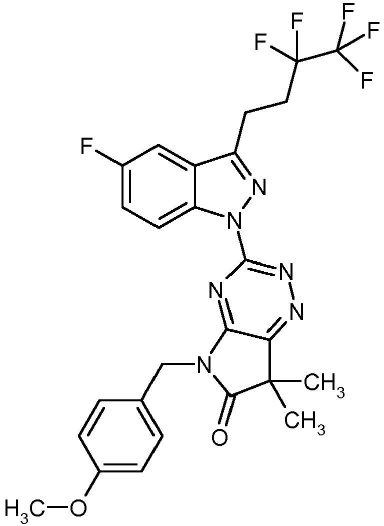

150 mg (0.53 mmol) Beispiel 51A wurden in 2 ml NMP gelöst, mit 21.3 mg (0.53 mmol) Natriumhydrid (60%ig) versetzt und 30 min bei RT gerührt. Anschließend wurden 141 mg (0.44 mmol) Beispiel 48A zugegeben, es wurde 7 h bei RT gerührt und 2 Tage stehen gelassen. Es wurden weiter 12.4 mg (0.31 mmol) Natriumhydrid und 85 mg (0.27 mmol) Beispiel 48A zugegeben und 3 h bei 60°C gerührt. Nach dem Abkühlen wurde mit 5 M Ameisensäure ein pH von 4-5 eingestellt und über die präp. HPLC (Reprosil C18, Gradient aus Acetonitril/0.01% aq. Ameisensäure) gereinigt. Ausb.: 74 mg (25% d. Th.)
LC-MS (Methode 4): Rₜ=1.38 min; MS (ESIpos): m/z = 565 (M+H)^{+ 1}H NMR (400 MHz, DMSO-d₆) δ [ppm] = 1.50 (s, 6 H), 2.77 - 2.96 (m, 2 H), 3.35 (t, 2 H), 3.71 (s, 3 H), 4.92 (s, 2 H), 6.90 (d, 2 H), 7.39 (d, 2 H), 7.51 (td, 1 H), 7.89 (d, 1 H), 8.51 (dd, 1 H)

### Beispiel 53A

### 6-Chlor-1-(2-fluorbenzyl)-1H-indazol-3-carbonitril

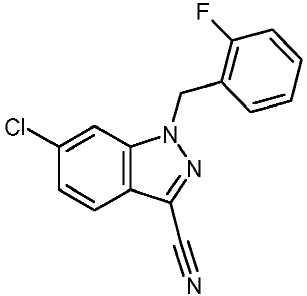

Unter Argon wurden zu einer Mischung von 2.3 g (12.9 mmol) 6-Chlor-1H-indazol-3-carbonitril (WO 2011/149921, Expl. 58C) und 2.15 g (15.5 mmol) Kaliumcarbonat in 40 ml DMF 2.45 ml (14.2 mmol) 2-Fluorbenzylbromid, gelöst in 10 ml DMF, gegeben und es wurde über Nacht bei RT gerührt. Dann wurde auf 90 ml Wasser gegossen und 30 min bei Raumtemperatur gerührt. Der ausgefallene Niederschlag wurde abfiltriert, mit Wasser gewaschen und über Nacht am Hochvakuum getrocknet. Es wurden 3.77 g der Titelverbindung als Rohprodukt erhalten.
LC-MS (Methode 1): Rₜ=1.22 min; MS (ESIpos): m/z = 286 (M+H)⁺
¹H-NMR (400MHz, DMSO-d₆): δ [ppm] = 5.87 (s, 2 H), 7.16 - 7.36 (m, 3 H), 7.37 - 7.50 (m, 2 H), 7.95 (d, 1 H), 8.26 (s, 1 H).

### Beispiel 54A

### 6-Chlor-1-(2-fluorbenzyl)-1H-indazol-3-carboximidamidacetat(1:1)

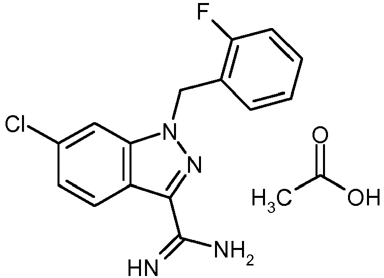

Unter Argon wurden zu 0.71 g (13.20 mmol) Natriummethylat in 60 ml Methanol 3.77 g (13.20 mmol, theor. 12.95 mmol) Beispiel 53A gegeben und es wurde über Nacht bei RT gerührt. Dann wurden 0.85 g (15.83 mmol) Ammoniumchlorid und 2.95 ml (51.46 mmol) Essigsäure zugegeben und über Nacht bei 80°C gerührt. Es wurde abgekühlt, im Vakuum eingeengt, mit Essisäureethylester und 1M wässriger Natronlauge versetzt und 30 min bei Raumtemperatur gerührt. Der Feststoff wurde abfilrtiert, mit Essigsäureethylester gewaschen und über Nacht im Hochvakuum getrocknet. Es wurden 2.56 g der Titelverbindung erhalten (55% d. Th.).
LC-MS (Methode 1): Rₜ=0.70 min; MS (ESIpos): m/z = 303 (M+H)⁺
¹H-NMR (400MHz, DMSO-d₆): δ [ppm] = 1.82 (s, 3 H), 5.80 (s, 2 H), 7.06 - 7.20 (m, 2 H), 7.20 - 7.28 (m, 1 H), 7.28 - 7.43 (m, 2 H), 8.03 (s, 1 H), 8.22 (d, 1 H).

### Beispiel 55A

### 6-Chlor-1-(2-fluorbenzyl)-1H-indazol-3-carboximidohydrazid

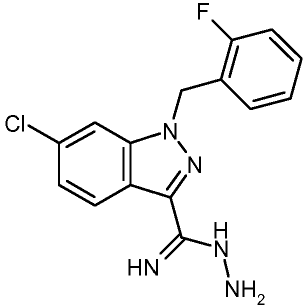

Bei 0°C wurden 500 mg (1.38 mmol) Beispiel 54A in 15 ml Ethanol mit 0.77 ml (5.51 mmol) Triethylamin und 0.08 ml (1.38 mmol) 80%-igem Hydrazinhydrat versetzt und es wurde erst 10 min bei 0°C und dann über Nacht bei Raumtemperatur gerührt. Anschließend wurde 10%ige wäßr.Natriumchloridlösung zugegeben und zweimal mit Essigsäureethylester extrahiert. Die vereinigten organischen Phasen wurden mit 10%iger wässriger Natriumchloridlösung gewaschen, über Natriumsulfat getrocknet, bei Raumtemperatur im Vakuum eingeengt und über Nacht im Hochvakuum getrocknet. Es wurden 408 mg (93% d. Th.) der Titelverbindung erhalten.
LC-MS (Methode 1): Rₜ=0.75 min; MS (ESIpos): m/z = 318 (M+H)⁺
¹H-NMR (400MHz, DMSO-d₆): δ [ppm] = 5.31 (br. s., 2 H), 5.44 (br. s, 2 H), 5.70 (s, 2 H), 6.99 - 7.06 (m, 1 H), 7.10 - 7.27 (m, 3 H), 7.31 - 7.40 (m, 1 H), 7.88 (d, 1 H), 8.19 (d, 1 H).

### Beispiel 56A

### Methyl-2-{3-[6-chlor-1-(2-fluorbenzyl)-1H-indazol-3-yl]-5-hydroxy-1,2,4-triazin-6-yl}-2-methylpropanoat

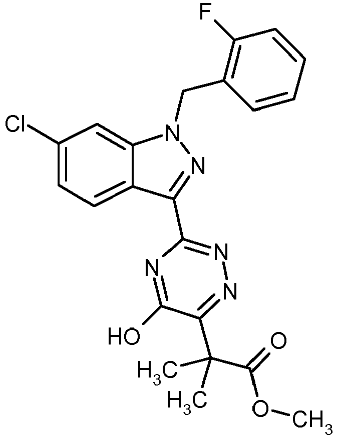

479.7 mg (2.55 mmol) Dimethyl-2,2-dimethyl-3-oxobutandioat (Helv. Chim. Acta, 1959, 42, 2584) in 10 ml Ethanol wurden zum Rückfluß erhitzt, mit einer Suspension von 405 mg (1.28 mmol) Beispiel 55A in 10 ml Ethanol versetzt und über Nacht am Rückfluss gerührt. Nach dem Abkühlen wurde der ausgefallene Feststoff abfiltriert, mit Ethanol und Ether gewaschen und getrocknet. Ausbeute: 198.4 mg (34% d. Th.). Nach Einengen des Filtrates wurden weitere 527 mg Rohprodukt der Titelverbindung erhalten.
LC-MS (Methode 1): Rₜ=1.17 min; MS (ESIpos): m/z = 456 (M+H)⁺

### Beispiel 57A

### 6-Chlor-1-[(3-fluorpyridin-2-yl)methyl]-1H-indazol-3-carbonitril

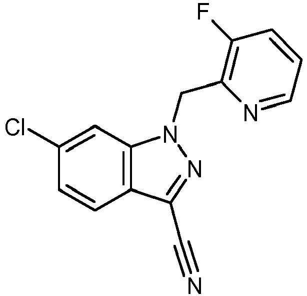

2.3 g (12.95 mmol) 6-Chlor-1H-indazol-3-carbonitril (WO 2011/149921, Expl. 58C) wurden in Analogie zu Beispiel 53A mit 2.07 g (14.25 mmol) 2-(Chlormethyl)-3-fluorpyridin umgesetzt. Es wurden 3.44 g der Titelverbindung erhalten (93% d. Th.).
LC-MS (Methode 1): Rₜ=1.09 min; MS (ESIpos): m/z = 287 (M+H)⁺
¹H-NMR (400MHz, DMSO-d₆): δ [ppm] = 6.05 (s, 2 H), 7.41 - 7.51 (m, 2 H), 7.76 - 7.85 (m, 1H), 7.96 (d, 1 H), 8.22 (s, 1 H), 8.29 (d, 1 H).

### Beispiel 58A

### 6-Chlor-1-[(3-fluorpyridin-2-yl)methyl]-1H-indazol-3-carboximidamid Acetat

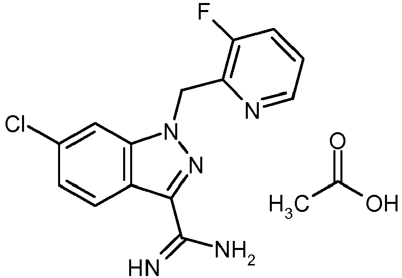

3.44 g (12.0 mmol) Beispiel 57A wurden in Analogie zu Beispiel 54A umgesetzt. Es wurden 3.02 g (69% d. Th.) der Titelverbindung erhalten.
LC-MS (Methode 1): Rₜ=0.73 min; MS (ESIpos): m/z = 303 (M+H)⁺
¹H-NMR (400MHz, DMSO-d₆): δ [ppm] = 1.78 (s, 3 H), 5.94 (s, 2 H), 7.30 (dd, 1 H), 7.45 (dt, 1 H), 7.78 (t, 1 H), 7.99 (s, 1 H), 8.24 (d, 1 H), 8.30 (d, 1 H).

### Beispiel 59A

### 6-Chlor-1-[(3-fluorpyridin-2-yl)methyl]-1H-indazol-3-carboximidohydrazid

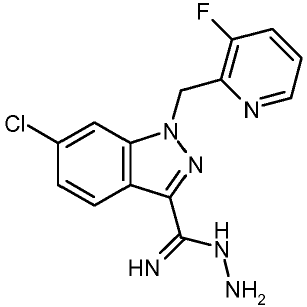

500 mg (1.37 mmol) Beispiel 58A wurden in Analogie zu Beispiel 55A umgesetzt. Es wurden 393.3 mg der Titelverbindung erhalten (90% d. Th.).
LC-MS (Methode 1): Rₜ=0.65 min; MS (ESIpos): m/z = 319 (M+H)⁺
¹H-NMR (400MHz, DMSO-d₆): δ [ppm] = 5.26-5.57 (m, 3 H), 5.85 (s, 2 H), 7.18 (dd, 1 H), 7.40 -7.48 (m, 1 H), 7.76 (t, 1 H), 7.87 (s, 1 H), 8.18 (d, 1 H), 8.30 (d, 1 H).

### Beispiel 60A

### Methyl-2-(3-{6-chlor-1-[(3-fluorpyridin-2-yl)methyl]-1H-indazol-3-yl}-5-hydroxy-1,2,4-triazin-6-yl)-2-methylpropanoat

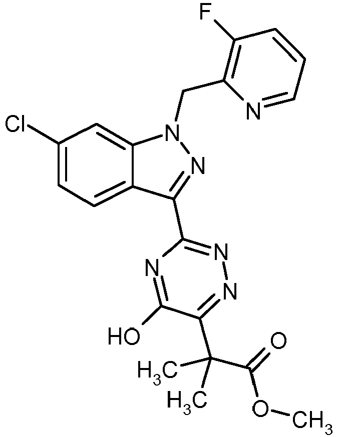

391 mg (1.23 mmol) Beispiel 59A wurden in Analogie zu Beispiel 56A umgesetzt. Die Titelverbindung wurde als Rohprodukt (685.2 mg) nach Einengen der Reaktionslösung erhalten.
LC-MS (Methode 1): Rₜ=1.05 min; MS (ESIpos): m/z = 457 (M+H)⁺

### Beispiel 61A

### 6-Chlor-1-(3,3,4,4,4-pentafluorbutyl)-1H-indazol-3-carboximidohydrazid

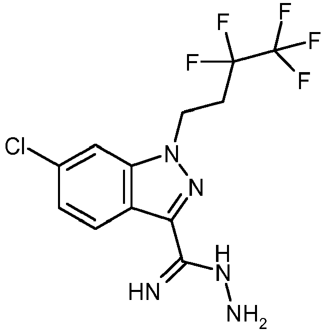

1 g (2.08 mmol, Reinheit 71%) 6-Chlor-1-(3,3,4,4,4-pentafluorbutyl)-1H-indazol-3-carboximidamid (WO 2011/ 149921 Expl.58E) wurden analog Beispiel 55A umgesetzt. Erhalten wurden 1.04 g der Titelverbindung als Rohprodukt.
LC-MS (Methode 1): Rₜ=0.77 min; MS (ESIpos): m/z = 356 (M+H)⁺

### Beispiel 62A

### Methyl-2-{3-[6-chlor-1-(3,3,4,4,4-pentafluorbutyl)-1H-indazol-3-yl]-5-hydroxy-1,2,4-triazin-6-yl}-2-methylpropanoat

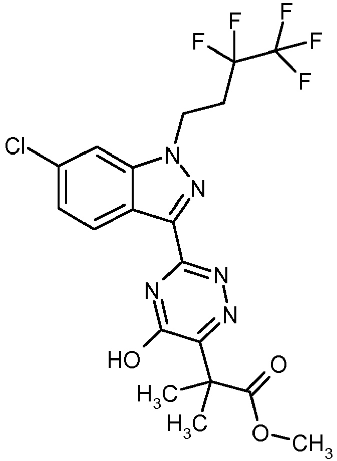

629.6 mg (1.77 mmol) Beispiel 61A (Rohprodukt) wurden analog Beispiel 56A umgesetzt. Erhalten wurden 488.4 mg (56% d. Th.) der Titelverbindung als Rohprodukt.
LC-MS (Methode 1): Rₜ = 1.17 min; MS (ESIpos): m/z = 494 (M+H)⁺

### Ausführungsbeispiele

### Beispiel 1

### 2-[5-Chlor-3-(2,3,6-trifluorbenzyl)-1H-indazol-1-yl]-4-iod-5,5-dimethyl-5,7-dihydro-6H-pyrrolo[2,3-d]pyrimidin-6-on

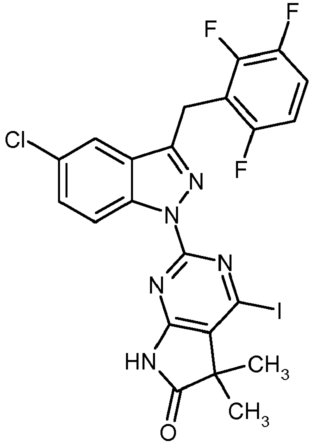

100 mg (0.211 mmol) Beispiel 3A wurden in iso-Pentylnitrit (0.612 ml) und Diiodmethan (1.60 ml) vorgelegt und über Nacht auf 85°C erhitzt. Nach Abkühlen wurde mittels präparativer HPLC gereinigt (Acetonitril:Wasser (+0.05 % Ameisensäure) - Gradient). Es wurden 53 mg der Titelverbindung erhalten (42% d. Th.).
LC-MS (Methode 2): Rₜ = 1.44 min; MS (ESIpos): m/z = 584 (M+H)⁺
¹H-NMR (400 MHz, DMSO-d₆): δ [ppm] = 1.40 (s, 6H), 4.50 (s, 2H), 7.19-7.24 (m, 1H), 7.47-7.53 (m, 1H), 7.72 (dd, 1H), 8.00 (d, 1H), 8.56 (d, 1H), 11.89 (s, 1H).

### Beispiel 2

### 2-[5-Chlor-3-(2,3,6-trifluorbenzyl)-1H-indazol-1-yl]-5,5-dimethyl-5,7-dihydro-6H-pyrrolo[2,3-d]pyrimidin-6-on

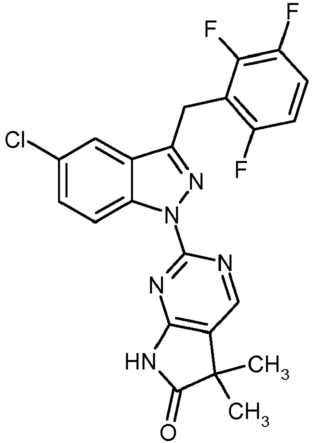

Eine Lösung von 52 mg (0.089 mmol) Beispiel 1 in DMF (9 ml) wurde zu 21.4 mg Palladium auf Kohle (10%-ig) in DMF (1 ml) gegeben und 5 h bei Wasserstoff-Normaldruck hydriert. Anschliessend wurde über Celite filtriert, mit DMF gewaschen und bis zur Trockene eingeengt. Der Rückstand wurde mittels präparativer HPLC gereinigt (Acetonitril:Wasser (+0.05 % Ameisensäure) - Gradient). Es wurden 29 mg der Titelverbindung erhalten (66% d. Th.).
LC-MS (Methode 2): Rₜ = 1.28 min; MS (ESIpos): m/z = 458 (M+H)⁺
¹H-NMR (400 MHz, DMSO-d₆): δ [ppm] = 1.36 (s, 6H), 4.49 (s, 2H), 7.19-7.23 (m, 1H), 7.46-7.53 (m, 1H), 7.65 (dd, 1H), 8.00 (d, 1H), 8.52 (s, 1H), 8.65 (d, 1H), 11.72 (s, 1H).

### Beispiel 3

### 2-[3-(2-Fluorbenzyl)-1H-indazol-1-yl]-4-iod-5,5-dimethyl-5,7-dihydro-6H-pyrrolo[2,3-d]pyrimidin-6-on

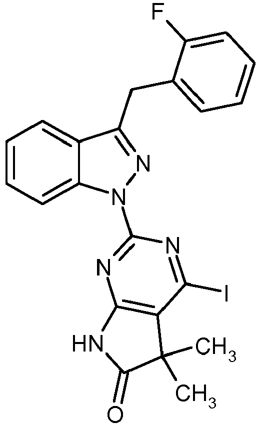

500 mg (1.242 mmol) von Beispiel 7A wurden in iso-Pentylnitrit (3.552 ml) und Diiodmethan (9.430 ml) vorgelegt und über Nacht auf 85°C erhitzt. Nach Abkühlen wurde das Reaktionsgemisch über Kieselgel filtriert (Dichlormethan:Methanol - Gradient) und eingeengt. Der Rückstand wurde mit Dichlormethan und Methanol versetzt und für 10 min bei Raumtemperatur gerührt. Der gebildete Feststoff wurde abfiltriert und mit Dichlormethan und Methanol nachgewaschen. Das Filtrat wurde eingeengt. Dieser Rückstand wurde dann mit Methanol und Acetonitril versetzt. Es bildete sich erneut ein Niederschlag, welcher abgesaugt wurde und mit Acetonitril nachgewaschen wurde. Nach Trocknung im Hochvakuum wurden 127 mg der Titelverbindung erhalten (18% d. Th.). Das Filtrat wurde eingeengt, so wurden weitere 334 mg der Titelverbindung in 57%iger Reinheit (30% d. Th.) erhalten.
LC-MS (Methode 1): Rₜ = 1.31 min; MS (ESIpos): m/z = 514 (M+H)⁺
¹H-NMR (400 MHz, DMSO-d₆): δ [ppm] = 1.42 (s, 6H), 4.43 (s, 2H), 7.13-7.23 (m, 2H), 7.29-7.41 (m, 3H), 7.62 (t, 1H), 7.74 (d, 1H), 8.58 (d, 1H), 11.89 (s, 1H).

Neben der Titelverbindung wurden 57 mg (9 % d. Th., 86%ige Reinheit) 2-[3-(2-Fluorbenzyl)-1H-indazol-1-yl]-4-hydroxy-5,5-dimethyl-5,7-dihydro-6H-pyrrolo[2,3-d]pyrimidin-6-on (Beispiel 4) erhalten.

### Beispiel 5

### 2-[3-(2-Fluorbenzyl)-1H-indazol-1-yl]-5,5-dimethyl-5,7-dihydro-6H-pyrrolo[2,3-d]pyrimidin-6-on

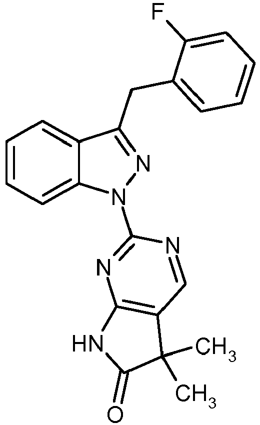

60 mg (0.117 mmol) von Beispiel 3 wurden in Analogie zur Vorschrift unter Beispiel 2 hydriert. Es wurden 14 mg der Titelverbindung erhalten (45% d. Th.).
LC-MS (Methode 2): Rₜ = 1.13 min; MS (ESIpos): m/z = 388 (M+H)⁺
¹H-NMR (400 MHz, DMSO-d₆): δ [ppm] = 1.38 (s, 6H), 4.42 (s, 2H), 7.14-7.22 (m, 2H), 7.28-7.33 (m, 2H), 7.40 (t, 1H), 7.57 (t, 1H), 7.76 (d, 1H), 8.54 (s, 1H), 8.67 (d, 1H), 11.71 (s, 1H).

### Beispiel 6

### 2-[5-Fluor-3-(2-fluorbenzyl)-1H-indazol-1-yl]-4-iod-5,5-dimethyl-5,7-dihydro-6H-pyrrolo[2,3-d]pyrimidin-6-on

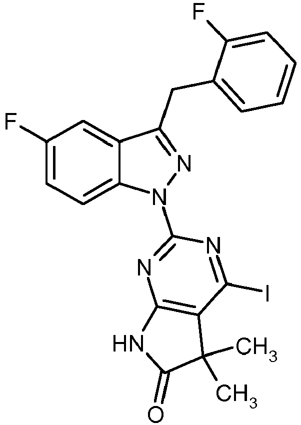

500 mg (1.189 mmol) von Beispiel 11A wurden in iso-Pentylnitrit (3.40 ml) und Diiodmethan (9.027 ml) vorgelegt und über Nacht auf 85°C erhitzt. Nach Abkühlen wurde über Kieselgel filtriert (Dichlormethan:Methanol - Gradient) und eingeengt. Der Rückstand wurde mittels präparativer HPLC gereinigt (Acetonitril:Wasser (+0.05 % Ameisensäure) - Gradient). Es wurden 274 mg der Titelverbindung erhalten (43% d. Th.).
LC-MS (Methode 1): Rₜ = 1.33 min; MS (ESIpos): m/z = 532 (M+H)⁺
¹H-NMR (400 MHz, DMSO-d₆): δ [ppm] = 1.41 (s, 6H), 4.41 (s, 2H), 7.14-7.23 (m, 2H), 7.29-7.35 (m, 1H), 7.39-7.43 (ddd, 1H), 7.52-7.61 (m, 2H), 8.58 (d, 1H), 11.91 (s, 1H).

Neben der Titelverbindung wurden 72 mg (14 % d. Th., 83%ige Reinheit) 2-[5-Fluor-3-(2-fluorbenzyl)-1H-indazol-1-yl]-4-hydroxy-5,5-dimethyl-5,7-dihydro-6H-pyrrolo[2,3-d]pyrimidin-6-on (Beispiel 7) erhalten.

### Beispiel 8

### 2-[5-Fluor-3-(2-fluorbenzyl)-1H-indazol-1-yl]-5,5-dimethyl-5,7-dihydro-6H-pyrrolo[2,3-d]pyrimidin-6-on

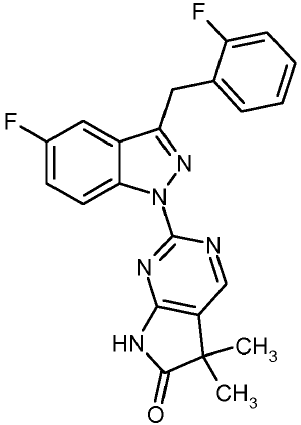

60 mg (0.113 mmol) von Beispiel 6 wurden in Analogie zur Vorschrift unter Beispiel 2 hydriert. Es wurden 34 mg der Titelverbindung erhalten (76% d. Th.).
LC-MS (Methode 1): Rₜ = 1.16 min; MS (ESIpos): m/z = 406 (M+H)⁺
¹H-NMR (400 MHz, DMSO-d₆): δ [ppm] = 1.38 (s, 6H), 4.41 (s, 2H), 7.15-7.23 (m, 2H), 7.29-7.34 (m, 1H), 7.40-7.46 (m, 1H), 7.47-7.51 (m, 1H), 7.58 (dd, 1H), 8.53 (s, 1H), 8.67 (dd, 1H), 11.73 (s br, 1H).

### Beispiel 9

### 3-[6-Fluor-3-(2-fluorbenzyl)-1H-pyrazolo[4,3-b]pyridin-1-yl]-7,7-dimethyl-5,7-dihydro-6H-pyrrolo[2,3-e][1,2,4]triazin-6-on

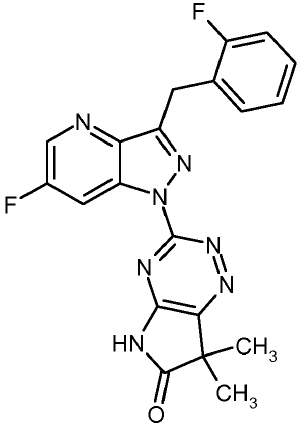

66 mg (0.125 mmol) Beispiel 49A wurden in 4 ml Acetonitril und 1 ml Wasser gelöst und mit 68 mg (0.125 mmol) Ammoniumcer(IV)nitrat versetzt und 1.5h bei RT gerührt. Nach Zugabe von weiteren 68 mg Ammoniumcer(IV)nitrat wurde über Nacht bei RT gerührt. Erneut wurden 68 mg Ammoniumcer(IV)nitrat zugegeben und 1.5h bei RT gerührt und dieser Prozeß noch einmal wiederholt. Schließlich wurden 55.5 mg (0.750 mmol) Natriumhydrogensulfidhydrat zugesetzt, 30 min bei RT nachgerührt und 2 d stehengelassen. Die Reaktionsmischung wurde mit Acetonitril verdünnt und über Kieselgur filtriert. Das Filtrat wurde eingeengt und der Rückstand per präparativer HPLC (Reprosil C18, Gradient aus Acetonitril/0.01% aq. Ameisensäure) gereinigt.
Ausb.: 18 mg (35 % d. Th.)
LC-MS (Methode 1): Rₜ = 1.03 min; MS (ESIpos): m/z = 408 (M+H)⁺
¹H NMR (500 MHz, DMSO-*d*₆) δ [ppm] = 1.45 (s, 6 H), 4.48 (s, 2 H), 7.10 - 7.23 (m, 2 H), 7.30 (q, 1 H), 7.42 (t, 1 H), 8.70 (d, 1 H), 8.70 (d, 1 H), 8.77 (br. s., 1 H), 12.38 (br. s., 1 H)

### Beispiel 10

### 2-[1-(2-Fluorbenzyl)-1,4,5,6-tetrahydrocyclopenta[c]pyrazol-3-yl]-4-iod-5,5-dimethyl-5,7-dihydro-6H-pyrrolo[2,3-d]pyrimidin-6-on

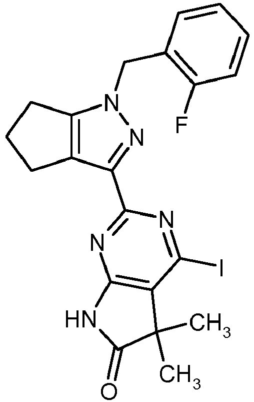

285 mg (0.68 mmol) 4-Amino-2-[1-(2-fluorbenzyl)-1,4,5,6-tetrahydrocyclopenta[c]pyrazol-3-yl]-5,5-dimethyl-5,7-dihydro-6H-pyrrolo[2,3-d]pyrimidin-6-on wurden in absolutem Dimethoxyethan vorgelegt und mit 800 mg (6.83 mmol) Isopentylnitrit, 87 mg (0.34 mmol) Iod, 39 mg (0.21 mmol) Kupfer(I)iodid sowie 177 mg (0.68 mmol) Cäsiumiodid versetzt. Das Gemisch wurde 40 min bei 100 °C gerührt. Das Gemisch wurde am Rotationsverdampfer eingeengt, der Rückstand in Dichlormethan aufgenommen und mit 5%-iger wässriger Natriumthiosulfat-Lösung und gesättigter wässriger Natriumchlorid-Lösung gewaschen. Die organische Phase wurde über Natriumsulfat getrocknet, eingeengt und mittels präparativer HPLC gereinigt (Eluent: Acetonitril/Wasser mit 0.1% Essigsäure, Gradient 20:80 → 100:0). Es wurden 148 mg (40% d. Th.) der Zielverbindung erhalten.
LC-MS (Methode 1): Rₜ = 1.23 min; MS (ESIpos): m/z = 504 (M+H)⁺

### Beispiel 11

### 2-[1-(2-Fluorbenzyl)-1,4,5,6-tetrahydrocyclopenta[c]pyrazol-3-yl]-5,5-dimethyl-5,7-dihydro-6H-pyrrolo[2,3-d]pyrimidin-6-on

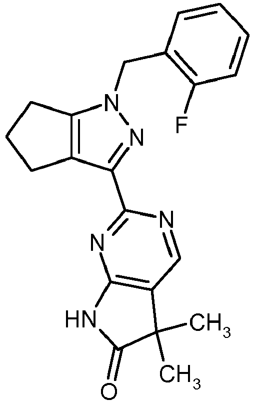

45 mg (0.08 mmol) 2-[1-(2-Fluorbenzyl)-1,4,5,6-tetrahydrocyclopenta[c]pyrazol-3-yl]-4-iod-5,5-dimethyl-5,7-dihydro-6H-pyrrolo[2,3-d]pyrimidin-6-on wurden in 5 ml absolutem DMF gelöst, mit 18 mg (0.02 mmol) 10%-igem Palladium auf Kohle versetzt und unter Wasserstoff-Normaldruck über Nacht hydriert. Das Reaktionsgemisch wurde über Celite filtriert, eingeengt und der Rückstand mittels präparativer HPLC (Eluent: Acetonitril/Wasser, Gradient 20:80 → 100:0) gereinigt. Es wurden 25 mg der Zielverbindung erhalten (80% d. Th.).
LC-MS (Methode 1): Rₜ = 0.99 min; MS (ESIpos): m/z = 378 (M+H)⁺
¹H-NMR (400MHz, DMSO-d₆): δ [ppm]= 1.36 (s, 6H), 2.64-2.68 (m, 2H), 2.79-2.82 (m, 2H), 5.41 (s, 2H), 7.21-7.32 (m, 3H), 7.39-7.45 (m, 1H), 8.56 (s, 1H), 12.00 (s br, 1H).

### Beispiel 12

### 3-[1-(2-Fluorbenzyl)-1,4,5,6-tetrahydrocyclopenta[c]pyrazol-3-yl]-7,7-dimethyl-5,7-dihydro-6H-pyrrolo[2,3-e][1,2,4]triazin-6-on

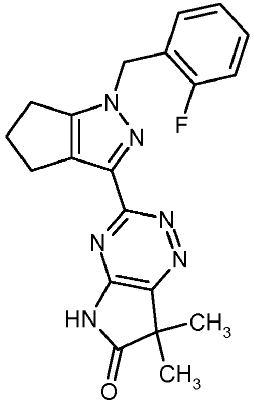

Es wurden 48 mg (0.06 mmol, Reinheit 54%) Methyl-2-{3-[1-(2-fluorbenzyl)-1,4,5,6-tetrahydrocyclopenta[c]pyrazol-3-yl]-5-hydroxy-1,2,4-triazin-6-yl}-2-methylpropanoat mit 0.5 ml (5.36 mmol) Phosphorylchlorid versetzt und 2.5 h bei RT gerührt. Die Reaktionslösung wurde mit 10 ml trockenem Acetonitril verdünnt, unter Eiskühlung langsam in 5 ml einer 25%-igen wässrigen Ammoniak-Lösung getropft und 8 h bei RT gerührt. Das Reaktionsgemisch wurde am Rotationsverdampfer eingeengt und der Rückstand zwischen Dichlormethan/Wasser verteilt. Die organische Phase wurde über Natriumsulfat getrocknet und am Rotationsverdampfer eingeengt. Der Rückstand wurde mittels präparativer HPLC (Eluent: Acetonitril/Wasser mit 0.1% Salzsäure, Gradient 20:80 → 100:0) gereinigt. Es wurden 4.5 mg der Zielverbindung erhalten (18% d. Th.).
LC-MS (Methode 1): Rₜ = 0.96 min; MS (ESIpos): m/z = 379 (M+H)⁺
¹H-NMR (400MHz, DMSO-d₆): δ [ppm]= 1.40 (s, 6H), 2.66-2.70 (m, 2H), 2.79-2.83 (m, 2H), 5.36 (s, 2H), 7.19-7.31 (m, 3H), 7.37-7.43 (m, 1H), 12.00 (s br, 1H).

### Beispiel 13

### 2-[1-(2-Fluorbenzyl)-6-methyl-1H-pyrazolo[3,4-d]pyrimidin-3-yl]-4-iod-5,5-dimethyl-5,7-dihydro-6H-pyrrolo[2,3-d]pyrimidin-6-on

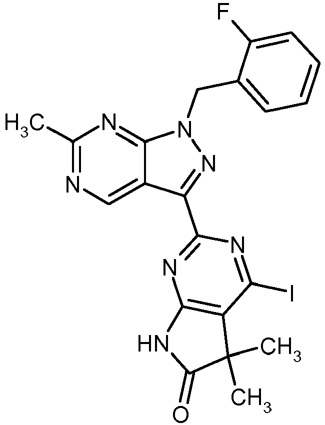

70 mg (0.17 mmol) 4-Amino-2-[1-(2-fluorbenzyl)-6-methyl-1H-pyrazolo[3,4-d]pyrimidin-3-yl]-5,5-dimethyl-5,7-dihydro-6H-pyrrolo[2,3-d]pyrimidin-6-on wurden mit 3.770 g (14.08 mmol) Diiodmethan sowie 411 mg (3.51 mmol) Isopentylnitrit versetzt. Das Gemisch wurde 8 h bei 85 °C gerührt. Nach dem Abkühlen wurde mit Acetonitril verdünnt und das Gemisch mittels präparativer HPLC gereinigt (Eluent: Acetonitril/Wasser mit Gradient 30:70 → 95:5). Es wurden 35 mg (24% d. Th.) der Zielverbindung erhalten.
LC-MS (Methode 3): Rₜ = 2.37 min; MS (ESIpos): m/z = 530 (M+H)⁺

Ausserdem wurden 10 mg (14% d. Th.) 2-[1-(2-Fluorbenzyl)-6-methyl-1H-pyrazolo[3,4-d]pyrimidin-3-yl]-4-hydroxy-5,5-dimethyl-5,7-dihydro-6H-pyrrolo[2,3-d]pyrimidin-6-on (s. Beispiel 20) erhalten.

### Beispiel 14

### 2-[1-(2,3-Difluorbenzyl)-6-methyl-1H-pyrazolo[3,4-d]pyrimidin-3-yl]-4-iod-5,5-dimethyl-5,7-dihydro-6H-pyrrolo[2,3-d]pyrimidin-6-on

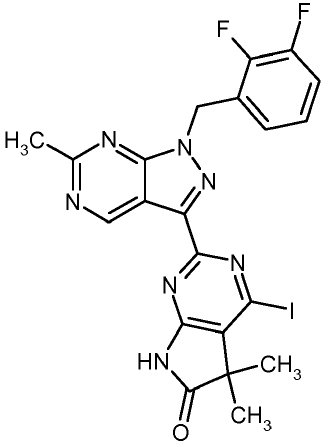

150 mg (0.34 mmol) 4-Amino-2-[1-(2,3-difluorbenzyl)-6-methyl-1H-pyrazolo[3,4-d]pyrimidin-3-yl]-5,5-dimethyl-5,7-dihydro-6H-pyrrolo[2,3-d]pyrimidin-6-on wurden mit 6.650 g (19.86 mmol) Diiodmethan sowie 402 mg (3.44 mmol) Isopentylnitrit versetzt. Das Gemisch wurde 8 h bei 85 °C gerührt. Es wurden 70 mg (0.69 mmol) Triethylamin addiert und erneut bei 85°C unter Rückfluss gekocht. Nach dem Abkühlen wurde mit Acetonitril verdünnt und das Gemisch mittels päparativer HPLC gereinigt (Eluent: Acetonitril/Wasser mit, Gradient 30:70 → 95:5). Es wurden 44 mg (Reinheit 67%, 16% d. Th.) der Zielverbindung erhalten.
LC-MS (Methode 1): Rₜ = 1.14 min; MS (ESIpos): m/z = 548 (M+H)⁺

### Beispiel 15

### 2-[1-(2-Fluorbenzyl)-6-methyl-1H-pyrazolo[3,4-d]pyrimidin-3-yl]-5,5-dimethyl-5,7-dihydro-6H-pyrrolo[2,3-d]pyrimidin-6-on

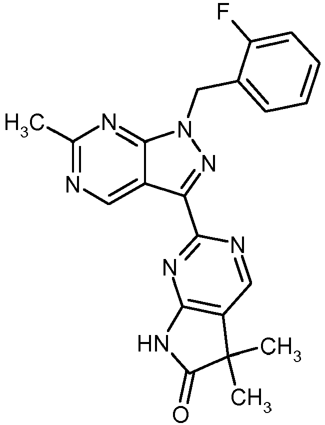

35 mg (0.07 mmol) 2-[1-(2-Fluorbenzyl)-6-methyl-1H-pyrazolo[3,4-d]pyrimidin-3-yl]-4-iod-5,5-dimethyl-5,7-dihydro-6H-pyrrolo[2,3-d]pyrimidin-6-on wurden in 8 ml absolutem DMF gelöst, mit 50 mg 10%-igem Palladium auf Kohle versetzt und unter Wasserstoff-Normaldruck über Nacht hydriert. Das Reaktionsgemisch wurde über Celite filtriert und eingeengt. Der Rückstand wurde mit 1 ml Acetonitril verrührt, abgesaugt und im Hochvakuum getrocknet. Es wurden 5 mg der Zielverbindung erhalten (Reinheit 92%, 17% d. Th.).
LC-MS (Methode 1): Rₜ = 0.94 min; MS (ESIpos): m/z = 404 (M+H)⁺

### Beispiel 16

### 3-[1-(2-Fluorbenzyl)-6-methyl-1H-pyrazolo[3,4-d]pyrimidin-3-yl]-7,7-dimethyl-5,7-dihydro-6H-pyrrolo[2,3-e][1,2,4]triazin-6-on

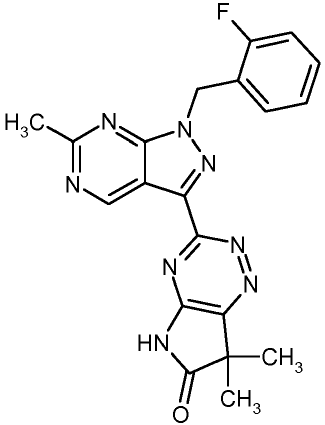

Es wurden 182 mg (0.42 mmol) Methyl-2-{3-[1-(2-fluorbenzyl)-6-methyl-1H-pyrazolo[3,4-d]pyrimidin-3-yl]-5-hydroxy-1,2,4-triazin-6-yl}-2-methylpropanoat mit 3.8 ml (40.87 mmol) Phosphorylchlorid versetzt und 2.5 h bei RT gerührt. Die Reaktionslösung wurde mit 20 ml trockenem Acetonitril verdünnt, unter Eiskühlung langsam in 40 ml einer 25%-igen wässrigen Ammoniak-Lösung getropft und 2 h bei RT gerührt. Das Reaktionsgemisch wurde am Rotationsverdampfer eingeengt und der Niederschlag abfiltriert. Der Rückstand wurde mit DMF/Methanol verrührt, abgesaugt und im Hochvakuum getrocknet.. Es wurden 98 mg der Zielverbindung erhalten (Reinheit 92%, 53% d. Th.).
LC-MS (Methode 2) Rₜ = 0.89 min; MS (ESIpos): m/z = 405 (M+H)⁺
¹H-NMR (400MHz, DMSO-d₆): δ [ppm]= 1.45 (s, 6H), 2.81 (s, 3H), 5.81 (s, 2H), 7.16-7.31 (m, 3H), 7.36-7.42 (m, 1H), 9.71 (s, 1H), 12.21 (s, 1H).

### Beispiel 17

### 2-[1-(2,3-Difluorbenzyl)-6-methyl-1H-pyrazolo[3,4-d]pyrimidin-3-yl]-5,5-dimethyl-5,7-dihydro-6H-pyrrolo[2,3-d]pyrimidin-6-on

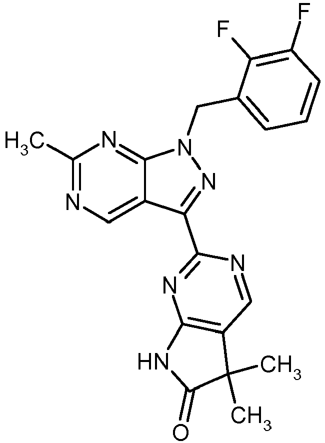

44 mg (0.05 mmol) 2-[1-(2,3-Difluorbenzyl)-6-methyl-1H-pyrazolo[3,4-d]pyrimidin-3-yl]-4-iod-5,5-dimethyl-5,7-dihydro-6H-pyrrolo[2,3-d]pyrimidin-6-on wurden in 8 ml absolutem DMF gelöst, mit 11 mg (0.01 mmol) 10%-igem Palladium auf Kohle versetzt und unter Wasserstoff-Normaldruck für 4 h hydriert. Das Reaktionsgemisch wurde filtriert und mittels präparativer HPLC (Eluent: Acetonitril/Wasser, Gradient 20:80 → 100:0) gereinigt. Es wurden 6 mg der Zielverbindung erhalten (Reinheit 81%, 21% d. Th.).
LC-MS (Methode 1): Rₜ = 0.95 min; MS (ESIpos): m/z = 422 (M+H)⁺
¹H-NMR (400MHz, DMSO-d₆): δ [ppm]= 1.38 (s, 6H), 2.80 (s, 3H), 5.83 (s, 2H), 7.09-7.11 (m, 1H), 7.17-7.22 (m, 1H), 7.39-7.46 (m, 1H), 8.67 (s, 1H), 9.73 (s, 1H), 11.64 (s, 1H).

### Beispiel 18

### 3-[1-(2,3-Difluorbenzyl)-6-methyl-1H-pyrazolo[3,4-d]pyrimidin-3-yl]-7,7-dimethyl-5,7-dihydro-6H-pyrrolo[2,3-e][1,2,4]triazin-6-on

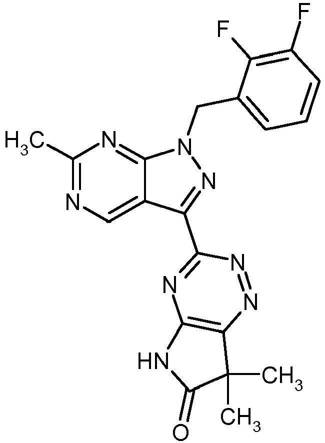

Es wurden 160 mg (Reinheit 89%, 0.313 mmol) Methyl-2-{3-[1-(2,3-difluorbenzyl)-6-methyl-1H-pyrazolo[3,4-d]pyrimidin-3-yl]-5-hydroxy-1,2,4-triazin-6-yl}-2-methylpropanoat mit 4 ml (42.913 mmol) Phosphorylchlorid versetzt und 2.5 h bei RT gerührt. Die Reaktionslösung wurde mit 20 ml trockenem Acetonitril verdünnt, unter Eiskühlung langsam in 40 ml einer 25%-igen wässrigen Ammoniak-Lösung getropft und 1 h bei RT gerührt. Das Reaktionsgemisch wurde am Rotationsverdampfer eingeengt und der Niederschlag abfiltriert. Der Rückstand wurde mit Ethanol/Wasser heiß verrührt, abgesaugt und im Hochvakuum getrocknet. Es wurden 70 mg der Zielverbindung erhalten (52% d. Th.).
LC-MS (Methode 1): Rₜ = 0.92 min; MS (ESIpos): m/z = 423 (M+H)⁺
¹H-NMR (400MHz, DMSO-d₆): δ [ppm]= 1.46 (s, 6H), 2.81 (s, 3H), 5.86 (s, 2H), 7.09-7.19 (m, 1H), 7.17-7.23 (m, 1H), 7.39-7.46 (m, 1H), 9.71 (s, 1H), 12.24 (s br, 1H).

### Beispiel 19

### 3-[1-(2-Fluorbenzyl)-1H-pyrazolo[3,4-d]pyrimidin-3-yl]-7,7-dimethyl-5,7-dihydro-6H-pyrrolo[2,3-e] [1,2,4]triazin-6-on

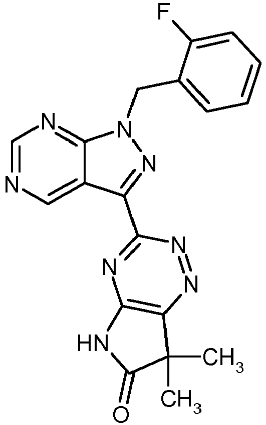

Es wurden 186 mg (0.438 mmol) Methyl-2-{3-[1-(2-fluorbenzyl)-1H-pyrazolo[3,4-d]pyrimidin-3-yl]-5-hydroxy-1,2,4-triazin-6-yl}-2-methylpropanoat mit 4 ml (42.913 mmol) Phosphorylchlorid versetzt und 2.5 h bei RT gerührt. Die Reaktionslösung wurde mit 20 ml trockenem Acetonitril verdünnt, unter Eiskühlung langsam in 30 ml einer 25%-igen wässrigen Ammoniak-Lösung getropft und über Nacht bei RT gerührt. Das Reaktionsgemisch wurde am Rotationsverdampfer eingeengt und der Niederschlag abfiltriert. Es wurden 127 mg der Zielverbindung erhalten (74% d. Th.).
LC-MS (Methode 2) Rt = 0.85 min; MS (ESIpos): m/z = 391 (M+H)+
¹H-NMR (400MHz, DMSO-d6): δ [ppm] = 1.45 (s, 6H), 5.87 (s, 2H), 7.17-7.26 (m, 2H), 7.32-7.42 (m, 2H), 9.21 (s, 1H), 9.83 (s, 1H).

### Beispiel 21

### 3-[5-Fluor-3-(2-fluorbenzyl)-1H-indazol-1-yl]-7,7-dimethyl-5,7-dihydro-6H-pyrrolo[2,3-e] [1,2,4]triazin-6-on

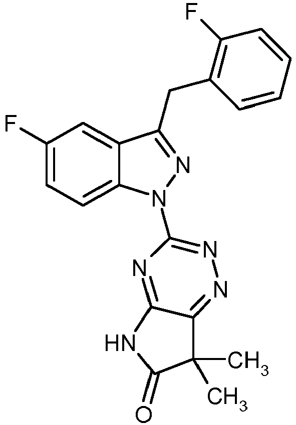

100 mg (0.41 mmol) Beispiel 50A wurden in 0.5 ml NMP gelöst, mit 122 mg (0.614 mmol) Beispiel 47A versetzt und über Nacht bei 80°C gerührt. Danach wurden 50 mg (0.2 mmol) Beispiel 47A zugegeben und 3h bei 100°C gerührt, dann 0.5 ml NMP und 50 mg (0.2 mmol) Beispiel 47A zugegeben und 1h bei 100°C gerührt, schließlich 26 mg (0.1 mmol) Beispiel 47A zugegeben und 3h bei 100°C gerührt. Nach dem Abkühlen wurde mit einem Testansatz aus 10 mg (0.041 mmol) Beispiel 47A vereinigt, etwas aq. 5 M wässrige Ameisensäure zugesetzt und der ausgefallene Feststoff abgetrennt. Das Filtrat wurde über präparativeHPLC (Reprosil C18, Gradient aus Acetonitril/0.01% aq. Ameisensäure) gereinigt und die produkthaltigen Fraktionen eingeengt. Der Rückstand wurde mit Acetonitril am Ultraschallbad digeriert und dann abfiltriert.
Ausb.: 63.5 mg, Feststoff, (35 % d. Th)
LC-MS (Methode 1): Rₜ = 1.11 min; MS (ESIpos): m/z = 407 (M+H)⁺
¹H NMR (400 MHz, DMSO-*d*₆) δ 8ppm] = 1.45 (s, 6 H), 4.43 (s, 2 H), 7.11 - 7.25 (m, 2 H), 7.27 - 7.36 (m, 1 H), 7.44 (t, 1 H), 7.54 (t, 1 H), 7.64 (d, 1 H), 8.60 - 8.73 (m, 1 H), 12.33 (br. s, 1 H).

### Beispiel 22

### 3-[5-Fluor-3-(3,3,4,4,4-pentafluorbutyl)-1H-indazol-1-yl]-7,7-dimethyl-5,7-dihydro-6H-pyrrolo[2,3-e] [1,2,4]triazin-6-on

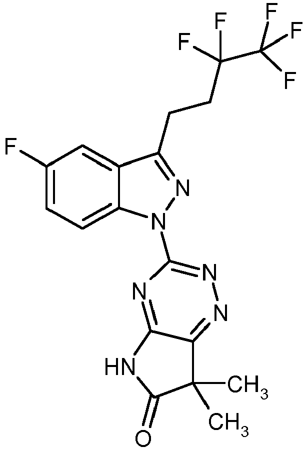

Eine Lösung von 33.5 mg (0.06 mmol) Beispiel 52A in 1 ml Acetonitril wurde bei 0-4°C mit einer vorgekühlten Lösung von 97.6 mg (0.18 mmol) Ammoniumcer(IV)nitrat in 0.25 ml Wasser versetzt, 1 h in diesem Temperaturbereich und über Nacht bei RT gerührt. Dann wurden weitere 97.6 mg (0.18 mmol) Ammoniumcer(IV)nitrat fest zugegeben und 3 h bei RT gerührt. Es wurde Essigsäureethylester zugegeben, zweimal mit gesättigter wässriger Natriumchlorid-Lösung gewaschen und die vereinigten wässrigen Phasen nochmals mit Essigsäureethylester extrahiert. Die vereinigten org. Phasen wurden getrocknet und eingeengt. Der Rückstand wurde per präparativer HPLC (Reprosil C18, Gradient aus Acetonitril/0.01% aq. Ameisensäure) gereinigt. Ausb.: 9.7 mg (37% d. Th.)
LC-MS (Methode 1): Rₜ = 1.14 min; MS (ESIpos): m/z = 445 (M+H)⁺
¹H NMR (400 MHz, DMSO-*d*₆) δ [ppm] = 1.46 (s, 6 H), 2.72 - 2.94 (m, 2 H), 7.51 - 7.62 (m, 1 H), 7.85 - 7.94 (m, 1 H), 8.59 - 8.74 (m, 1 H), 12.34 (br.s., 1 H), eine CH₂-Gruppe partiell von Wassersignal überlagert.

### Beispiel 23

### 4-Ethyl-2-[5-fluor-3-(2-fluorbenzyl)-1H-indazol-1-yl]-5,5-dimethyl-5,7-dihydro-6H-pyrrolo[2,3-d]pyrimidin-6-on

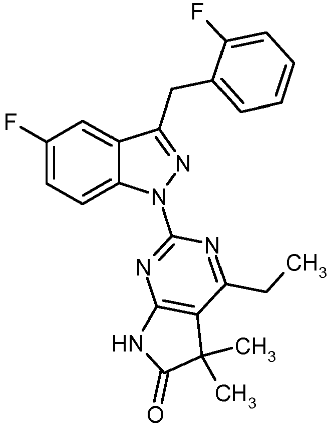

Eine Lösung von 50 mg (0.09 mmol) Beispiel 6 in 2 ml Dioxan wurde bei RT dreimal evakuiert und mit Argon belüftet, mit 2 mg (2.2 µM) [1,1'-Bis(diphenylphosphin)ferrocen]dichlorpalladium(II)-Komplex mit Dichlormethan versetzt, erneut evakuiert und mit Argon belüftet. Dann wurden 340 µl Diethylzink (15%ig in Toluol) in ca. 2 min zugetropft, 10 min bei RT und 16h bei 90°C nachgerührt. Zu der Suspension wurden bei RT nochmals 500µl Diethylzink (15%ig in Toluol) in 2 min zugegeben und über Nacht bei 90°C gerührt. Dieser Prozeß wird noch zweimal mit je 300 µl Diethylzink (15%ig in Toluol) wiederholt, wobei bei der letzten Zudosierung auch weitere 3 mg (3.7 µM) [1,1'-Bis(diphenylphosphin)ferrocen]dichlorpalladium(II)-Komplex mit Dichlormethan zugegeben wurden. Das Reaktionsgemisch wurde mit 2 ml Wasser versetzt und 1h bei RT gerührt. Das Dioxan wurde im Vakuum entfernt und nach Zugabe von Essigsäureethylester und Wasser wurde vom anorganischen Feststoff abfiltriert. Die Phasen wurden getrennt, die Wasserphase noch zweimal mit Essigsäureethylester extrahiert, die vereinigten organischen Phasen getrocknet und eingeengt. Der Rückstand wurde per präparativer HPLC (Reprosil C18, Gradient aus Acetonitril/0.1% aq. Ameisensäure) gereinigt.
Ausb.: 10.5 mg (26% d. Th.).
LC-MS (Methode 1): Rₜ = 1.14 min; MS (ESIpos): m/z = 434 (M+H)⁺
¹H NMR (400 MHz, DMSO-*d*₆) δ [ppm] = 1.30 - 1.52 (m, 9 H), 2.81 (q, 2 H), 4.41 (s, 2 H), 7.10 - 7.25 (m, 2 H), 7.27 - 7.44 (m, 2 H), 7.46 - 7.64 (m, 2 H), 8.73 (dd, 1 H), 11.72 (br. s., 1 H).

### Beispiel 24:

### (Referenzbeispiel) 3-[6-Chlor-1-(2-fluorbenzyl)-1H-indazol-3-yl]-7,7-dimethyl-5,7-dihydro-6H-pyrrolo[2,3-e] [1,2,4]triazin-6-on

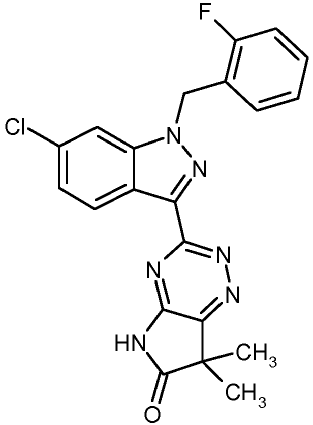

198 mg Feststoff und 527 mg Rohsubstanz (theoretisch 1.28 mmol) von Beispiel 56A wurden in 10 ml (107.3 mmol) Phosphorylchlorid über Nacht bei Raumtemperatur gerührt. Das Zwischenprodukt, Methyl-2-{5-chlor-3-[6-chlor-1-(2-fluorbenzyl)-1H-indazol-3-yl]-1,2,4-triazin-6-yl}-2-methylpropanoat, wurde mittels LC-MS detektiert:
LC-MS (Methode 1): Rₜ = 1.39 min; MS (ESIpos): m/z = 473 (M+H)⁺.

Die Reaktionsmischung wurde mit 50 ml Acetonitril verdünnt und bei 0°C langsam in 70 ml 33%ige wäßr. Ammoniaklösung eingetropft (Temperaturanstieg bis 12°C). Nach einer Nacht Rühren bei Raumtemperatur wurden die Phasen getrennt und die wässrige Phase zweimal mit Essigsäureethylester extrahiert. Die vereinigten organischen Phasen wurden einmal mit gesättigter Natriumchloridlösung gewaschen, über Natriumsulfat getrocknet, im Vakuum eingeengt und mittels präparativer HPLC (Gradient 0.05% Ameisensäure in Wasser / 20-95% Acetonitril) gereinigt. Erhalten wurden 203.4 mg (38% d. Th.) der Titelverbindung.
LC-MS (Methode 1): Rₜ = 1.13 min; MS (ESIpos): m/z = 423 (M+H)⁺
¹H-NMR (400MHz, DMSO-d₆): δ [ppm] = 1.45 (s, 6 H), 5.87 (s, 2 H), 7.10 - 7.30 (m, 3 H), 7.33 - 7.45 (m, 2 H), 8.11 (s, 1 H), 8.51 (d, 1 H), 12.15 (s, 1 H).

### Beispiel 25

### (Referenzbeispiel)

### 3-{6-Chlor-1-[(3-fluorpyridin-2-yl)methyl]-1H-indazol-3-yl} -7,7-dimethyl-5,7-dihydro-6H-pyrrolo[2,3-e][1,2,4]triazin-6-on

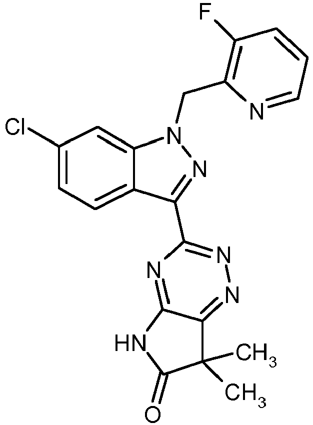

685 mg Rohsubstanz von Beispiel 60A wurden in Analogie zu Beispiel 24 umgesetzt. Es wurden 202 mg der Titelverbindung erhalten (39% d. Th.).
LC-MS (Methode 1): Rₜ = 1.01 min; MS (ESIpos): m/z = 424 (M+H)⁺
¹H-NMR (400MHz, DMSO-d₆): δ [ppm] = 1.45 (s, 6 H), 6.01 (s, 2 H), 7.39 (dd, 1 H), 7.43 - 7-49 (m, 1 H), 7.73 - 7.85 (m, 1 H), 8.04 - 8.14 (m, 1 H), 8.31 (d, 1 H), 8.50 (d, 1 H), 12.13 (s, 1 H).

### Beispiel 26

### (Referenzbeispiel) 3-[6-Chlor-1-(3,3,4,4,4-pentafluorbutyl)-1H-indazol-3-yl]-7,7-dimethyl-5,7-dihydro-6H-pyrrolo[2,3-e] [1,2,4]triazin-6-on

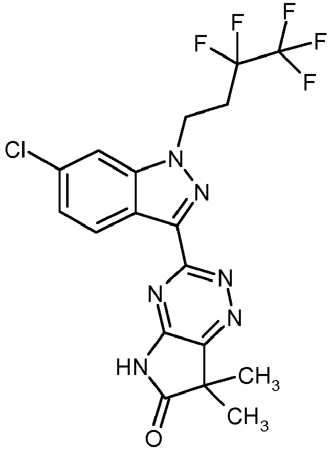

488 mg (0.99 mmol) Beispiel 62A (Rohprodukt) wurden anolog Beispiel 24 umgesetzt. Das Zwischenprodukt, Methyl-2-{5-chlor-3-[6-chlor-1-(3,3,4,4,4-pentafluorbutyl)-1H-indazol-3-yl]-1,2,4-triazin-6-yl}-2-methylpropanoat, wurde mittels LC-MS detektiert: LC-MS (Methode 1): Rₜ = 1.39 min; MS (ESIpos): m/z = 512 (M+H)⁺. Erhalten wurden 180 mg (38% d. Th.) der Titelverbindung.
LC-MS (Methode 1): Rₜ = 1.15 min; MS (ESIpos): m/z = 461 (M+H)⁺
¹H-NMR (400MHz, DMSO-d₆): δ [ppm] = 1.46 (s, 6H), 2.85 - 3.07 (m, 2H), 4.90 (t, 2H), 7.34 - 7.44 (m, 1H), 8.13 (d, 1H), 8.51 (d, 1H), 12.17 (s, 1H).

### Beispiel 27:

### 2-[5-Fluor-3-(2-fluorbenzyl)-1H-indazol-1-yl]-4,5,5-trimethyl-5,7-dihydro-6H-pyrrolo[2,3-d]pyrimidin-6-on

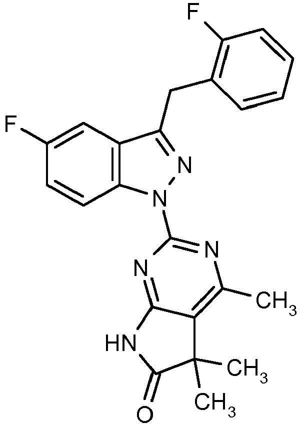

Unter Argon wurden 60 mg (0.11 mmol) Beispiel 6 in 2 ml Dioxan vorgelegt, mit 2.3 mg (2.8 µmol) PdCl₂(dppf)xCH₂Cl₂ sowie tropfenweise mit 0.23 ml einer 2 M Lösung von Dimethylzink in Toluol versetzt und 3 h 25 min in der Mikrowelle auf 120°C erhitzt. Es wurden nochmals 3 mg (3.7 µmol) PdCl₂(dppf)xCH₂Cl₂ und 0.23 ml der 2 M Dimethylzinklösung in Toluol zugegeben und 3 h bei 120°C in der Mikrowelle gerührt. 3 ml Wasser wurden vorsichtig zugesetzt und die Mischung unter vermindertem Druck eingeengt. Der Rückstand wurde in Acetonitril und 5 M wäßr. Ameisensäure aufgenommen, filtriert und das Filtrat über die präparative HPLC (Gradient 0.1% Ameisensäure in Wasser / 10-95% Acetonitril) gereinigt.
Ausb.: 20 mg (42% d. Th.)
LC-MS (Methode 1): Rₜ = 1.21 min; MS (ESIpos): m/z = 420 (M+H)⁺
¹H-NMR (400MHz, DMSO-d₆): δ [ppm] = 1.39 (s, 6H), 4.40 (s, 2H), 7.12 - 7.24 (m, 2H), 7.26 - 7.34 (m, 1H), 7.35 - 7.42 (m, 1H), 7.43 - 7.51 (m, 1H), 7.52 - 7.59 (m, 1H), 8.69 - 8.78 (m, 1H), 11.66 (br. s, 1H).

### Beispiel 28:

### 2-[3-(2-Fluorbenzyl)-1H-indazol-1-yl]-5,5-dimethyl-4-propyl-5,7-dihydro-6H-pyrrolo[2,3-d]-pyrimidin-6-on

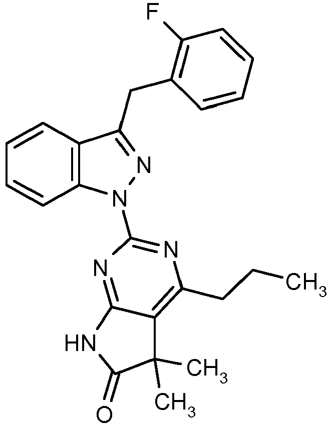

Analog der Vorschrift von Beispiel 27 wurden 200 mg (0.39 mmol) der Verbindung aus Beispiel 3 mit 3.12 ml (1.56 mmol) einer 0.5 M Lösung von Propylzinkbromid in THF umgesetzt. Reinigung über präparative HPLC (Gradient 0.1% Ameisensäure in Wasser / 35-95% Acetonitril). Ausb.: 51 mg (31 % d.Th.)
LC-MS (Methode 1): Rₜ = 1.36 min; MS (ESIpos): m/z = 430 (M+H)⁺
¹H-NMR (400MHz, DMSO-d₆): δ [ppm] = 1.02 (t, 3H), 1.32 - 1.46 (m, 6H), 1.87 (sxt, 2H), 2.77 (t, 2H), 4.42 (s, 2H), 7.10 - 7.24 (m, 2H), 7.26 - 7.33 (m, 2H), 7.38 (t, 1H), 7.58 (t, 1H), 7.74 (d, 1H), 8.70 (d, 1H), 11.70 (br. s, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert.

Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1 %.

Repräsentative IC₅₀-Werte für die erfindungsgemäßen Verbindungen sowie der Referenzverbindung 26 sind in der nachstehenden Tabelle (Tabelle 1) wiedergegeben:

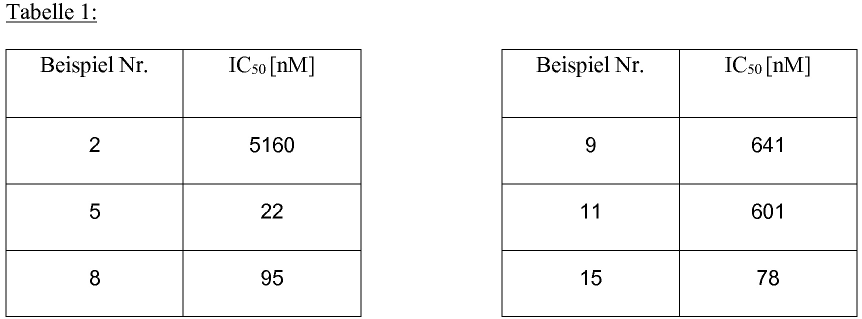

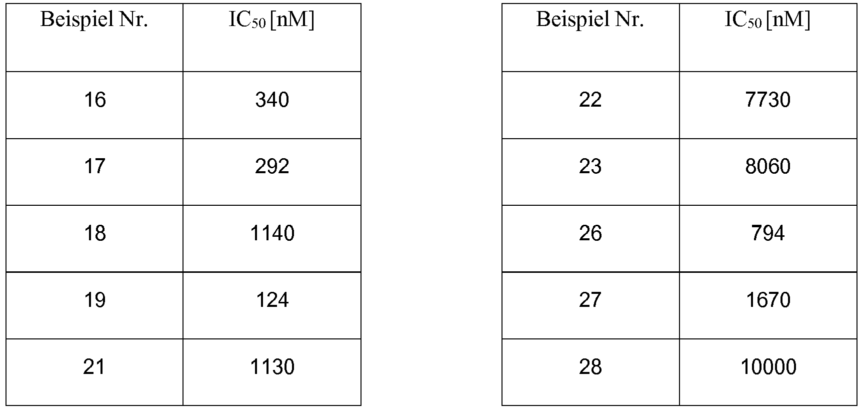

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen und die Referenzverbindungen 24-26 sind in der nachstehenden Tabelle (Tabelle 2) wiedergegeben:

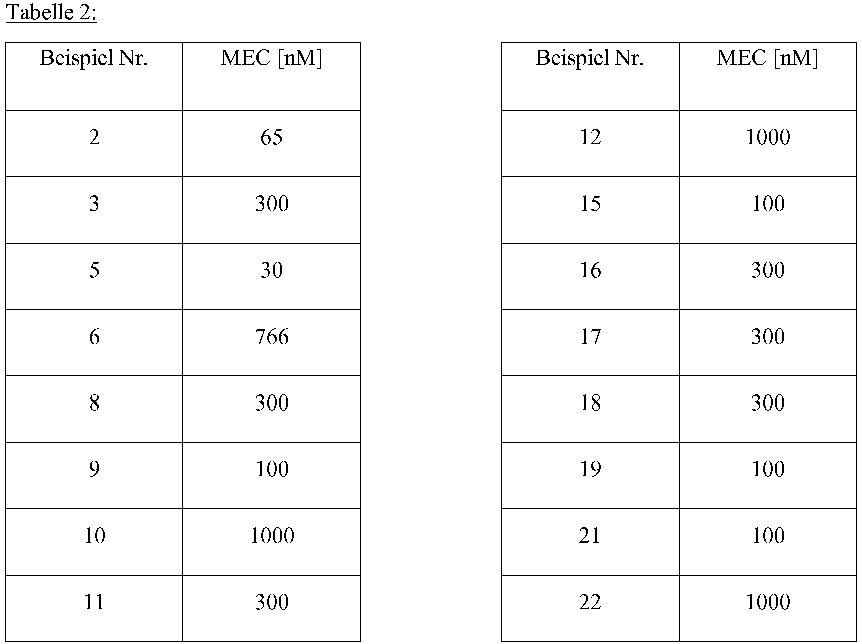

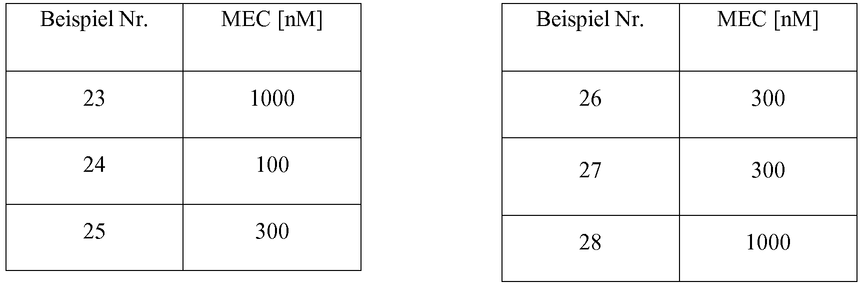

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### S enderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder - flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-6. Inhibition der humanen Phosphodiesterase 5 (PDE 5)

PDE 5-Präparationen werden aus humanen Plättchen durch Aufschluss (Microfluidizer®, 800 bar, 3 Passagen), gefolgt von Zentrifugation (75000 g, 60 min, 4°C) und Ionenaustauscher-Chromatographie des Überstandes auf einer Mono Q 10/10 Säule (linearer Natriumchlorid-Gradient, Elution mit einer 0.2-0.3M Lösung von Natriumchlorid in Puffer (20 mM Hepes pH 7.2, 2 mM - 136 -

Magnesiumchlorid) gewonnen. Fraktionen, die PDE 5 Aktivität aufweisen, werden vereinigt (PDE 5-Präparat) und bei -80°C gelagert.

Die Testsubstanzen werden zur Bestimmung ihrer in vitro Wirkung an humaner PDE 5 in 100% DMSO aufgelöst und seriell verdünnt. Typischerweise werden Verdünnungsreihen (1:3) von 200 µM bis 0.091 µM hergestellt (resultierende Endkonzentrationen im Test: 4 µM bis 0.0018 µM). Jeweils 2 µL der verdünnten Substanzlösungen werden in die Vertiefungen von Mikrotiterplatten (Isoplate-96 /200W; Perkin Elmer) vorgelegt. Anschließend werden 50 µL einer Verdünnung des oben beschriebenen PDE 5-Präparats hinzugefügt. Die Verdünnung des PDE 5 Präparats wird so gewählt, dass während der späteren Inkubation weniger als 70% des Substrates umgesetzt wird (typische Verdünnung: 1: 100; Verdünnungspuffer: 50 mM Tris/Salzsäure pH 7.5, 8.3 mM Magnesiumchlorid, 1.7 mM EDTA, 0.2% BSA). Das Substrat [8-³H] cyclisches Guanosin-3',5'-monophosphat (1 µCi/µL; Perkin Elmer) wird 1:2000 mit Assaypuffer (50 mM Tris/Salzsäure pH 7.5, 8.3 mM Magnesiumchlorid, 1.7 mM EDTA) auf eine Konzentration von 0.0005µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µL einer Suspension von 18 mg/mL Yttrium Scintillation Proximity Beads in Wasser (Phosphodiesterase beads für SPA Assays, RPNQ 0150, Perkin Elmer) gestoppt. Die Mikrotiterplatten werden mit einer Folie versiegelt und für 60 min bei Raumtemperatur stehengelassen. Anschließend werden die Platten für 30 s pro Vertiefung in einem Microbeta Szintillationszähler (Perkin Elmer) vermessen. IC₅₀-Werte werden anhand der graphischen Auftragung der Substanzkonzentration gegen die prozentuale PDE 5-Inhibition bestimmt.

Repräsentative IC₅₀-Werte für die erfindungsgemäßen Verbindungen und die Referenzverbindungen 24-26 sind in der nachstehenden Tabelle (Tabelle 3) wiedergegeben:

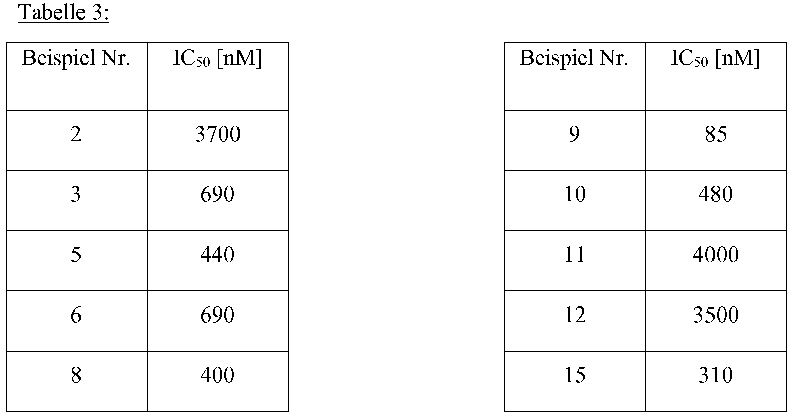

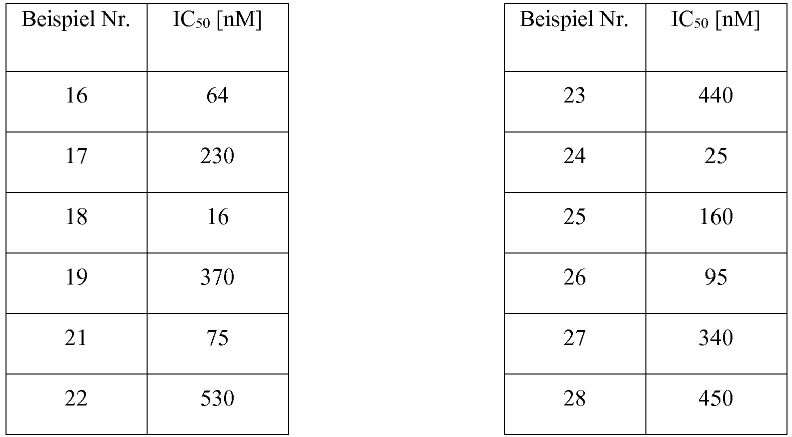

### B-7. Bestimmung der Organ-protektiven Wirkungen im Langzeitversuch an Ratten.

Die Organ-protektiven Wirkungen der sGC Stimulatoren wurden in einem therapeutisch relevanten "low nitric oxide (NO) / high renin" Hypertoniemodell an der Ratten gezeigt. Die Studie wurde in Anlehnung an die kürzlich erschienene Publikation durchgeführt (Sharkovska Y, Kalk P, Lawrenz B, Godes M, Hoffmann LS, Wellkisch K, Geschka S, Relle K, Hocher B, Stasch JP. NO-independent stimulation of soluble guanylate cyclase reduces target organ damage in low- and high-renin models of hypertension. J. Hypertension. 2010; 28: 1666-1675). Dabei wurden Renin-transgene Ratten (TGR(mRen2)27), denen der NO-Synthase-Inhibitor L-NAME über das Trinkwasser verabreicht wurde, gleichzeitig mit einem sGC Stimulator oder Vehikel über mehrere Wochen behandelt. Haemodynamische und renale Parameter wurden während des Behandlungszeitraums bestimmt. Am Ende der Langzeitstudie wurde die Organprotektion (Niere, Lunge, Herz, Aorta) durch histopathologische Untersuchungen, Biomarker, Expressionsanalysen und kardiovaskuläre Plasmaparameter gezeigt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 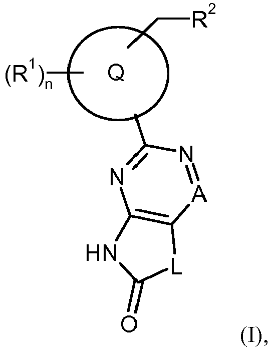 in welcher
A für Stickstoff oder CR³ steht,
wobei
R³ für Wasserstoff, Fluor, Difluormethyl, Trifluormethyl, Methyl, Ethyl, Cyclopropyl oder Cyclobutyl steht,
L für eine Gruppe #-CR^{4A}R^{4B}-(CR^{5A}R^{5B})ₘ-## steht,
wobei
# für die Anknüpfstelle an die Carbonylgruppe steht,
## für die Anknüpfstelle an den Pyrimidin- bzw. Triazinring steht,
m für eine Zahl 0 steht,
R^{4A} für Wasserstoff, Fluor, Methyl, Ethyl, Hydroxy oder Amino steht,
R^{4B} für Wasserstoff, Fluor, Difluormethyl, Trifluormethyl, Methyl, Ethyl, Cyclopropyl, Cyclobutyl, Cyclopentyl oder eine Gruppe der Formel -M-R⁶ steht,
worin Methyl und Ethyl mit 1 bis 3 Substituenten unabhängig voneinander ausgewählt aus der Gruppe Fluor, Cyano, Trifluormethyl, Cyclopropyl, Cyclobutyl, Difluormethoxy und Trifluormethoxy substituiert sein kann,
wobei R^{4B} für Wasserstoff, Difluormethyl, Trifluormethyl, Methyl, Ethyl, Cyclopropyl, Cyclobutyl, Cyclopentyl oder eine Gruppe der Formel -M-R⁶ wenn R^{4A} für Hydroxy steht,
und worin
M für eine Bindung steht,
R⁶ für -(C=O)ᵣ-NR⁷R⁸, Phenyl, Thiazolyl, Triazolyl, Oxadiazolyl, Thiadiazolyl oder Pyrimidinyl steht,
worin
r die Zahl 1 bedeutet,
R⁷ und R⁸ unabhängig voneinander jeweils für Wasserstoff, oder Cyclopropyl stehen,
und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmethyl und Cyclobutylmethyl substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
der Ring Q für eine Gruppe der Formel 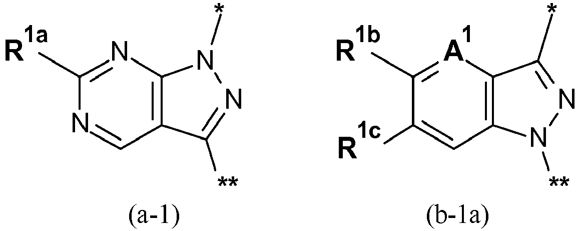 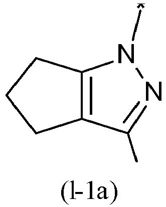 steht, wobei
* für die Anknüpfungsstelle an -CH₂-R² steht,
** für die Anknüpfungsstelle an den Pyrimidin- bzw. Triazinring steht,
A¹ für N oder CH steht,
R¹ für Halogen, Cyano, Difluormethyl, Trifluormethyl, (C₁-C₄)Alkyl, Hydroxy, Oxo oder (C₁-C₄)Alkoxy steht,
n für eine Zahl 0 steht,
R^{1a} für Wasserstoff oder Methyl steht,
R^{1b} für Wasserstoff, Fluor oder Chlor steht, wenn A¹ für CH steht,
R^{1b} für Wasserstoff steht, wenn A¹ für N steht,
R^{1c} für Wasserstoff oder Fluor steht,
R² für 3,3,3-Trifluorprop-1-yl, 2,2,3,3-Tetrafluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

2. Verbindung der Formel (I) nach Anspruch 1, in welcher
A für Stickstoff oder CR³ steht,
wobei
R³ für Wasserstoff steht,
L für eine Gruppe #-CR^{4A}R^{4B}-(CR^{5A}R^{5B})ₘ-## steht,
wobei
# für die Anknüpfstelle an die Carbonylgruppe steht,
## für die Anknüpfstelle an den Pyrimidin- bzw. Triazinring steht,
m für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
wobei R^{4B} für Wasserstoff, Trifluormethyl, 2,2,2-Trifluorethyl oder Methyl steht, wenn R^{4A} für Hydroxy steht,
der Ring Q für eine Gruppe der Formel 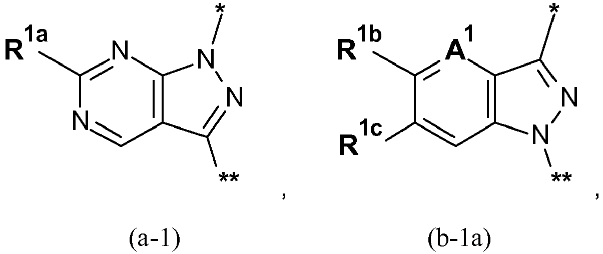 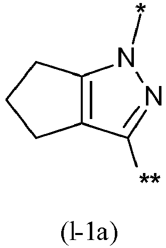 steht, wobei
* für die Anknüpfungsstelle an -CH₂-R² steht,
** für die Anknüpfungsstelle an den Pyrimidin- bzw. Triazinring steht,
A¹ für N oder CH steht,
R^{1a} für Wasserstoff oder Methyl steht,
R^{1b} für Wasserstoff, Fluor oder Chlor steht, wenn A¹ für CH steht,
R^{1b} für Wasserstoff steht, wenn A¹ für N steht,
R^{1c} für Wasserstoff oder Fluor steht,
R² für 3,3,3-Trifluorprop-1-yl, 2,2,3,3,3-Pentafluorprop-1-yl, Phenyl oder Pyridyl
steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

3. Verbindung der Formel (I) nach Anspruch 1 oder 2, in welcher
A für Stickstoff oder CR³ steht,
wobei
R³ für Wasserstoff steht,
L für eine Gruppe #-CR^{4A}R^{4B}-(CR^{5A}R5^{B})ₘ-## steht,
wobei
# für die Anknüpfstelle an die Carbonylgruppe steht,
## für die Anknüpfstelle an den Pyrimidin- bzw. Triazinring steht,
m für eine Zahl 0 steht,
R^{4A} für Methyl steht,
R^{4B} für Methyl steht,
der Ring Q für eine Gruppe der Formel 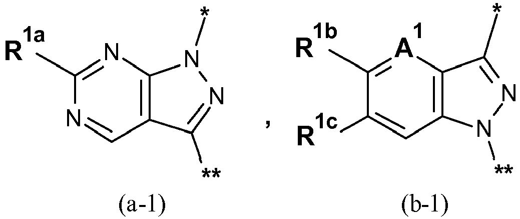 steht, wobei
* für die Anknüpfungsstelle an -CH₂-R² steht,
** für die Anknüpfungsstelle an den Pyrimidin- bzw. Triazinring steht,
A¹ für N oder CH steht,
R^{1a} für Wasserstoff oder Methyl steht,
R^{1b} für Wasserstoff, Fluor oder Chlor steht, wenn A¹ für CH steht,
R^{1b} für Wasserstoff steht, wenn A¹ für N steht,
R^{1c} für Wasserstoff oder Fluor steht,
R² für 2,2,3,3,3-Pentafluorprop-1-yl, Phenyl oder Pyridyl steht,
wobei Phenyl mit 1 oder 2 Substituenten Fluor substituiert ist, und
wobei Pyridyl mit 1 Substituenten Fluor substituiert sein kann,
sowie ihre Salze, Solvate und Solvate der Salze.

4. Verbindung der Formel (I) nach Anspruch 1, 2 oder 3, in welcher
A für Stickstoff steht,
L für eine Gruppe #-CR^{4A}R^{4B}-(CR^{5A}R^{5B})ₘ-## steht,
wobei
# für die Anknüpfstelle an die Carbonylgruppe steht,
## für die Anknüpfstelle an den Triazinring steht,
m für eine Zahl 0 steht,
R^{4A} für Methyl steht,
R^{4B} für Methyl steht,
der Ring Q für eine Gruppe der Formel 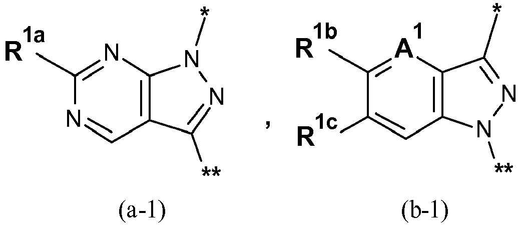 steht, wobei
* für die Anknüpfungsstelle an -CH₂-R² steht,
** für die Anknüpfungsstelle an den Triazinring steht,
A¹ für N oder CH steht,
R^{1a} für Wasserstoff oder Methyl steht,
R^{1b} für Wasserstoff, Fluor oder Chlor steht, wenn A¹ für CH steht,
R^{1b} für Wasserstoff steht, wenn A¹ für N steht,
R^{1c} für Wasserstoff oder Fluor steht,
R² für Phenyl steht,
wobei Phenyl mit 1 oder 2 Substituenten Fluor substituiert ist,
sowie ihre Salze, Solvate und Solvate der Salze.

5. Verbindungen nach Anspruch 1, die ausgewählt sind aus 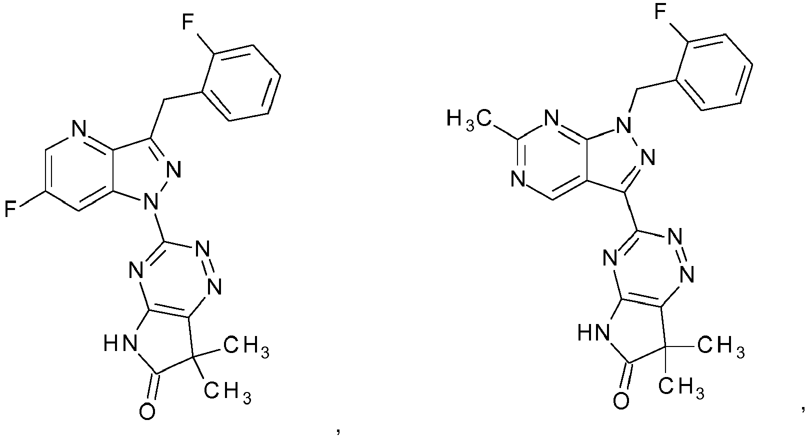 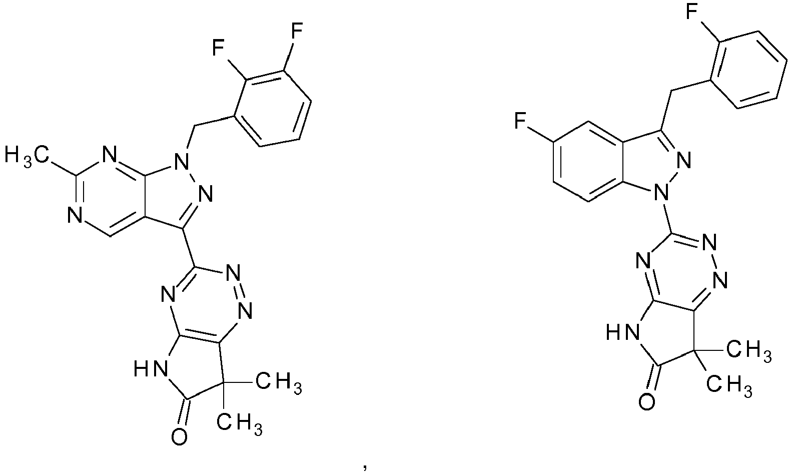 sowie ihre Salze, Solvate und Solvate der Salze.

6. Verfahren zur Herstellung von Verbindungen der Formel (I), wie in den Ansprüchen 1 bis 5 definiert, **dadurch gekennzeichnet, dass** man
[A] eine Verbindung der Formel (II) 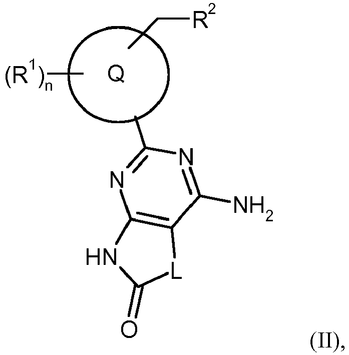 in welcher n, L, Q, R¹ und R² jeweils die in den Ansprüchen 1 bis 5 angegebenen Bedeutungen haben,
umsetzt, diese dann mit iso-Pentylnitrit und einem Halogen-Äquivalent in eine Verbindung der Formel (I-A) 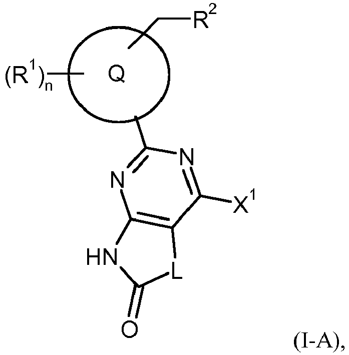 in welcher n, L, Q, R¹ und R² jeweils die in den Ansprüchen 1 bis 5 angegebenen Bedeutungen haben und
X¹ für Brom oder Iod steht,
überführt, oder
[B] eine Verbindung der Formel (I-A) in einen inerten Lösungsmittel in Gegenwart eines geeigneten Übergangsmetallkatalysators zu einer Verbindung der Formel (I-B) 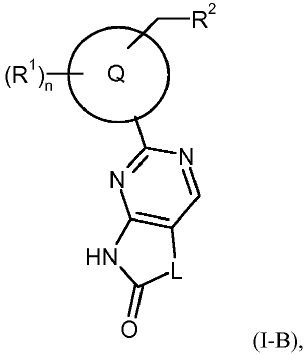 in welcher n, L, Q, R¹ und R² jeweils die in den Ansprüchen 1 bis 5 angegebenen Bedeutungen haben,
umsetzt,
oder
[C] eine Verbindung der Formel (I-A) in einem inerten Lösungsmittel in Gegenwart eines geeigneten Übergangsmetallkatalysators mit einer Verbindung der Formel (III-A), (III-B) bzw. (III-C) 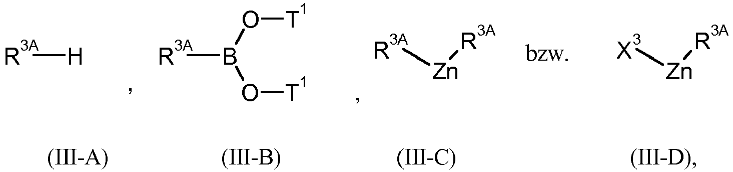 in welchen
R^{3A} für Halogen, Difluormethyl, Trifluormethyl, (C₁-C₄)-Alkyl, (C₂-C₄)-Alkenyl, (C₂-C₄)-Alkinyl, Cyclopropyl, Cyclobutyl, Phenyl oder 5- oder 6-gliedriges Heteroaryl steht,
worin (C₁-C₄)-Alkyl, (C₂-C₄)-Alkenyl, (C₂-C₄)-Alkinyl, Phenyl und 5- oder 6-gliedriges Heteroaryl mit 1 bis 3 Substituenten unabhängig voneinander ausgewählt aus der Gruppe mit Fluor, Difluormethyl, Trifluormethyl, (C₁-C₄)-Alkyl, Difluormethoxy, Trifluormethoxy, (C₁-C₄)-Alkoxy, (C₁-C₄)-Alkoxycarbonyl, Cyclopropyl und Cyclobutyl substituiert sein kann,
T¹ für Wasserstoff oder (C₁-C₄)-Alkyl steht, oder beide Reste R¹¹ zusammen eine -C(CH₃)₂-C(CH₃)₂-Brücke bilden,
und
X³ für Brom oder Iod steht,
zu einer Verbindung der Formel (I-C) 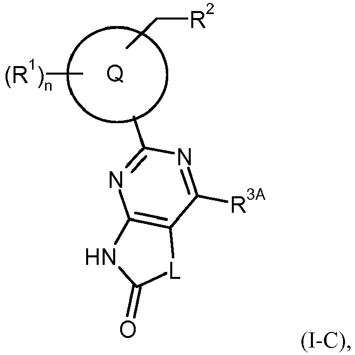 in welcher n, L, Q, R¹, R² und R^{3A} jeweils die zuvor angegebenen Bedeutungen haben,
umsetzt,
oder
[D] in einem inerten Lösungsmittel in Gegenwart einer geeigneten Base mit Hydrazinhydrat zu einer Verbindung der Formel (IV) 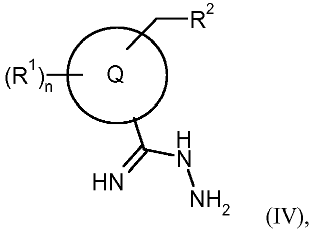 in welcher n, L, Q, R¹ und R² jeweils die in den Ansprüchen 1 bis 5 genannten Bedeutungen haben,
umsetzt, diese dann in einem inerten Lösungsmittel mit einer Verbindung der Formel (V) 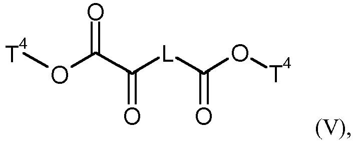 in welcher L die in den Ansprüchen 1 bis 5 angegebene Bedeutung hat und
T⁴ für (C₁-C₄)-Alkyl steht,
zu einer Verbindung der Formel (VI) 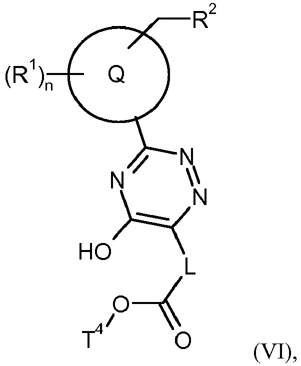 in welcher n, L, Q, R¹, R² und T⁴ jeweils die zuvor angegebenen Bedeutungen haben, reagiert, diese anschliessend mit Phosphorylchlorid in eine Verbindung der Formel (VII) 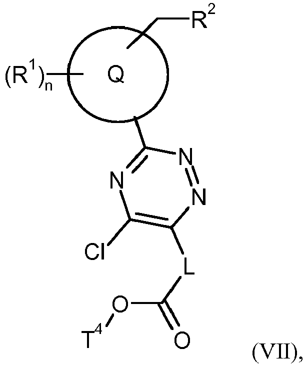 in welcher n, L, Q, R¹, R² und T⁴ jeweils die zuvor angegebenen Bedeutungen haben, überführt, und diese direkt mit Ammoniak zu einer Verbindung der Formel (VIII) 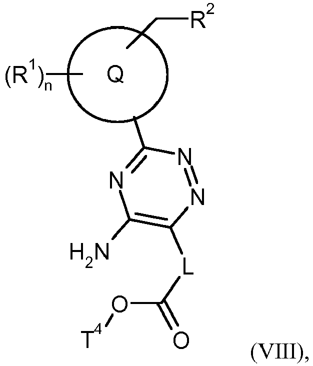 in welcher n, L, Q, R¹, R² und T⁴ jeweils die zuvor angegebenen Bedeutungen haben, umsetzt, und schliesslich in einem inerten Lösungsmittel gegebenenfalls in Gegenwart einer geeigneten Base zu einer Verbindung der Formel (I-D) 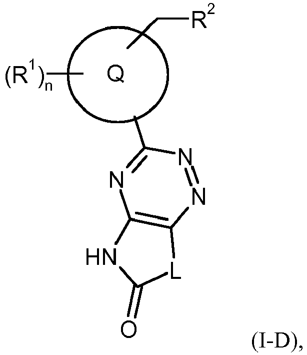 in welcher n, L, Q, R¹ und R² jeweils die in den Ansprüchen 1 bis 5 angegebenen Bedeutungen haben,
cyclisiert,
oder
[E] eine Verbindung der Formel (X) 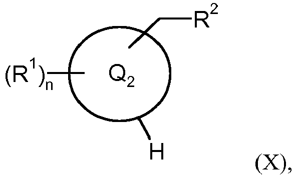 in welcher n, R¹ und R² jeweils die in den Ansprüchen 1 bis 5 angegebenen Bedeutungen haben und
der Ring Q² für eine Gruppe der Formel 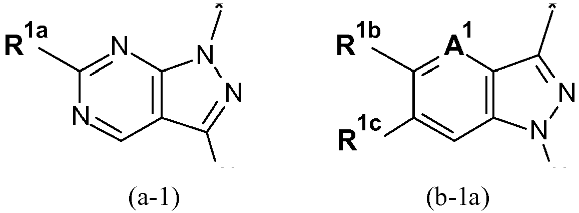 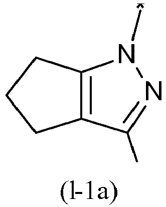 steht, wobei
* für die Anknüpfungsstelle an -CH₂-R² steht,
** für die Anknüpfungsstelle das Wasserstoffatom steht, wobei R^{1a}, R^{1b}, R^{1c} und A1 die in den Ansprüchen 1 bis 5 angegebenen Bedeutungen haben,
in einem inerten Lösungsmittel gegebenenfalls in Gegenwart einer geeigneten Base mit einer Verbindung der Formel (XI) 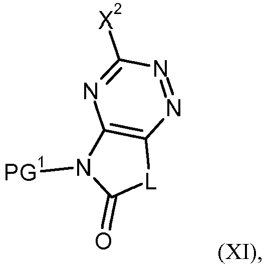 in welcher L die in den Ansprüchen 1 bis 5 angegebene Bedeutung hat,
X² für Chlor oder Brom steht und
PG¹ für eine geeignete Aminoschutzgruppe, insbesondere p-Methoxybenzyl steht,
zu einer Verbindung der Formel (XII) 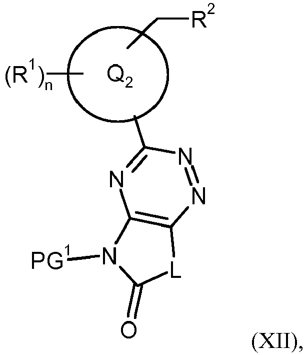 in welcher n, L, Q₂, R¹, R² und PG¹ jeweils die zuvor angegebenen Bedeutungen haben,
umsetzt, von dieser anschliessend die Schutzgruppe PG¹ zu einer Verbindung der Formel (I-E) 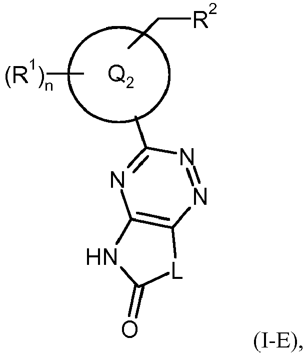 in welcher n, L, Q₂, R¹ und R² jeweils die zuvor angegebenen Bedeutungen haben, abspaltet
und gegebenenfalls die resultierenden Verbindungen der Formeln (I-A), (I-B), (I-C), (I-D) und (I-E) gegebenenfalls mit den entsprechenden (i) Lösungsmitteln und/oder (ii) Säuren oder Basen in ihre Solvate, Salze und/oder Solvate der Salze überführt.

7. Verbindung der Formel (I), wie in einem der Ansprüche 1 bis 5 definiert, zur Behandlung und/oder Prophylaxe von Krankheiten.

8. Verwendung einer Verbindung der Formel (I), wie in einem der Ansprüche 1 bis 5 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

9. Arzneimittel enthaltend eine Verbindung der Formel (I), wie in einem der Ansprüche 1 bis 5 definiert, in Kombination mit einem inerten, nicht-toxischen, pharmazeutisch geeigneten Hilfsstoff.

10. Arzneimittel enthaltend eine Verbindung der Formel (I), wie in einem der Ansprüche 1 bis 5 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

11. Arzneimittel nach Anspruch 9 oder 10 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (1) 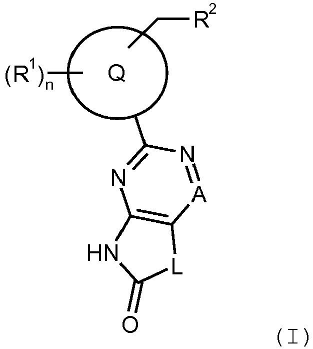 in which
A is nitrogen or CR³
where
R³ is hydrogen, fluorine, difluoromethyl, trifluoromethyl, methyl, ethyl, cyclopropyl or cyclobutyl,
L is a #-CR^{4A}R^{4B}-(CR^{5A}R^{5B})ₘ-## group
where
# is the attachment site to the carbonyl group,
## is the attachment site to the pyrimidine or triazine ring,
m is a number 0,
R^{4A} is hydrogen, fluorine, methyl, ethyl, hydroxyl or amino,
R^{4B} is hydrogen, fluorine, difluoromethyl, trifluoromethyl, methyl, ethyl, cyclopropyl, cyclobutyl, cyclopentyl or a group of the formula -M-R⁶,
in which methyl and ethyl may each be substituted by 1 to 3 substituents selected independently from the group of fluorine, cyano, trifluoromethyl, cyclopropyl, cyclobutyl, difluoromethoxy and trifluoromethoxy,
where R^{4B} is hydrogen, difluoromethyl, trifluoromethyl, methyl, ethyl, cyclopropyl, cyclobutyl, cyclopentyl or a group of the formula -M-R⁶ when R^{4A} is hydroxyl,
and in which
M is a bond,
R⁶ is -(C=O)ᵣ-NR⁷R⁸, phenyl, thiazolyl, triazolyl, oxadiazolyl, thiadiazolyl or pyrimidinyl,
in which
r is the number 1,
R⁷ and R⁸ are each independently hydrogen, or cyclopropyl,
and
in which phenyl, thiazolyl, triazolyl, oxadiazolyl, thiadiazolyl and pyrimidinyl may each in turn be substituted by 1 or 2 substituents selected independently from the group of fluorine, difluoromethyl, trifluoromethyl, methyl, ethyl, isopropyl, 2,2,2-trifluoroethyl, 1,1,2,2,2-pentafluoroethyl, cyclopropyl, cyclobutyl, cyclopropylmethyl and cyclobutylmethyl,
or
R^{4A} and R^{4B} together with the carbon atom to which they are bonded form a cyclopropyl, cyclobutyl, cyclopentyl, azetidinyl, tetrahydrofuranyl, pyrrolidinyl or tetrahydropyranyl ring,
in which the cyclopropyl, cyclobutyl, cyclopentyl, azetidinyl, tetrahydrofuranyl, pyrrolidinyl and tetrahydropyranyl ring may each be substituted by 1 or 2 substituents selected independently from the group of fluorine and methyl, the ring Q is a group of the formula 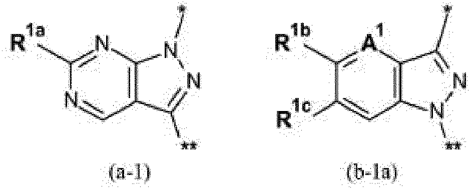 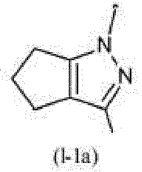
where
* is the attachment site to -CH₂-R²,
** is the attachment site to the pyrimidine or triazine ring,
A¹ is N or CH,
R¹ is halogen, cyano, difluoromethyl, trifluoromethyl, (C₁-C₄) -alkyl, hydroxyl, oxo or (C₁-C₄) - alkoxy,
N is a number 0,
R^{1a} is hydrogen or methyl,
R^{1b} is hydrogen, fluorine or chlorine when A¹ is CH, R^{1b} is hydrogen when A¹ is N,
R^{1c} is hydrogen or fluorine,
R² is 3,3,3-trifluoroprop-1-yl, 2,2,3,3-tetrafluoroprop-1-yl, 2,2,3,3,3-pentafluoroprop-1-yl, phenyl or pyridyl,
where phenyl is substituted by 1 to 3 fluorine substituents,
and
where pyridyl may be substituted by 1 fluorine substituent,
and the salts, solvates and solvates of the salts thereof.

2. Compound of the formula (I) according to Claim 1, in which
A is nitrogen or CR³
where
R³ is hydrogen,
L is a #-CR^{4A}R^{4B}-(CR^{5A}R^{5B})ₘ-## group where
# is the attachment site to the carbonyl group,
## is the attachment site to the pyrimidine or triazine ring,
m is a number 0,
R^{4A} is hydrogen, fluorine, methyl or hydroxyl,
R^{4B} is hydrogen, fluorine, trifluoromethyl, 2,2,2-trifluoroethyl or methyl,
where R^{4B} is hydrogen, trifluoromethyl, 2,2,2-trifluoroethyl or methyl when R^{4A} is hydroxyl,
the ring Q is a group of the formula 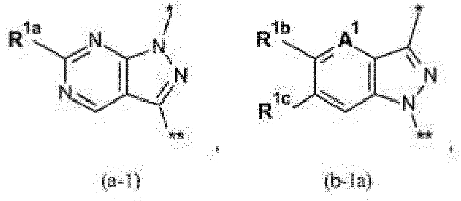 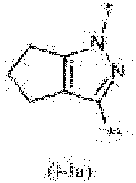
where
* is the attachment site to -CH₂-R²,
** is the attachment site to the pyrimidine or triazine ring,
A¹ is N or CH,
R^{1a} is hydrogen or methyl,
R^{1b} is hydrogen, fluorine or chlorine when A¹ is CH,
R^{1b} is hydrogen when A¹ is N,
R^{1c} is hydrogen or fluorine,
R² is 3,3,3-trifluoroprop-1-yl, 2,2,3,3,3-pentafluoroprop-1-yl, phenyl or pyridyl,
where phenyl is substituted by 1 to 3 fluorine substituents,
and
where pyridyl may be substituted by 1 fluorine substituent,
and the salts, solvates and solvates of the salts thereof.

3. Compound of the formula (I) according to Claim 1 or 2, in which
A is nitrogen or CR³
where
R³ is hydrogen,
L is a #-CR^{4A}R^{4B}-(CR^{5A}R^{5B})ₘ-## group where
# is the attachment site to the carbonyl group,
## is the attachment site to the pyrimidine or triazine ring,
m is a number 0,
R^{4A} is methyl,
R^{4B} is methyl,
the ring Q is a group of the formula 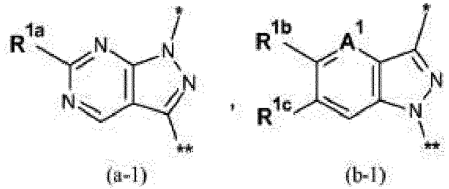 where
* is the attachment site to -CH₂-R²,
** is the attachment site to the pyrimidine or triazine ring,
A¹ is N or CH,
R^{1a} is hydrogen or methyl,
R^{1b} is hydrogen, fluorine or chlorine when A¹ is CH,
R^{1b} is hydrogen when A¹ is N,
R^{1c} is hydrogen or fluorine,
R² is 2,2,3,3,3-pentafluoroprop-1-yl, phenyl or pyridyl,
where phenyl is substituted by 1 or 2 fluorine substituents,
and where pyridyl may be substituted by 1 fluorine substituent, and the salts, solvates and solvates of the salts thereof.

4. Compound of the formula (I) according to Claim 1, 2 or 3, in which
A is nitrogen,
L is a #-CR^{4A}R^{4B}-(CR^{5A}R^{5B})ₘ-## group where
# is the attachment site to the carbonyl group,
## is the attachment site to the triazine ring,
m is a number 0,
R^{4A} is methyl,
R^{4B} is methyl,
the ring Q is a group of the formula 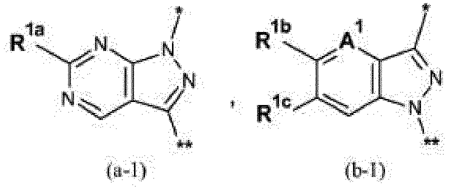 where
* is the attachment site to -CH₂-R²,
** is the attachment site to the triazine ring,
A¹ is N or CH,
R^{1a} is hydrogen or methyl,
R^{1b} is hydrogen, fluorine or chlorine when A¹ is CH,
R^{1b} is hydrogen when A¹ is N,
R^{1c} is hydrogen or fluorine,
R² is phenyl,
where phenyl is substituted by 1 or 2 fluorine substituents, and the salts, solvates and solvates of the salts thereof.

5. Compounds according to Claim 1 that are selected from 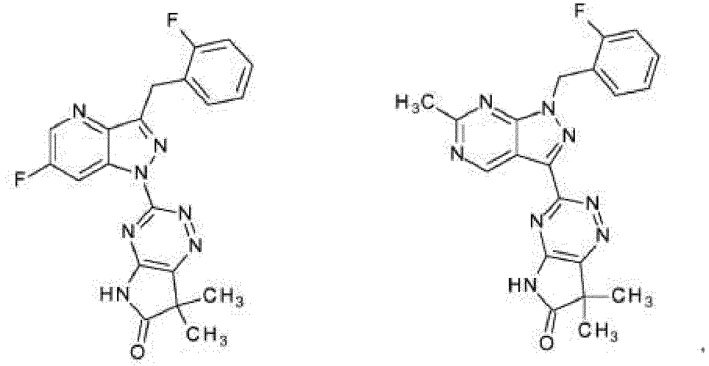 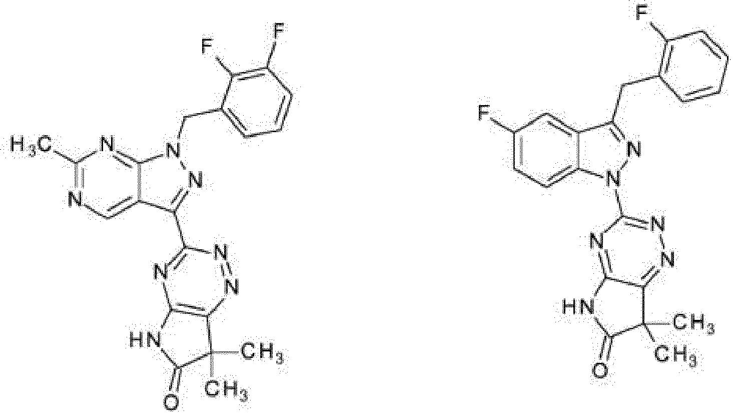 and the salts, solvates and solvates of the salts thereof.

6. Process for preparing compounds of the formula (I) as defined in Claims 1 to 5, **characterized in that**
[A] a compound of the formula (II) 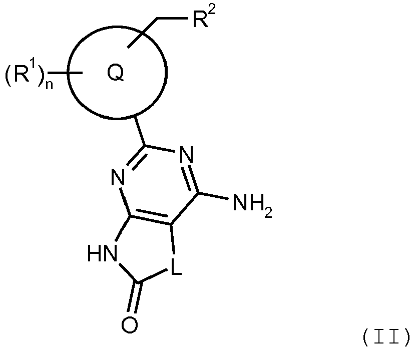
in which n, L, Q, R¹ and R² are each as defined in Claims 1 to 5
is reacted, then this is converted using isopentyl nitrite and a halogen equivalent to a compound of the formula (I-A) 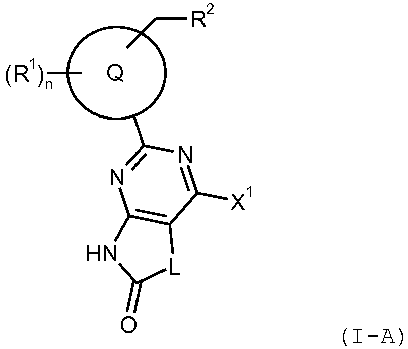
in which n, L, Q, R¹ and R² are each as defined in Claims 1 to 5 and
X¹ is bromine or iodine,
or
[B] a compound of the formula (I-A) is reacted in an inert solvent in the presence of a suitable transition metal catalyst to give a compound of the formula (I-B) 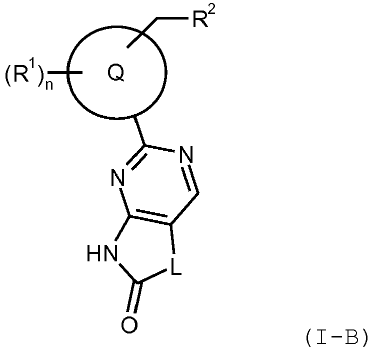 in which n, L, Q, R¹ and R² are each as defined in Claims 1 to 5, or
[C] a compound of the formula (I-A) is reacted in an inert solvent in the presence of a suitable transition metal catalyst with a compound of the formula (III-A), (III-B) or (III-C) 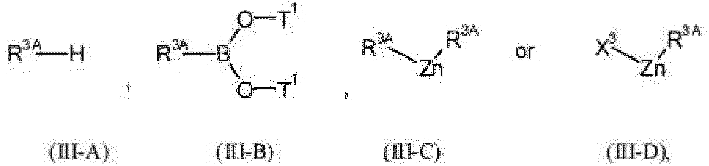 in which
R^{3A} is halogen, difluoromethyl, trifluoromethyl, (C₁-C₄) -alkyl, (C₂-C₄) -alkenyl, (C₂-C₄) -alkynyl, cyclopropyl, cyclobutyl, phenyl or 5- or 6-membered heteroaryl, in which (C₁-C₄) -alkyl, (C₂-C₄) -alkenyl, (C₂-C₄) - alkynyl, phenyl and 5- or 6-membered heteroaryl may each be substituted by 1 to 3 substituents selected independently from the group comprising fluorine, difluoromethyl, trifluoromethyl, (C₁-C₄)-alkyl, difluoromethoxy, trifluoromethoxy, (C₁-C₄)-alkoxy, (C₁-C₄)-alkoxycarbonyl, cyclopropyl and cyclobutyl,
T¹ is hydrogen or (C₁-C₄)-alkyl, or both R¹¹ radicals together form a -C(CH₃)₂-C(CH₃)₂- bridge,
and
X³ is bromine or iodine,
to give a compound of the formula (I-C) 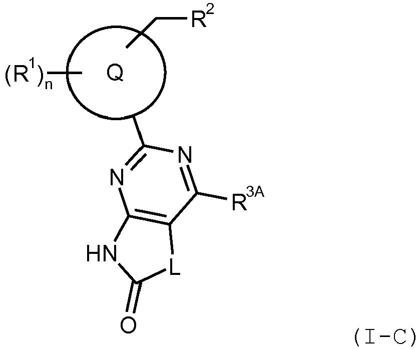
in which n, L, Q, R¹, R² and R^{3A} are each as defined above,
or
[D] is reacted in an inert solvent in the presence of a suitable base with hydrazine hydrate to give a compound of the formula (IV) 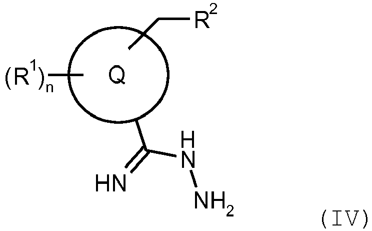
in which n, L, Q, R¹ and R² are each as defined in Claims 1 to 5,
this is then reacted in an inert solvent with a compound of the formula (V) 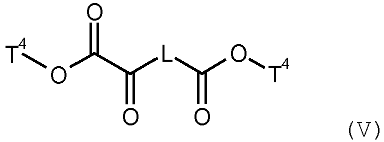
in which L is as defined in Claims 1 to 5 and T⁴ is (C₁-C₄)-alkyl
to give a compound of the formula (VI) 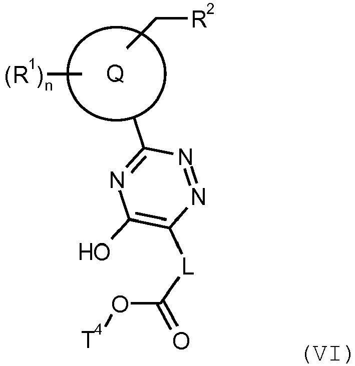
in which n, L, Q, R¹, R² and T⁴ are each as defined above,
then this is converted using phosphoryl chloride to a compound of the formula (VII) 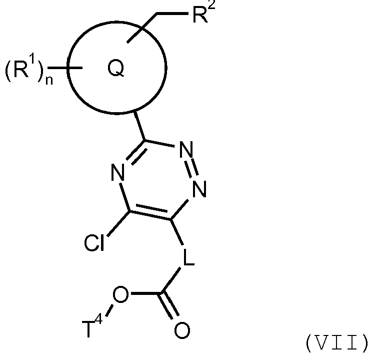
in which n, L, Q, R¹, R² and T⁴ are each as defined above,
and this is reacted directly with ammonia to give a compound of the formula (VIII) 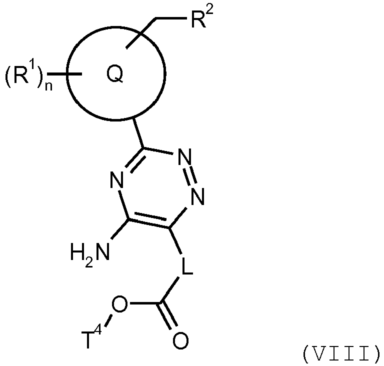
in which n, L, Q, R¹, R² and T⁴ are each as defined above,
and finally cyclized in an inert solvent, optionally in the presence of a suitable base, to give a compound of the formula (I-D) 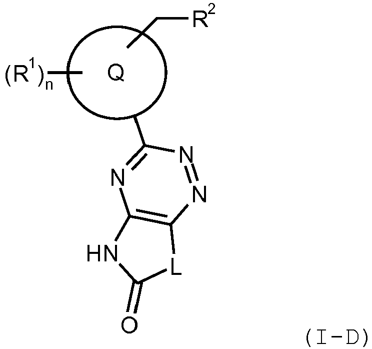
in which n, L, Q, R¹ and R² are each as defined in Claims 1 to 5,
or
[E] a compound of the formula (X) 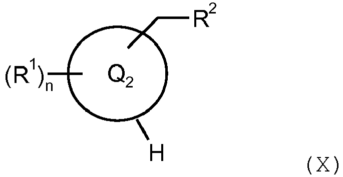
in which n, R¹ and R² are each as defined in Claims 1 to 5 and
the ring Q² is a group of the formula 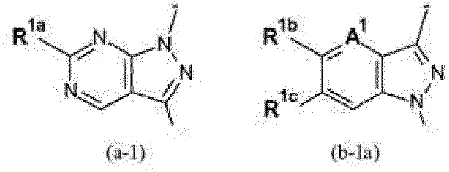 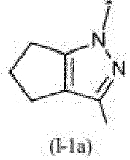
where
* is the attachment site to -CH₂-R²,
** is the attachment site to the hydrogen atom,
where R^{1a}, R^{1b}, R^{1c} and A1 are as defined in Claims 1 to 5,
is converted in an inert solvent, optionally in the presence of a suitable base, with a compound of the formula (XI) 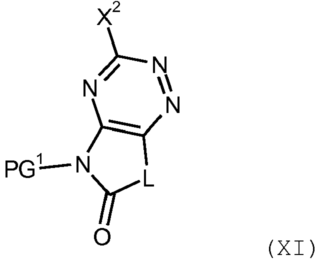
in which L is as defined in Claims 1 to 5,
X² is chlorine or bromine and
PG¹ is a suitable amino protecting group, especially p-methoxybenzyl,
to give a compound of the formula (XII) 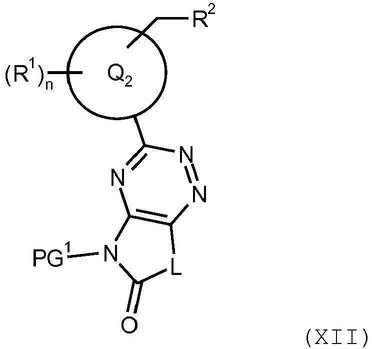
in which n, L, Q₂, R¹, R² and PG¹ are each as defined above,
the protecting group PG¹ is subsequently detached therefrom to give a compound of the formula (I-E) 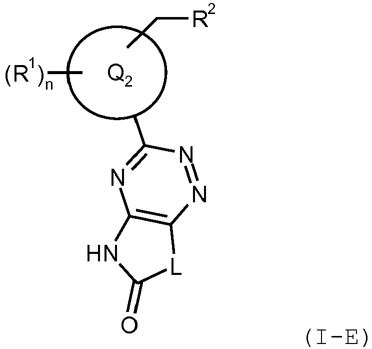
in which n, L, Q₂, R¹ and R² are each as defined above, and, if appropriate, the resulting compounds of the formulae (I-A), (I-B), (I-C), (I-D) and (I-E) are optionally converted with the appropriate (i) solvents and/or (ii) acids or bases to the solvates, salts and/or solvates of the salts thereof.

7. Compound of the formula (I) as defined in any of Claims 1 to 5 for treatment and/or prophylaxis of diseases.

8. Use of a compound of the formula (I) as defined in any of Claims 1 to 5 for production of a medicament for treatment and/or prophylaxis of heart failure, angina pectoris, hypertension, pulmonary hypertension, ischaemia, vascular disorders, kidney failure, thromboembolic disorders, fibrotic disorders and arteriosclerosis.

9. Medicament comprising a compound of the formula (I) as defined in any of Claims 1 to 5 in combination with an inert, nontoxic, pharmaceutically suitable excipient.

10. Medicament comprising a compound of the formula (I) as defined in any of Claims 1 to 5 in combination with a further active ingredient selected from the group consisting of organic nitrates, NO donors, cGMP-PDE inhibitors, antithrombotic agents, hypotensive agents and lipid metabolism modifiers.

11. Medicament according to Claim 9 or 10 for treatment and/or prophylaxis of heart failure, angina pectoris, hypertension, pulmonary hypertension, ischaemia, vascular disorders, kidney failure, thromboembolic disorders, fibrotic disorders and arteriosclerosis.

## Revendications

1. Compose de formule (I) 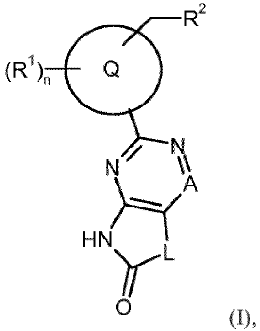 dans lequel
A represente azote ou CR³,
R³ représentant hydrogene, fluor, difluoromethyle, trifluoromethyle, methyle, ethyle, cyclopropyle ou cyclobutyle,
L represente un groupe #-CR^{4A}R^{4B}-(CR^{5A}R^{5B})ₘ-##,
#
carbonyle,
## pyrimidine ou triazine,
m représentant un nombre 0,
R^{4A} représentant hydrogene, fluor, methyle, ethyle, hydroxy ou amino,
R^{4B} représentant hydrogene, fluor, difluoromethyle, trifluoromethyle, methyle, ethyle, cyclopropyle, cyclobutyle, cyclopentyle ou un groupe de formule M-R⁶,
avec 1 a 3 substituants choisis indépendamment les uns des autres dans le groupe constitue par fluor, cyano, trifluoromethyle, cyclopropyle, cyclobutyle, difluoromethoxy et trifluoromethoxy,
R^{4B} représentant hydrogene, difluoromethyle, trifluoromethyle, methyle, ethyle, cyclopropyle, cyclobutyle, cyclopentyle ou un groupe de formule M-R⁶ lorsque R^{4A} represente hydroxy,
M représentant une liaison,
R⁶ representant (C=O)ᵣ-NR⁷R⁸, phenyle, thiazolyle, triazolyle, oxadiazolyle, thiadiazolyle ou pyrimidinyle,
r signifiant le nombre 1,
R⁷ et R⁸ représentant chacun independamment et
le phenyle, le thiazolyle, le triazolyle, lyle, le thiadiazolyle et le pyrimidinyle pouvant de leur cote etre substitues avec 1 ou 2
dans le groupe constitue par fluor, difluoromethyle, trifluoromethyle, methyle, ethyle, isopropyle, 2,2,2-trifluoroethyle, 1,1,2,2,2-pentafluoroethyle, cyclopropyle, cyclobutyle, cyclopropylmethyle et cyclobutylmethyle,
ou
R^{4A} et R^{4B}
auquel ils sont relies un cycle cyclopropyle, cyclobutyle, cyclopentyle, azetidinyle, tetrahydrofuranyle, pyrrolidinyle ou tetrahydropyranyle,
le cycle cyclopropyle, le cyclobutyle, le drofuranyle, le pyrrolidinyle et le tetrahydropyranyle pouvant etre substitues avec 1 ou 2 substituants choisis par fluor et methyle,
le cycle Q represente un groupe de formule 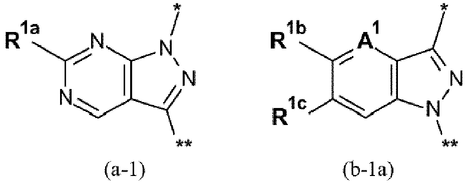 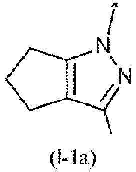
dans lesquelles
* CH₂-R²,
**
pyrimidine ou triazine,
A¹ represente N ou CH,
R¹ represente halogene, cyano, difluoromethyle, trifluoromethyle, alkyle en (C₁-C₄), hydroxy, oxo ou alcoxy en (C₁-C₄),
n represente un nombre 0,
R^{1a} represente hydrogene ou methyle,
R^{1b} 1 represente CH,
R^{1b} represente N,
R^{1c} represente hydrogene ou fluor,
R² represente 3,3,3-trifluoroprop-1-yle, 2,2,3,3-tetrafluroprop-1-yle, 2,2,3,3,3-pentafluoroprop-1-yle, phenyle ou pyridyle,
le phenyle étant substitue avec 1 a 3 substituants fluor,
et
le pyridyle pouvant etre substitue avec 1 substituant fluor,
ainsi que ses sels, solvates et solvates des sels.

2. Compose de formule (I) selon la revendication 1, dans lequel
A represente azote ou CR³,
R³ représentant hydrogene,
L represente un groupe #-CR^{4A}R^{4B}-(CR^{5A}R^{5B})ₘ-##,
#
carbonyle,
## pyrimidine ou triazine,
m représentant un nombre 0,
R^{4A} représentant hydrogene, fluor, methyle ou hydroxy,
R^{4B} représentant hydrogene, fluor, trifluoromethyle, 2,2,2-trifluoroethyle ou methyle,
R^{4B} représentant hydrogene, trifluoromethyle, 2,2,2-trifluoroethyle ou methyle lorsque R^{4A} represente hydroxy,
le cycle Q represente un groupe de formule 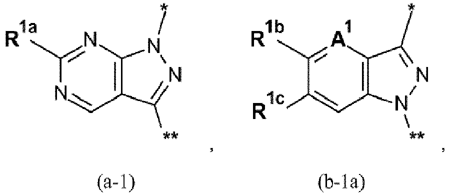 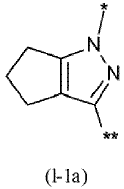 dans lesquelles
* liaison a CH₂-R²,
**
pyrimidine ou triazine,
A¹ represente N ou CH,
R^{1a} represente hydrogene ou methyle,
R^{1b} 1
represente CH,
R^{1b} represente N,
R^{1c} represente hydrogene ou fluor,
R² represente 3,3,3-trifluoroprop-1-yle, 2,2,3,3,3-pentafluoroprop-1-yle, phenyle ou pyridyle,
le phenyle étant substitue avec 1 a 3 substituants fluor,
et
le pyridyle pouvant etre substitue avec 1 substituant fluor,
ainsi que ses sels, solvates et solvates des sels.

3. Compose de formule (I) selon la revendication 1 ou 2, dans lequel
A represente azote ou CR³,
R³ représentant hydrogene,
L represente un groupe #-CR^{4A}R^{4B}-(CR^{5A}R^{5B})ₘ-##,
# representant
carbonyle,
##
pyrimidine ou triazine,
m représentant un nombre 0,
R^{4A} représentant methyle,
R^{4B} representant methyle,
le cycle Q represente un groupe de formule 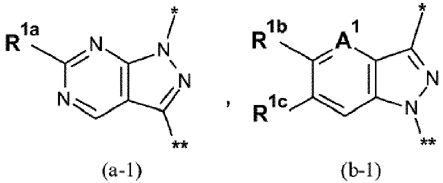 dans lesquelles
* CH₂-R²,
** pyrimidine ou triazine,
A¹ represente N ou CH,
R^{1a} represente hydrogene ou methyle,
R^{1b}
represente CH,
R^{1b} represente N,
R^{1c} represente hydrogene ou fluor,
R² represente 2,2,3,3,3-pentafluoroprop-1-yle, phenyle ou pyridyle,
le phenyle étant substitue avec 1 ou 2 substituants fluor,
et
le pyridyle pouvant etre substitue avec 1 substituant fluor,
ainsi que ses sels, solvates et solvates des sels.

4. Compose de formule (I) selon la revendication 1, 2 ou 3, dans lequel
A represente azote,
L represente un groupe #-CR^{4A}R^{4B}-(CR^{5A}R^{5B})ₘ-##,
# liaison au groupe carbonyle,
##
triazine,
m représentant un nombre 0,
R^{4A} representant methyle,
R^{4B} representant methyle,
le cycle Q represente un groupe de formule 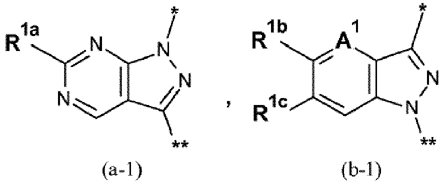
dans lesquelles
* cement de liaison a CH₂-R²,
** triazine,
A¹ represente N ou CH,
R^{1a} represente hydrogene ou methyle,
R^{1b} 1
represente CH,
R^{1b} represente N,
R^{1c} represente hydrogene ou fluor,
R² represente phenyle,
le phenyle étant substitue avec 1 ou 2 substituants fluor,
ainsi que ses sels, solvates et solvates des sels.

5. Composes selon la revendication 1, qui sont choisis parmi 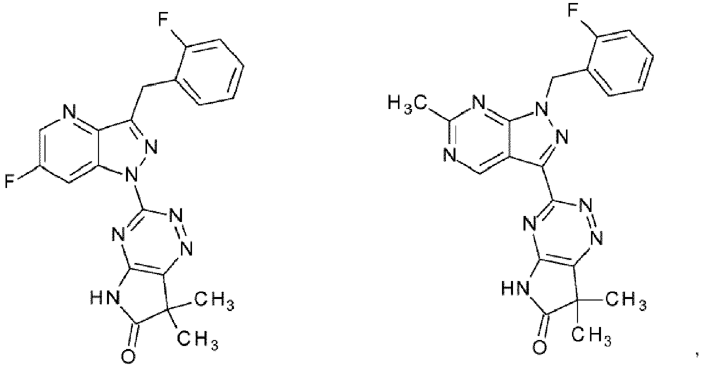 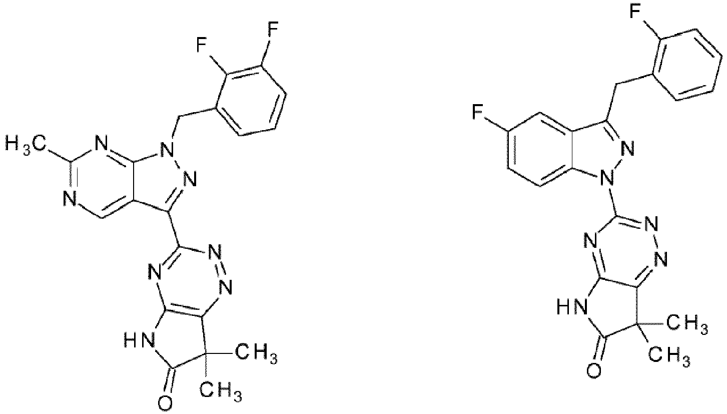 ainsi que leurs sels, solvates et solvates des sels.

6. Procede de fabrication de composes de formule (I), tels que définis dans les revendications 1 a 5, **caractérise en ce que**
[A] un compose de formule (II) 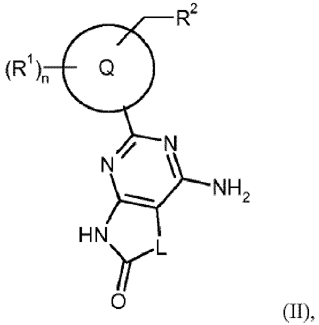 dans laquelle n, L, Q, R¹ et R² ont chacun les significations indiquées dans les revendications 1 a 5, est mis en reaction, celui-ci étant alors transforme en un compose de formule (I-A) 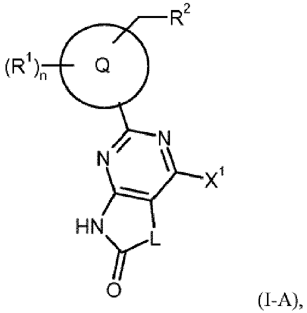
dans laquelle n, L, Q, R¹ et R² ont chacun les significations indiquées dans les revendications 1 a 5, et
X¹ represente brome ou iode,
ou
[B] un compose de formule (I-A) est transforme dans un transition approprie en un compose de formule (I-B) 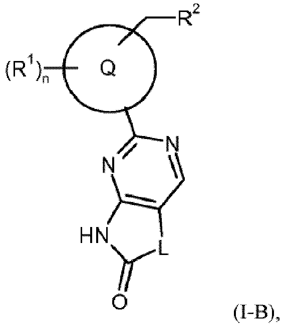 dans laquelle n, L, Q, R¹ et R² ont chacun les significations indiquées dans les revendications 1 a 5, ou
[C] un compose de formule (I-A) est mis en reaction metal de transition approprie avec un compose de formule (III-A), (III-B) ou (III-C) 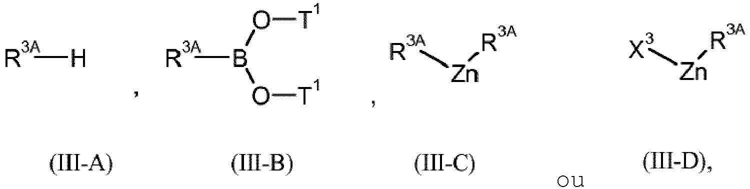 dans lesquelles
R^{3A} represente halogene, difluoromethyle, trifluoromethyle, alkyle en (C₁-C₄), alcenyle en (C₂-C₄),
alcynyle en (C₂-C₄), cyclopropyle, cyclobutyle, phenyle ou heteroaryle a 5 ou 6 elements,
₁-C_{4 2}-C₄),
₂-C₄
ou 6 elements pouvant etre substitues avec 1 a 3 substituants choisis indépendamment les uns des autres dans le groupe constitue par fluor, difluoromethyle, trifluoromethyle, alkyle en (C₁-C₄), difluoromethoxy, trifluoromethoxy, alcoxy en (C₁-C₄), alcoxycarbonyle en (C₁-C₄), cyclopropyle et cyclobutyle,
T¹ represente hydrogene ou alkyle en (C₁-C₄), ou les deux radicaux R¹¹ forment ensemble un pont C(CH₃)₂-C(CH₃)₂,
et
X³ represente brome ou iode,
pour former un compose de formule (I-C) 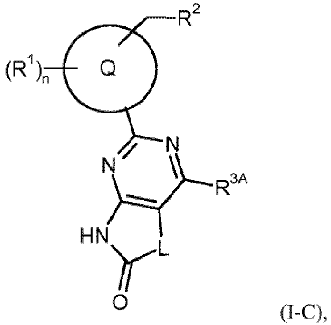
dans laquelle n, L, Q, R¹, R² et R^{3A} ont chacun les significations indiquées precedemment,
ou
[D] mis en reaction dans un solvant inerte, en
un compose de formule (IV) 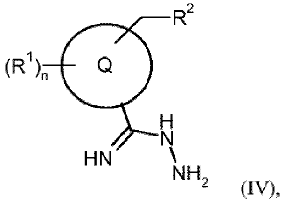 dans laquelle n, L, Q, R¹ et R² ont chacun les significations indiquées dans les revendications 1 a 5, puis celui-ci est mis en reaction dans un solvant inerte avec un compose de formule (V) 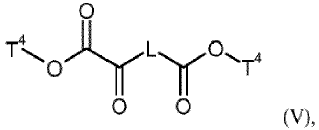
dans laquelle L a la signification indiquée dans les revendications 1 a 5, et
T⁴ represente alkyle en (C₁-C₄),
pour former un compose de formule (VI) 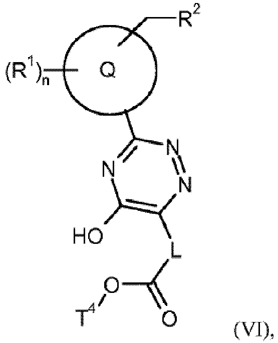
dans laquelle n, L, Q, R¹, R² et T⁴ ont chacun les significations indiquées precedemment,
puis celui-ci est transforme avec du chlorure de phosphoryle en un compose de formule (VII) 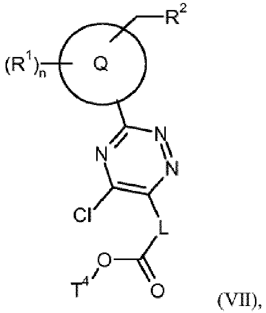
dans laquelle n, L, Q, R¹, R² et T⁴ ont chacun les significations indiquées precedemment,
et celui-ci est directement mis en reaction avec de 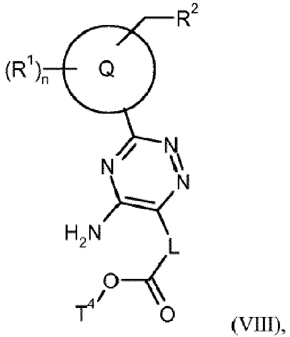
dans laquelle n, L, Q, R¹, R² et T⁴ ont chacun les significations indiquées precedemment,
et enfin cyclise dans un solvant inerte, eventuellement
base appropriée, pour former un compose de formule (I-D) 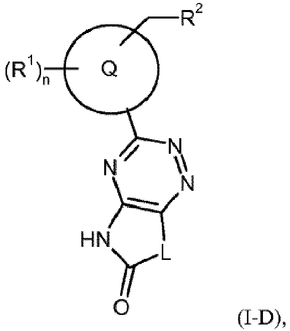 dans laquelle n, L, Q, R¹ et R² ont chacun les significations indiquees dans les revendications 1 a 5, ou
[E] un compose de formule (X) 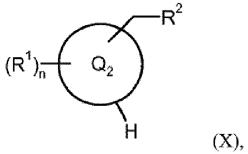
dans laquelle n, R¹ et R² ont chacun les significations indiquées dans les revendications 1 a 5, et
le cycle Q² represente un groupe de formule 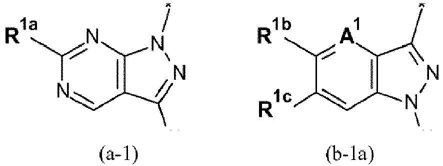 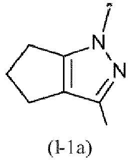
dans lesquelles
* CH₂-R²,
** R^{1a}, R^{1b}, R^{1c} et A¹ ayant les significations indiquees dans les revendications 1 a 5, est mis en reaction dans un solvant inerte, un compose de formule (XI) 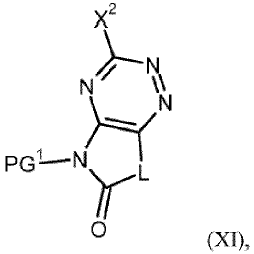
dans laquelle L a la signification indiquée dans les revendications 1 a 5,
X² represente chlore ou brome, et
PG¹
notamment p-methoxybenzyle, pour former un compose de formule (XII) 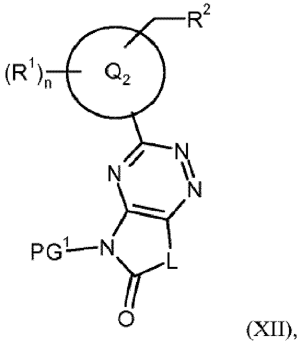
dans laquelle n, L, Q₂, R¹, R² et PG¹ ont chacun les significations indiquees precedemment,
puis le groupe protecteur PG¹ est clive de celui-ci pour former un compose de formule (I-E) 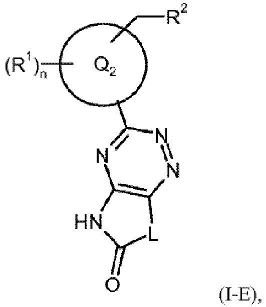
dans laquelle n, L, Q₂, R¹ et R² ont chacun les significations indiquees precedemment,
et les composes des formules (I-A), (I-B), (I-C), (I-D) et (I-E) résultants sont éventuellement transformes avec (i) les solvants et/ou (ii) les acides ou les bases appropries en leurs solvates, sels et/ou solvates des sels.

7. Compose de formule (I), tel que defini quelconque des revendications 1 a 5, pour le traitement et/ou la prophylaxie de maladies.

8. de formule (I), tel que defini
et/ou la prophylaxie de
thromboemboliques, des maladies fibrotiques et de

9. Medicament contenant un compose de formule (I), tel que defini
1 a 5, en combinaison avec un adjuvant inerte, non toxique, pharmaceutiquement approprie.

10. Medicament contenant un compose de formule (I), tel que defini
1 a 5, en combinaison avec un autre agent actif choisi dans le groupe constitue par les nitrates organiques, les donneurs de NO, les inhibiteurs de cGMP-PDE, les agents a effet antithrombotique, les agents abaissant la tension arterielle et les agents modifiant le metabolisme des lipides.

11. Medicament selon la revendication 9 ou 10, pour le
pulmonaire, des ischemies, des maladies thromboemboliques, des maladies fibrotiques et
